(19) Europäisches Patentamt
European Patent Office
Office européen des brevets

(11) EP 1 089 973 B1

(12) EUROPEAN PATENT SPECIFICATION

(45) Date of publication and mention of the grant of the patent: **09.11.2005 Bulletin 2005/45**

(21) Application number: **99930402.5**

(22) Date of filing: **18.06.1999**

(51) Int Cl.[7]: **C07D 205/08**, C07D 205/09, C07D 403/12, C07D 401/06, C07D 403/06, A61K 31/395

(86) International application number: **PCT/US1999/013811**

(87) International publication number: **WO 1999/067215 (29.12.1999 Gazette 1999/52)**

# (54) AMIDINO AND GUANIDINO AZETIDINONE TRYPTASE INHIBITORS

AMIDINO- UND GUANIDINO-AZETIDINONE TRYPTASE-INHIBITOREN

AMIDINO ET GUANIDINO AZETIDINONE INHIBITEURS DE TRYPTASE

(84) Designated Contracting States:
**AT BE CH CY DE DK ES FI FR GB GR IE IT LI LU MC NL PT SE**

(30) Priority: **25.06.1998 US 90636 P**

(43) Date of publication of application: **11.04.2001 Bulletin 2001/15**

(73) Proprietor: **Bristol-Myers Squibb Company Princeton, NJ 08543-4000 (US)**

(72) Inventors:
- **BISACCHI, Gregory Ringoes, NJ 08551 (US)**
- **SLUSARCHYK, William, A. Skillman, NJ 08558 (US)**
- **TREUNER, Uwe Yardley, PA 19067 (US)**
- **SUTTON, James, C. Princeton Junction, NJ 08550 (US)**
- **ZAHLER, Robert Pennington, NJ 08534 (US)**
- **SEILER, Steven Pennington, NJ 08534 (US)**
- **KRONENTHAL, David, R. Yardley, PA 19067 (US)**
- **RANDAZZO, Michael, E. East Windsor, NJ 08520 (US)**
- **XU, Zhongmin Plainsboro, NJ 08536 (US)**
- **SHI, Zhongping West Windsor, NJ 08550 (US)**
- **SCHWINDEN, Mark, D. Holland, PA 18966 (US)**

(74) Representative: **Vossius & Partner Siebertstrasse 4 81675 München (DE)**

(56) References cited:
**US-A- 5 037 819**

- **HAN W.T. ET AL: 'Azetidin-2-one Derivvatives as Inhibitors of Thrombin' BIOORGANIC & MEDICAL CHEMISTRY vol. 3, no. 8, August 1995, pages 1123 - 1143, XP002924637**

Remarks:
The file contains technical information submitted after the application was filed and not included in this specification

EP 1 089 973 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

## Background Of The Invention

Han in U.S. Patents 5,037,819, 5,110,812, 5,175,283, 5,250,677 and 5,326,863 discloses 3-guanidinoalkyl-2-azetidinones of the formula

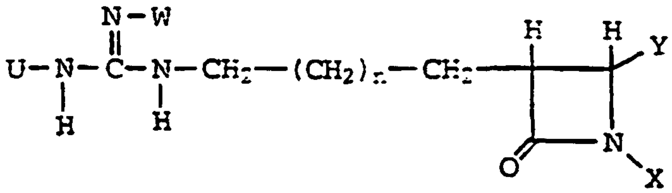

wherein:

U and W are independently selected from hydrogen and amino protecting groups;
n is an integer from 1 to 3;
X is hydrogen, trialkylsilyl, arylsulfonyl, amino substituted arylsulfonyl, alkylsulfonyl, arylaminocarbonyl, alkylcarbonyl or arylcarbonyl;
Y is hydrogen, arylalkenyl, arylalkyl, formyl, carboxy, alkoxycarbonyl, acyloxy, arylthio, arylsulfinyl, arylsulfonyl, alkythio, alkylsulfinyl, alkylsulfonyl, arylaminocarbonyl,

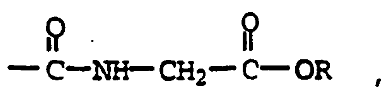

or

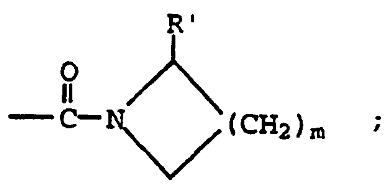

R is hydrogen, alkyl, or arylalkyl;
m is an integer from 1 to 3; and
R' is hydrogen or $-CO_2R''$ wherein R" is hydrogen, alkyl, or arylalkyl.

Han further discloses that the above compounds wherein:

U and W are hydrogen;
X is arylsulfonyl, amino substituted arylsulfonyl, alkylsulfonyl, arylaminocarbonyl, alkylcarbonyl, or arylcarbonyl; and
Y is hydrogen, arylalkyl, carboxy, alkoxycarbonyl, acyloxy, arylsulfonyl, alkylthio, alkylsulfonyl, arylaminocarbonyl,

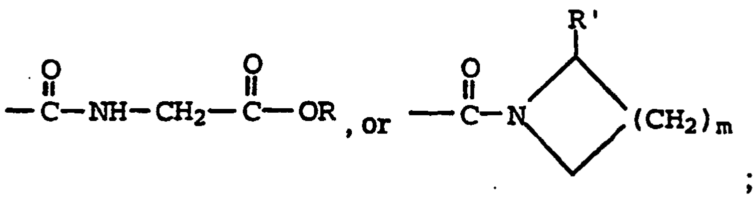

R is hydrogen, alkyl or arylalkyl;
R' is hydrogen or $-CO_2R"$;
R" is hydrogen, alkyl, or arylalkyl and pharmaceutically acceptable salts thereof are inhibitors against serine proteases, particularly against thrombin and trypsin, and can be used to control blood coagulation or to treat pancreatitis.

Han defines "aryl" as a phenyl or naphthyl group which may be unsubstituted or substituted with one or more groups such as amino, nitro, or alkyl and defines "amino" as unsubstituted or substituted with one or two alkyl radicals.

## Summary Of The Invention

This invention is directed to the novel beta lactam compounds of formulas I, II, III, IV, and V shown below and to the use of such compounds as inhibitors of various *in vivo* enzyme systems including tryptase, thrombin, trypsin, Factor Xa, Factor VIIa, and urokinase-type plasminogen activator. This invention is also directed to the use of the compounds of formula VI shown below as tryptase, Factor Xa, Factor VIIa, and urokinase-type plasminogen activator inhibitors.

Compounds of this invention include the formula:

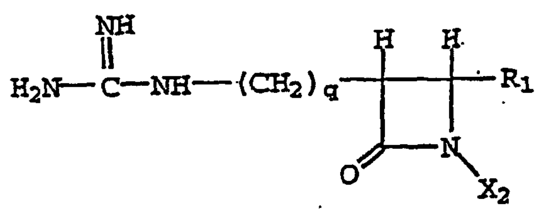

(IV)

;

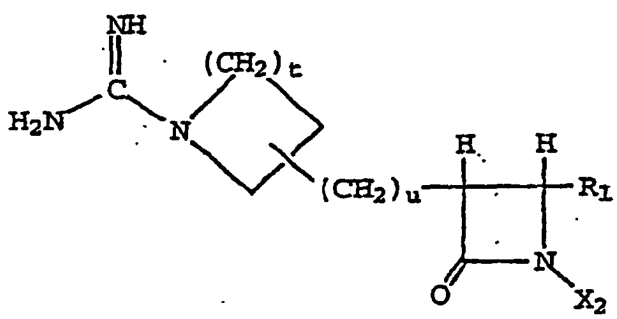

(I')

including an inner salt or a pharmaceutically acceptable salt thereof
wherein:

q is 3; t is two or three, u is one;
$R_1$ is carboxy,

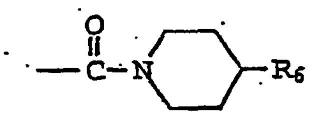,
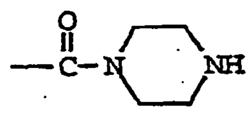,
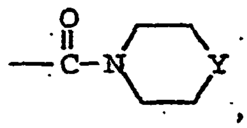,

or

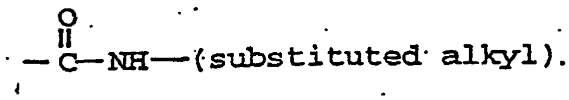

$X_2$ is

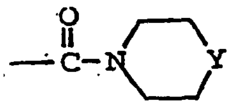,
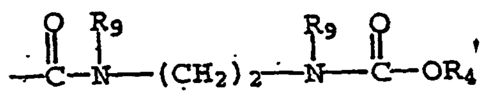,

or

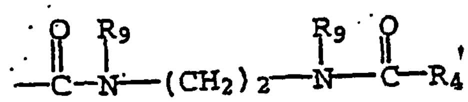;

$R_6$ is aminocarbonyl,

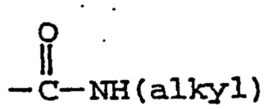, or
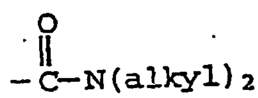;

Y is N-$R_4'$,

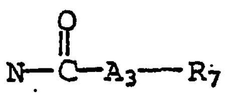,
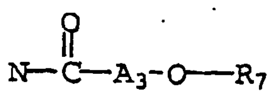

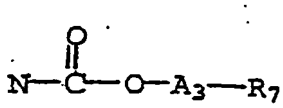,
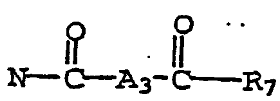,

or

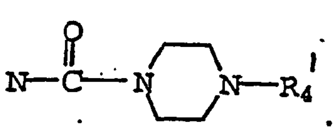;

$R_4'$ in the definition of Y and $X_2$ is alkyl, cycloalkyl, substituted alkyl, substituted cycloalkyl, or heteroaryl;
$-R_7$ is alkyl, cycloalkyl, substituted alkyl, substituted cycloalkyl, phenyl, $-(CH_2)_{1\ to\ 4}$-phenyl, $-(CH_2)_{1\ to\ 4}$-phenyl-$A_3$-phenyl,

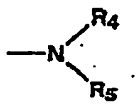

or

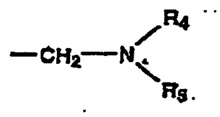

;

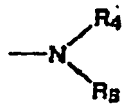

is amino, -NH(alkyl) or $-N(alkyl)_{2}$.
$R_9$ is $C_1$-$C_4$ alkyl;
$A_3$ is a bond, an alkylene bridge of 1 to 6 carbons,

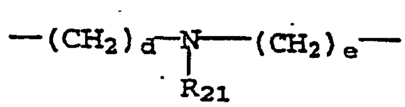

or

$$-(CH_2)_d-O-(CH_2)_e- ;$$

d and e are independently selected from zero and an integer from 1 to 6; and
$R_{21}$ is hydrogen or $C_1$-$C_4$ alkyl.

## Detailed Description Of The Invention

The term "alkyl" refers to straight or branched chain radicals having up to ten carbon atoms The term "lower alkyl" refers to straight or branched radicals having up to four carbon atoms and is a preferred subgrouping for the term alkyl.

The term "substituted alkyl" refers to such straight or branched chain radicals of 1 to 10 carbons wherein one or more, preferably one, two or three, hydrogens have been replaced by a hydroxy, amino, cyano, halo, trifluoromethyl, nitro, -NH(lower alkyl), $-N(lower\ alkyl)_2$, alkoxy, alkylthio, carboxy, alkoxycarbonyl, aminocarbonyl, or alkoxycarbonylamino.

The term "alkoxy" refers to such alkyl groups as defined above attached to an oxygen. The term "alkylthio" refers to such alkyl groups as defined above attached to a sulfur. The terms "lower alkoxy" and "lower alkylthio" refer to such lower alkyl groups as defined above attached to an oxygen or sulfur.

The term "cycloalkyl" refers to fully or partially saturated rings of 3 to 7 carbons.

The term "substituted cycloalkyl" refers to such rings of 3 to 7 carbons having one or more substituents selected from lower alkyl, lower alkoxy, lower alkylthio, halo, hydroxy, trifluoromethyl, nitro, cyano, amino, -NH(lower alkyl), $-N(lower\ alkyl)_2$, or carboxy as well as such rings fused to a phenyl ring such as tetrahydronaphthyL

The term "heteroaryl" refers to unsaturated and partially saturated rings of 4 to 7 atoms containing one or two O and S atoms and/or one to four N atoms, one to three N atoms when the ring is 4 atoms, provided that the total number of hetero atoms in the ring is 4 or less, 3 or less when the ring is 4 atoms. The heteroaryl ring is attached by way of an available carbon or nitrogen atom. Preferred heteroaryl groups include 2-,3-, or 4-pyridyl, 4-imidazolyl,4-thiazolyl, 2- and 3-thienyl, 2- and 3-furyl, and 2-(1,4,5,6-tetrahydropyrimidinyl). The term heteroaryl also includes bicyclic rings wherein the 4 to 7 membered ring containing O, S and N atoms as defined above is fused to a benzene, cycloalkyl, heteroaryl or heterocycloalkyl ring. Preferred bicyclic rings are 2- and 3-indolyl and 4-and 5-quinolinyl. The mono or bicyclic heteroaryl ring can also be additionally substituted at one or more available carbon atoms by a lower alkyl, halo, carboxy, hydroxy, $A_2$-lower alkoxy, $A_2$-guanido, benzyl or cyclohexylmethyl. Also, if the mono or bicyclic ring has

an available N-atom such N atom can also be substituted by an N-protecting group such as benzyloxycarbonyl, *tert*-butoxycarbonyl, benzyl or benzhydryl.

The term "heterocycloalkyl" refers to fully saturated rings of 4 to 7 atoms containing one or two O and S atoms and/or one to four N atoms, one to three N atoms when the ring is 4 atoms, provided that the total number of hetero atoms in the ring is 4 or less, 3 or less when the ring is 4 atoms. The heterocycloalkyl is attached by way of an available carbon or nitrogen atom. Preferred heterocycloalkyl groups include pyrrolidinyl, tetrahydrofuranyl, tetrahydrothienyl, morpholinyl, tetrahydro-1,2-thiazinyl, piperazinyl, piperidinyl, homopiperizinyl and azetidinyl. The term heterocycloalkyl also includes bicyclic rings wherein the 4 to 7 membered saturated ring containing O, S and N atoms as defined above is fused to a cycloalkyl, benzene, heteroaryl, or heterocycloalkyl ring. The mono or bicyclic heterocycloalkyl ring can also be substituted at one or more available carbon atoms by a lower alkyl, halo, carboxy, hydroxy, $A_2$-lower alkoxy, $A_2$-guanido, benzyl or cyclohexylmethyl. Also, if the mono or bicyclic heterocycloalky ring has an available N atom such N atom can also be substituted by an N-protecting group such as benzyloxycarbonyl, *tert*-butoxycarbonyl, benzyl or benzhydryl.

The term "halo" refers to chloro, bromo, fluoro and iodo.

The terms "alkylene" and "substitued alkylene" refer to a bridge of 1 to 10 carbons such as $-CH_2-$, $-(CH_2)_2-$, $-(CH_2)_9-$, etc. One or more hydrogens, preferably one, in the alkylene bridge can be replaced by an alkyl, substituted alkyl, carboxy, alkoxycarbonyl, amino, -NH(lower alkyl), -N(lower alkyl)$_2$, hydroxy, aminocarbonyl, alkoxycarbonylamino, halo, cycloalkyl, substituted cycloalkyl, aryl, substituted aryl, hetereoaryl, or heterocycloalkyl, e.g.

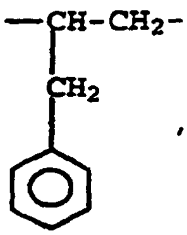 , 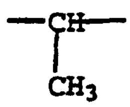 , 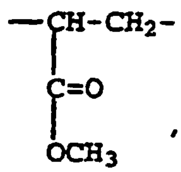 , 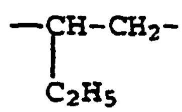 ,

etc.

The terms "alkenyl" and "substituted alkenyl" refer to a bridge of 2 to 10 carbons having one or more double bonds, preferably 2 to 6 carbons with one double bond, such as -CH=CH-, $-CH=CH-CH_2-$, $-CH_2-CH=CH-$, etc. One or more hydrogens, preferably one, in the alkenyl bridge can be replaced by an alkyl, substituted alkyl, carboxy, alkoxycarbonyl, amino, -NH(lower alkyl), -N(lower alkyl)$_2$, hydroxy, aminocarbonyl, alkoxycarbonylamiao, halo, cycloalkyl substituted cycloalkyl, aryl, substituted aryl, heteroaryl, or heterocycloalkyl, e.g.

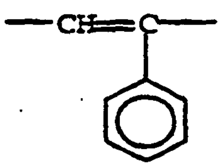 , 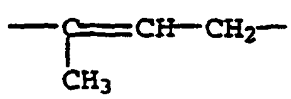 ,

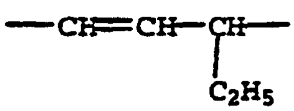 ,

etc.

The compounds of formula IV, can be prepared as follows.

The carboxy substituted azetidinone of the formula

(VII)

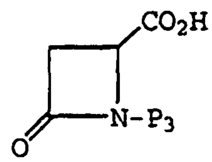

wherein $P_3$ is a silyl protecting group such as *tert*-butyldimethylsilyl is treated with an alkyldihalide of the formula

(VIII) $Cl-(CH_2)_q-I$

in the presence of base to give the carboxy substituted azetidinone of the formula

(IX)

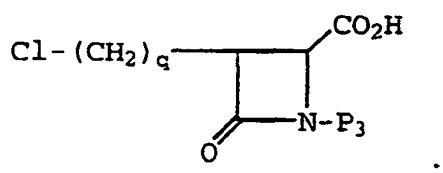

The carboxy substituted azetidinone of formula IX is then treated with an azide such as sodium azide followed by a fluoride ion salt such as tetrabutylammonium fluoride to remove the silyl group and give

(X)

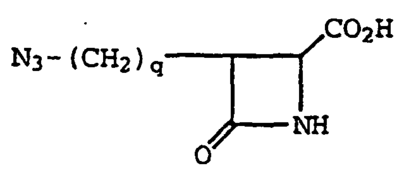

Hydrogenation of the compound of formula X by treating with hydrogen in the presence of palladium on carbon catalyst gives the alkylamino compound of the formula

(XI)

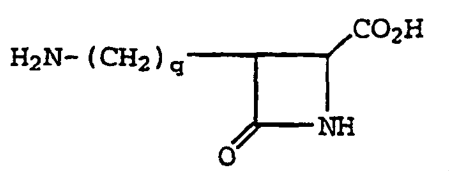

The alkylamino compound of formula XI, preferably as an acid salt, is reacted with the diprotected guanylating agent of the formula

(XII)

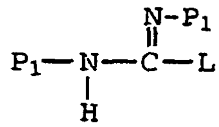

wherein $P_1$ is an N-protecting group such as *tert*-butoxycarbonyl or benzyloxycarbonyl and L is a leaving group such as methylthio or pyrazolyl to give the azetidinone compound of the formula

(XIII)

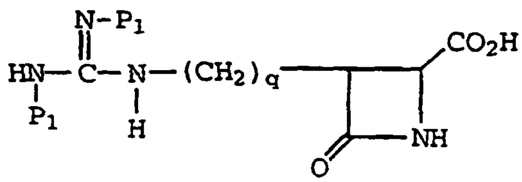

.

Coupling the intermediate of compound XIII with an amine selected from

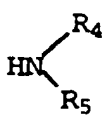

,

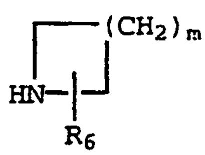

,

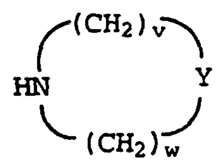

,

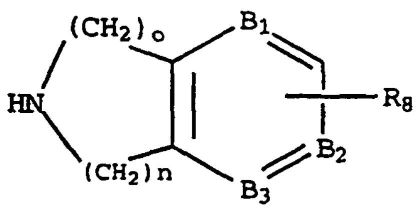

,

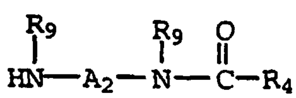

, or

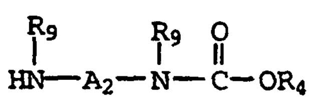

gives the compound of the formula

(XIV)

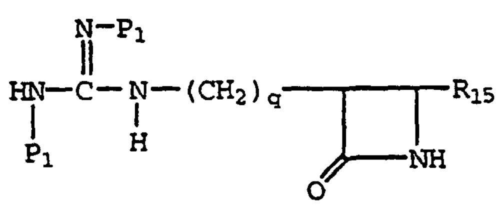

wherein $R_{15}$ is

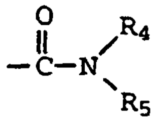 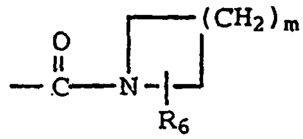 ,

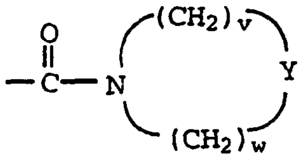 , 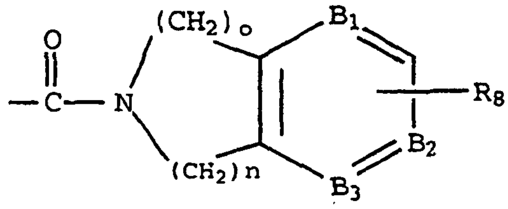 ,

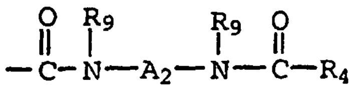 , or 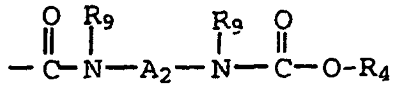 .

Reacting the intermediate of formula XIV with an acid chloride selected from

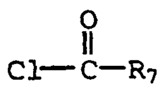 , 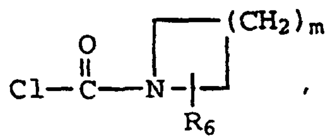 ,

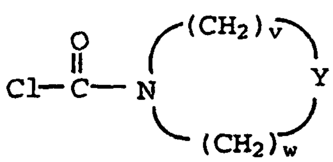 , 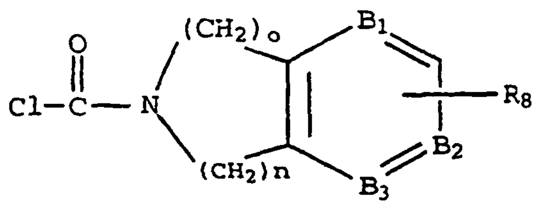 ,

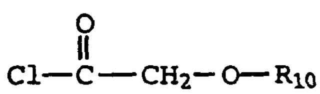 , 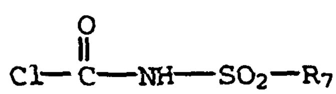 ,

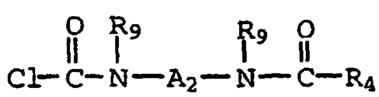 , 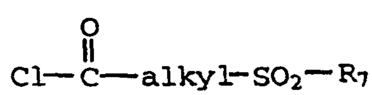 ,

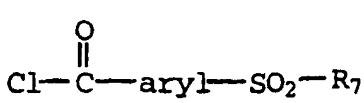 , 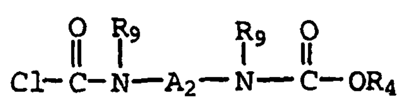

or $Cl\text{-}SO_2\text{-}R_7$ or reacting with $OCN\text{-}SO_2\text{-}R_9$ gives the compound of the formula

(XV)

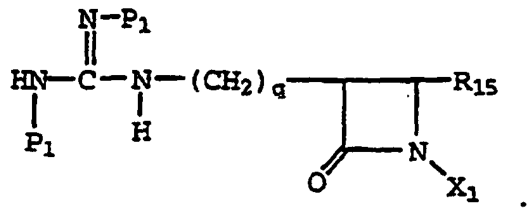

Removal of the N-protecting groups gives the compounds of formula IV.

The compounds of formula IV wherein $R_1$ is carboxy or alkoxy carbonyl can be prepared by reacting the intermediate of formula XIII with an alcohol, bromide, or iodide of the formula

(XVI) HO-Z , Br-Z, or I-Z

wherein Z is alkyl, substituted alkyl, benzyl or benzhydryl. When XVI is HO-Z, the reaction is performed in the presence of a coupling reagent such as dicyclohexylcarbodiimide, 1-ethyl-3-(3-dimethylamino)propyl carbodiimide, benzotriazol-1-yloxytris(dimethylamino)phosphonium hexafluorophosphate, or carbonyldiimidazole. When XVI is Br-Z or I-Z, the reaction is performed in the presence of a base such as sodium carbonate or bicarbonate. The reaction gives the compound of the formula

(XVII)

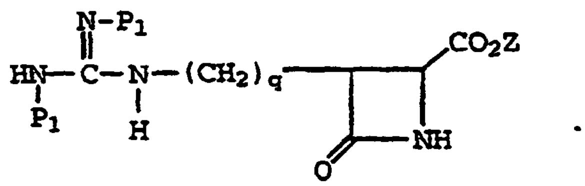

Reacting the intermediate of formula XVII with an acid chloride selected from

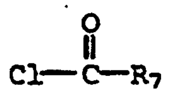 , 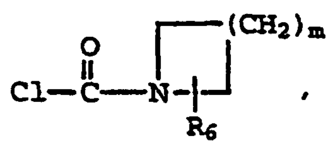 ,

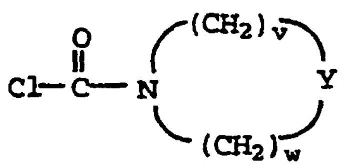 , 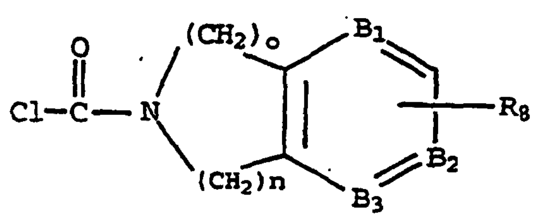 ,

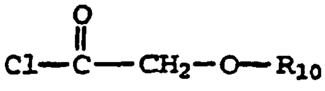 , 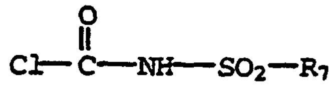 ,

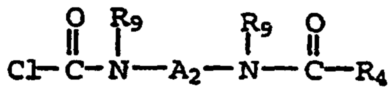, 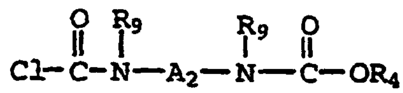,

$Cl-SO_2-R_7$, 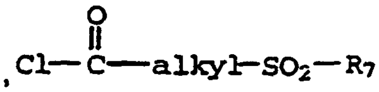, or 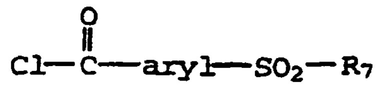,

or reacting with $OCN-SO_2-R_7$ gives the compound of the formula

(XVIII)

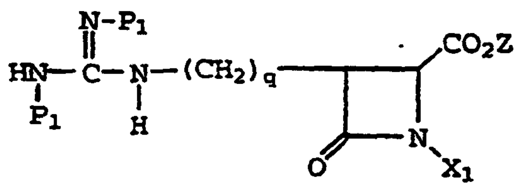

When Z is a protecting group such as benzyl or benzhydryl, removal of this group and the N-protecting groups from the compound of formula XVIII gives the desired compounds of formula IV wherein $R_1$ is carboxy.

Also, when Z is alkyl or substituted alkyl and $X_1$ is

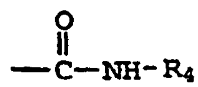,

the compound of formula XVIII can be treated to remove the N-protectings groups followed by mild aqueous hydrolysis to give the desired compounds of formula IV wherein $R_1$ is carboxy.

Compounds of formula II, can be prepared by reacting the acid chloride of the formula

(XXIX)

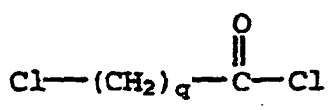

with the N-protected compound of the formula

(XXX)

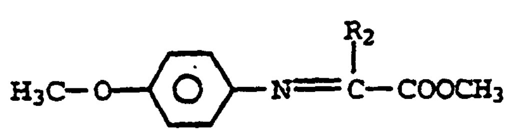

in the presence of base to give the azetidinone of the formula

(XXXI)

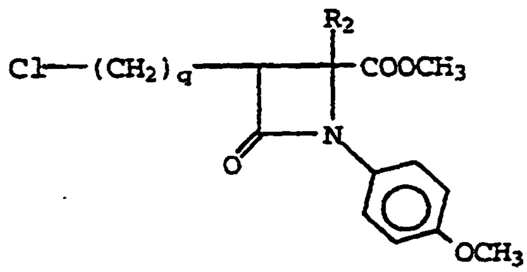

Treatment of the azetidinone of formula XXXI with an azide such as sodium azide gives the azetidinone

(XXXII)

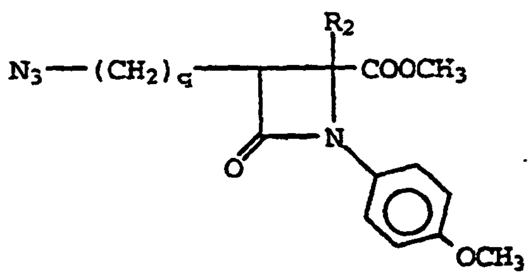

Treatment of the compound of formula XXXII with ceric ammonium nitrate removes the methoxyphenyl group and the resulting azetidinone can be reacted in the same manner as the azetidinone of formula X described above to give the desired compounds of formula II.

The compounds of formula I ' can be prepared by reacting the compound of the formula

(XXXIII)

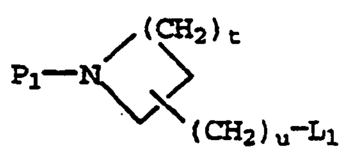

wherein $L_1$ is a leaving group such as bromo or iodo with the azetidinone of formula VII, XXI, XXIV or XXV in the presence of base to give the azetidinone of the formula

(XXXIV)

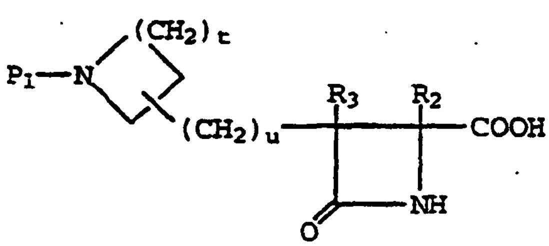

Removal of the $P_1$ protecting group and treatment of this azetidinone as described above for the azetidinone of formula XI gives the desired compounds of formula I. Alternatively, the azetidinone of formula XXXIV can first be treated with an acid chloride to introduce the desired $X_1$ group, deprotected, and then reacted with the guanylating agent of formula XII.

Alternatively, the compounds of formula I wherein $A_1$ is

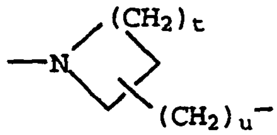

can be prepared from the azetidinone of formula XXXVII. According to this process, the ring nitrogen is protected by treating the azetidinone of formula XXXVII with, for example, *tert*-butyldimethylsilyl chloride. Treating with ozone reduces the moiety

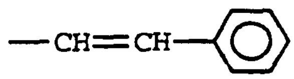

to an aldehyde which is then converted to a carboxylic acid by Jones oxidation or by treating with sodium chlorite and sulfamic acid. Removal of the silyl protecting group gives the azetidinone of the formula

(XXXVIII)

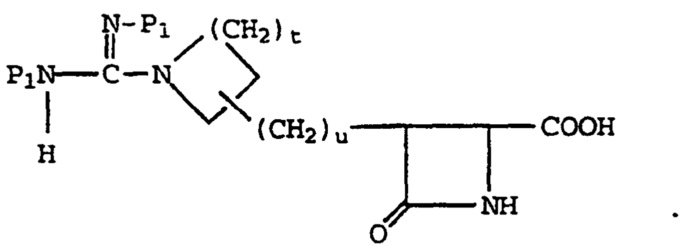

Treatment of this azetidinone as described above for the azetidinone of formula XIII gives the desired compounds of formula I.

The following is a preferred route to the intermediate of formula XIII. According to this procedure, the silyl protected azetidinone of formula VII is treated with the N-protected iodo compound of the formula

(XXXIX)

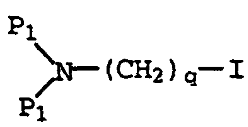

to give after removal of the silyl protecting group the azetidinone of the formula

(XL)

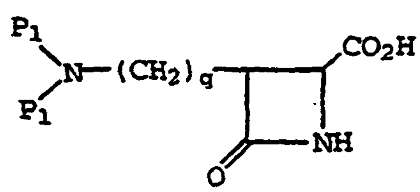

which may be isolated as an amine salt such as the tert-butylamine salt.

The $P_1$ protecting groups are removed from the azetidinone of formula XL and the resulting compound is reacted with the diprotected guanylating agent of formula XII to give the intermediate of formula XIII which again may

be isolated as an amine salt such as the *tert*-butylamine salt.

The iodo compound of formula XXXIX can be prepared by reacting the diprotected amine of the formula

(XLI)

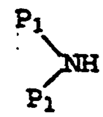

with the alkyldihalide of formula VIII to give the chloro compound of the formula

(XLII)

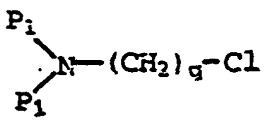

The chloro compound of formula XLII is then treated with sodium iodide in the presence of base to give the iodo compound of formula XXXIX.

The following alternate procedure can also be employed to prepare the compounds of formula IV.

The azetidinone of formula IX is reacted with benzylchloroformate in the presence of triethylamine and dimethylaminopyridine to give the benzyl ester of the formula

(XLIII)

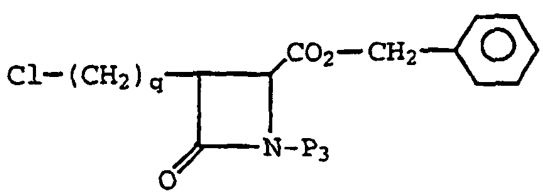

Treatment of the chloro compound of formula XLIII with sodium iodide gives the iodo compound of the formula

(XLIV)

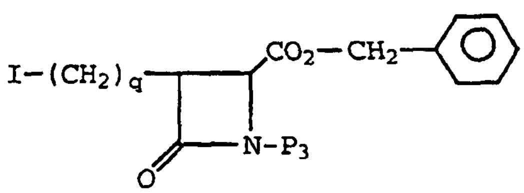

The iodo compound of formula XLIV is reacted with the diprotected guanidine of the formula

(XLV)

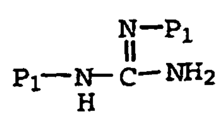

to give the azetidinone compound of the formula

(XLVI)

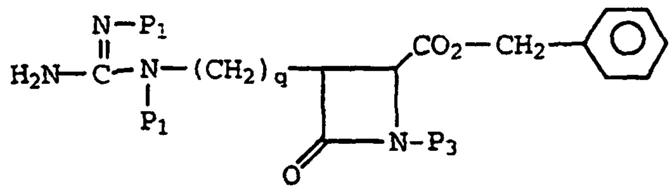

.

Removal of the silyl protecting group $P_3$ from the azetidinone of formula XLVI for example by reacting with ammonium fluoride gives the azetidinone compound of the formula

(XLVII)

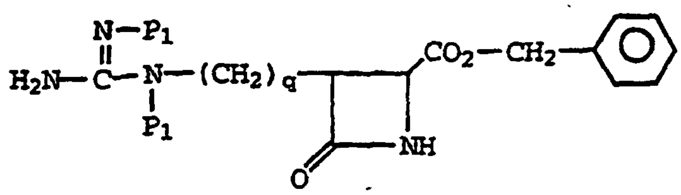

.

Reacting the intermediate of formula XLVII with an acid chloride selected from

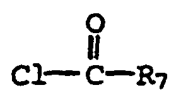
,
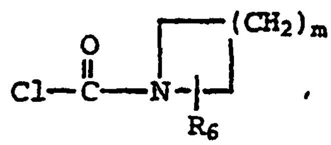
,

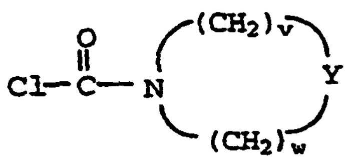
,
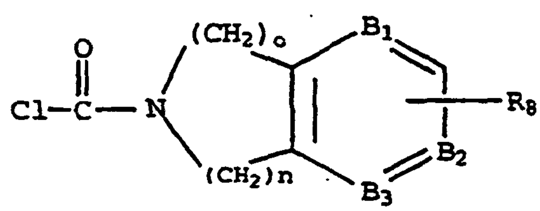
,

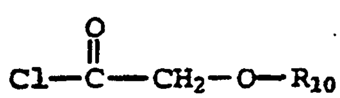
,
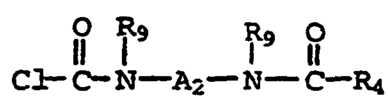
,

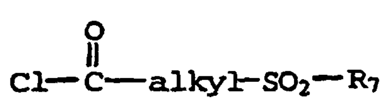
,
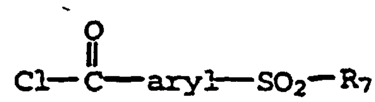
,

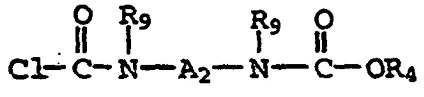
,
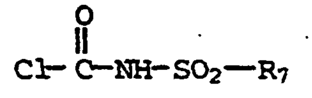
,

or Cl-$SO_2$-$R_7$, or reacting with OCN-$SO_2$-$R_7$ gives the compound of the formula

(XLVIII)

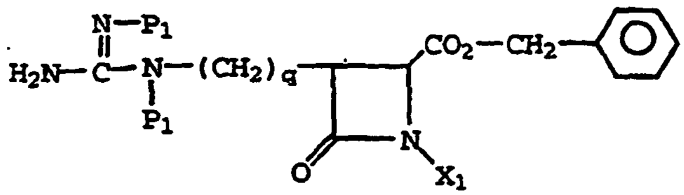

.

Removal of the benzyl protecting group and the $P_1$ N-protecting groups from the azetidinone of formula XLVIII gives the desired compounds of formula IV.

The compounds of formula I wherein $A_1$ is

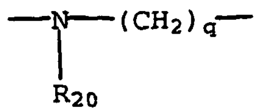

can be prepared by reacting the azetidinone of formula XL with an alcohol, bromide, or iodide of formula XVI to give the azetidinone of the formula

(XLIX)

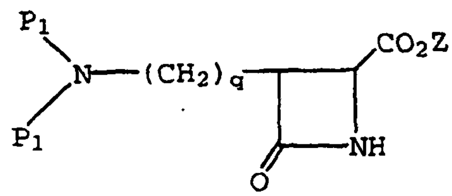

.

Reacting the intermediate of formula XLIX with an acid chloride selected from

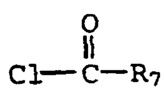

,

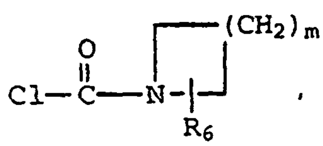

,

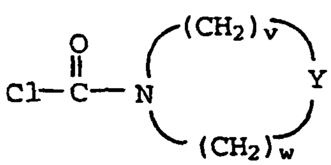

,

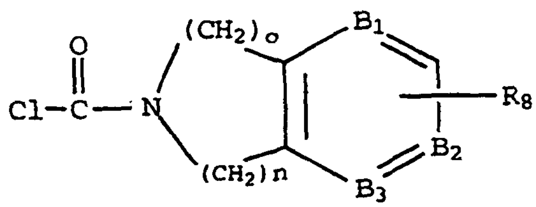

,

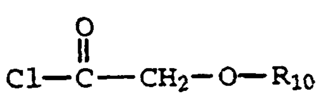

,

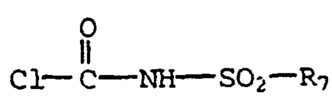

,

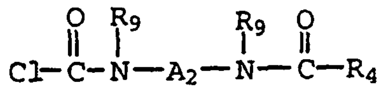, 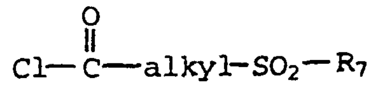,

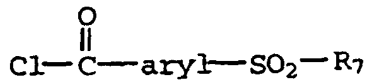, 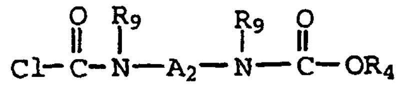.

or Cl-$SO_2$-$R_7$ or reacting with OCN-$SO_2$-$R_9$ gives the compound of the formula

(L)

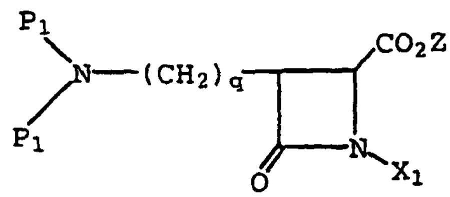

Removal of the $P_1$ protecting groups such as by treatment with trifluoroacetic acid when $P_1$ is *tert*-butoxycarbonyl gives the trifluoroacetic acid amine salt of the formula

(LI)

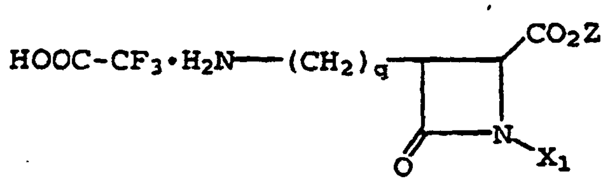

Treatment of the trifluoroacetic acid amine salt of formula LI with the appropriate aldehyde in the presence of a reducing agent such as triacetoxy borohydride or sodium cyanoborohydride gives the compound of the formula

(LII)

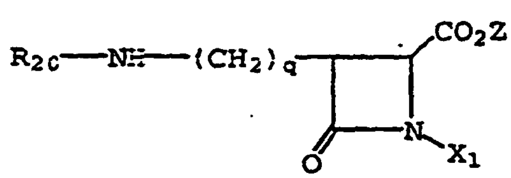

The compound of formula LII is reacted with the diprotected guanylating agent of formula XII to give the azetidinone of the formula

(LIII)

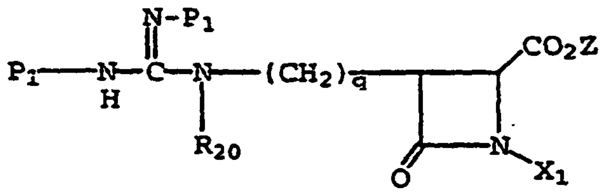

Removal of the $P_1$ and Z protecting groups gives the desired compounds of formula I.

The azetidinone compounds of formula I' and IV and various intermediates and starting materials employed in their synthesis contain one or two asymmetric carbons as denoted below at ring positions 3 and 4

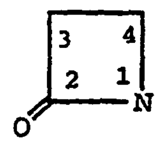

Additional asymmetric carbons may be present in the compounds of formula I' and IV depending upon the definitions of the substituents $R_1$, $A_1$, $X_1$, $X_2$, $R_{13}$, $X_3$ and $R_{14}$. As is well known in the art, see for example J. March. Advanced Organic Chemistry, Fourth Edition, John Wiley & Sons, New York, NY (1991), pages 94-164, such asymmetric carbon atoms give rise to enantiomers and diastereomers, and all such stereoisomers, either in pure form or in the form of mixtures, are included within the scope of this invention. In addition, when alkenes are present in the compounds of formula I' or IV, they may, when appropriately substituted exist as cis or trans isomers, or as mixtures thereof. Again, all such forms are within the scope of this invention.

The compounds of formula I' or IV can be obtained as a pharmaceutically acceptable salt, as a physiologically hydrolyzable ester, or as a solvate. The compounds of formulas I and IV wherein $R_1$ or $R_{14}$ is carboxy can exist in the form of an inner salt or zwitterion. All such forms are within the scope of this invention. Pharmaceutically acceptable salts include salts with mineral acids such as hydrochloric, hydrobromic, phosphoric and sulfuric as well as salts with organic carboxylic acids or sulfonic acids such as acetic, trifluoroacetic, citric, maleic, oxalic, succinic, benzoic, tartaric, fumaric, mandelic, ascorbic, malic, methanesulfonic, p-toluensulfonic and the like. Preparation of these acid addition salts is carried out by conventional techniques.

The novel compounds of formulas I' and IV possess tryptase inhibition activity. This activity was confirmed using either isolated human skin tryptase or recombinant human tryptase; prepared from the human recombinant beta-protryptase expressed by baculovirus in insect cells. The expressed beta-protryptase was purified using sequential immobilized heparin affinity resin followed by an immunoaffinity column using an anti-tryptase monoclonoal antibody. The protryptase was activated by auto-catalytic removal of the N-terminal in the presence of dextran sulfate followed by dipeptidyl peptidase I (DPPI) removal of the two N-terminal amino acids to give the mature active enzyme (Sakai et al., J. Clin. Ivest., 97, pages 988 - 995, 1996). Essentially equivalent results were obtained using isolated native enzyme or the activated expressed enzyme. The tryptase enzyme was maintained in 2M sodium chloride, 10 nM 4-morpholinepropanesulfonic acid, pH 6.8.

The assay procedure employed a 96 well microplate. To each well of the microplate (Nunc MaxiSorp), 250 µl of assay buffer [containing low molecular weight heparin and tris (hydroxymethyl)aminomethane] was added followed by 2.0 µl of the test compound in dimethylsulfoxide. The substrate (10 µl) was then added to each well to give a final concentration of either 370 µM benzoyl-arginine-*p*-nitroaniline (BAPNA) or 100 µM benzyloxycarbonyl-glycine-proline-arginine-*p*-nitroaniline (CBz-Gly-Pro-Arg-pNA). Similar data was obtained using either substrate. The microplate was then shaken on a platform vortex mixer at a setting of 800 (Sarstedt TPM-2). After a total of three minutes incubation, 10 µl of the working stock solution of tryptase (6.1 mM final tryptase concentration for use with BAPNA or 0.74 nM for use with CBz-Gly-Pro-Arg-pNA) was added to each well. The microplate was vortexed again for one minute and then incubated without shaking at room temperature for an additional 2 minutes. After this time the microplate was read on a microplate reader (Molecular Devices UV max) in the kinetic mode (405 nm wavelength) over twenty minutes at room temperature. To determine the compound concentration that inhibited half of the enzyme activity ($IC_{50}$), the fraction of control activity (FCA) was plotted as a function of the inhibitor concentration (I) and curve to fit FCA/(1 + [I]/$IC_{50}$). The $IC_{50}$ for each compound was determined 2 - 4 times and the obtained values were averaged.

As a result of this tryptase activity, the compounds of formula I' and IV as well as an inner salt thereof, a pharmaceutically acceptable salt thereof, a hydrolyzable ester thereof, or a solvate thereof, are useful as antiinflammatory agents particularly in the treatment of chronic asthma and may also be useful in treating or preventing allergic rhinitis, inflammatory bowel disease, psoriasis, conjunctivitis, atopic dermatitis, rheumatoid arthritis, osteoarthritis, and other chronic inflammatory joint diseases, or diseases of joint cartilage destruction. Additionally, these compounds may be useful in treating or preventing myocardial infarction, stroke, angina and other consequences of atherosclerotic plaque rupture. Additionally, these compounds may be useful for treating or preventing diabetic retinopathy, tumor growth and other consequences of angiogenosis. Additionally, these compounds may be useful for treating or preventing fibrotic conditions, for example, fibrosis, scteroderma, pulmonary fibrosis, liver cirrhosis, myocardial fibrosis, neurofibromas and hypertrophic scars.

The compounds of formula I' and IV are also inhibitors of Factor Xa and/or Factor VIIa. As a result, the compounds of formula I' and IV as well as an inner salt or a pharmaceutically acceptable salt thereof, a hydrolyzable ester thereof, or a solvate thereof may also be useful in the treatment or prevention of thrombotic events associated with coronary artery and cerebrovascular disease which include the formation and/or rupture of atherosclerotic plaques, venous or arterial thrombosis, coagulation syndromes, ischemia and angina (stable and unstable), deep vein thrombosis (DVT), disseminated intravascular coagulopathy, Kasacach-Merritt syndrome, pulmonary embolism, myocardial infarction, cerebral infarction, cerebral thrombosis,transient ischemic attacks, atriala fibrillation, cerebral embolism, thromboembolic complications of surgery (such as hip or knee replacement, introduction of artificial heart valves and endarterectomy) and peripheral arterial occulsion and may also be useful in treating or preventing myocardial infarction, stroke, angina and other consequences of atherosclerotic plaque rupture. The compounds of formula I to VI possessing Factor Xa and/or Factor VIIa inhibtion activity may also be useful as inhibitors of blood coagulation such as during the preparation, storage and fractionation of whole blood.

The compounds of formula I to VI are also inhibitors of urokinase-type plasminogen activator. As a result, the compounds of formula I' and IV as well as an inner salt or a pharmaceutically acceptable salt thereof, a hydrolyzable ester thereof, or a solvate thereof may be useful in the treatment or prevention of restenosis and aneurysms, in the treatment or prevention of myocardial infarction, stroke, angina and other consequences of atherosclerotic plaque rupture, and may also be useful in the treatment of malignancies, prevention of metastases, prevention of prothrombotic complications of cancer, and as an adjunct to chemotherapy.

The compounds of formulas I' and IV also possess thrombin and trypsin inhibitory activity similar to that reported by Han in the U.S. patents noted previously for the compounds of formula VI. As a result, the compounds of formula I' and IV as well as an inner salt or a pharmaceutically acceptable salt thereof, a hydrolyzable ester thereof, or a solvate thereof may be useful in treating or preventing pancreatitis, in the treatment or prevention of thrombotic events associated with coronary artery and cerebrovascular disease as described above, and may also be useful as inhibitors of blood coagulation such as during the preparation, storage, and fractionation of whole blood.

Certain compounds of formulas I' and IV are also useful due to their selective tryptase inhibition activity. These compounds while having potent tryptase inhibition activity are much less active against other enzyme systems including trypsin, thrombin and Factor Xa. The compounds of formulas I' and IV as well as an inner salt, a pharmaceutically acceptable salt thereof, a hydrolyzable ester thereof, or a solvate thereof, are useful as antiinflammatory agents particularly in the treatment of chronic asthma and may also be useful in treating or preventing allergic rhinitis as well as some of the other diseases described above for the non-selective tryptase inhibitors. It is believed that as a result of their selective tryptase inhibition activity that these compounds will have less tendency to produce unwanted side-effects.

The compounds of formula I' and IV as well as an inner salt or a pharmaceutically acceptable salt thereof, a hydrolyzable ester thereof, or a solvate thereof may be administered orally, topically, rectally or parenterally or may be administered by inhalation into the bronchioles or nasal passages. The method of administration will, or course, vary upon the type of disease being treated. The amount of active compound administered will also vary according to the method of administration and the disease being treated. An effective amount will be within the dosage range of about 0.1 to about 100 mg/kg, preferably about 0.2 to about 50 mg/kg and more preferably about 0.5 to about 25 mg/kg per day in a single or multiple doses administered at appropriate intervals throughout the day.

The composition used in these therapies can be in a variety of forms. These include, for example, solid, semi-solid and liquid dosage forms such as tablets, pills, powders, liquid solutions or suspensions, liposomes, injectable and infusible solutions. Such compositions can include pharmaceutically acceptable carriers, preservatives, stabilizers, and other agents conventionally employed in the pharmaceutical industry.

When the compounds of formula I' and IV as well as an inner salt or a pharmaceutically acceptable salt thereof, a hydrolyzable ester thereof, or a solvate thereof are employed to treat asthma or allergic rhinitis they will preferably be formulated as aerosols. The term "aerosol" includes any gas-borne suspended phase of the active compound which is capable of being inhaled into the bronchioles or nasal passage. Aerosol formulations include a gas-borne suspension of droplets of the active compound as produced in a metered dose inhaler or nebulizer or in a mist sprayer. Aerosol

formulations also include a dry powder composition suspended in air or other carrier gas. The solutions of the active compounds of formulas I to VI used to make the aerosol formulation will be in a concentration of from about 0.1 to about 100 mg/ ml, more preferably 0.1 to about 30 mg/ml, and most preferably from about 1 to about 10 mg/ml. The solution will usually include a pharmaceutically acceptable buffer such as a phosphate or bicarbonate to give a pH of from about 5 to 9, preferably 6.5 to 7.8, and more preferably 7.0 to 7.6. Preservatives and other agents can be included according to conventional pharmaceutical practice.

Other pharmaceutically active agents can be employed in combination with the compounds of formula I' and IV depending upon the disease being treated. For example, in the treatment of asthma, β-adrenergic agonists such as albuterol, terbutaline, formoterol, fenoterol or prenaline can be included as can anticholinergics such as ipratropium bromide, anti-inflammatory cortiocosteroids such as beclomethasone, triamcinolone, flurisolide or dexamethasone, and anti-inflammatory agents such as cromolyn and nedocromil.

In addition to the novel compounds of formulas I' and IV and the methods of use for the compounds of formulas I' and IV, this invention is also directed to novel intermediates and novel synthetic routes employed in the preparation of such compounds.

Preferred compounds of this invention are those of formula IV wherein:

q is 3;

$R_1$ is carboxy,

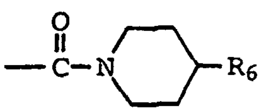
,
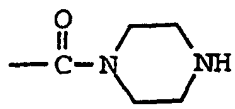
,
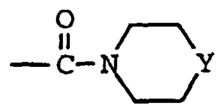
,

or

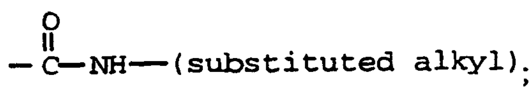

$X_2$ is

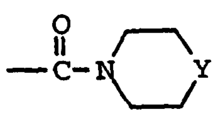
,
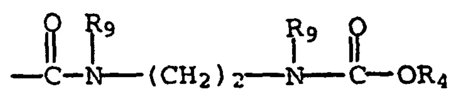
,

or

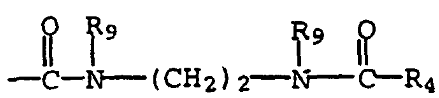

$R_6$ is aminocarbonyl,

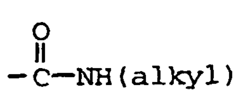
, or
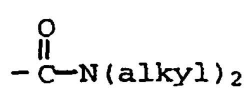
;

Y is N-$R_4$,

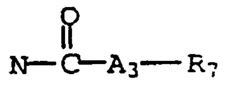, 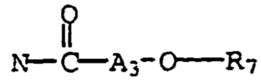

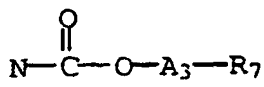, 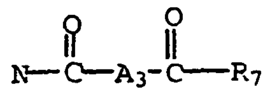,

or

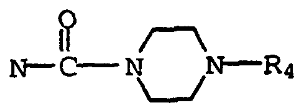;

$R_4$ in the definition of Y and $X_2$ is alkyl, cycloalkyl, substituted alkyl, substituted cycloalkyl, or heteroaryl;
$R_7$ is alkyl, cycloalkyl, substituted alkyl, substituted cycloalkyl, aryl, $-(CH_2)_{1\ to\ 4}$-aryl, $-(CH_2)_{1\ to\ 4}$-aryl-$A_3$-aryl,

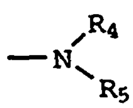, or 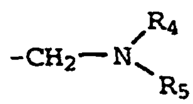;

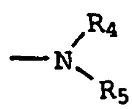

is amino, -NH(alkyl), or -N $(alkyl)_2$ ;
$R_9$ is lower alkyl;
$A_3$ is a bond, an alkylene bridge of 1 to 6 carbons,

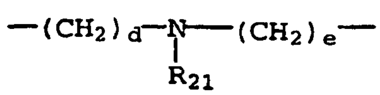

or $-(CH_2)_d-O-(CH_2)_e-$ ;
d and e are independently selected from zero and an integer from 1 to 6;
$R_{21}$ is hydrogen or lower alkyl; and an inner salt or pharmaceutically acceptable salt thereof.

Also preferred are the compounds of this invention of formula I wherein

$A_1$ is

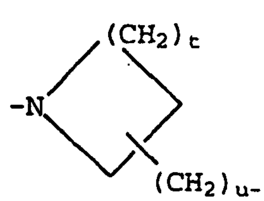;

$R_2$ and $R_3$ are both hydrogen;
$R_1$ is a defined above;
$X_1$ is

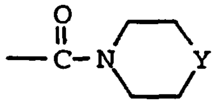
,
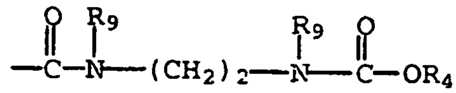
;

or

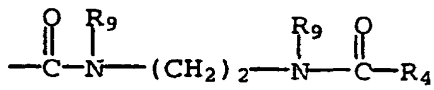

wherein Y, $R_4$ and $R_9$ are as preferably defined above;
t is two or three;
u is one; and an inner salt or a pharmaceutically acceptable salt thereof.

Most preferred are the following compounds of formula IV including an inner salt or a pharmaceutically acceptable salt thereof:

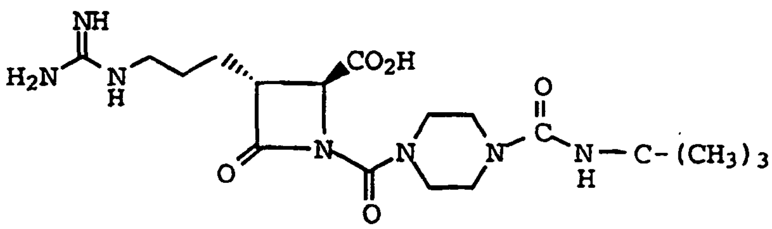
;

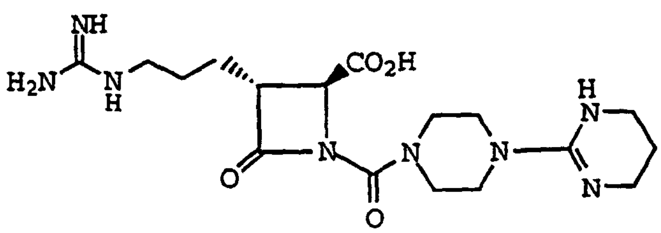
;

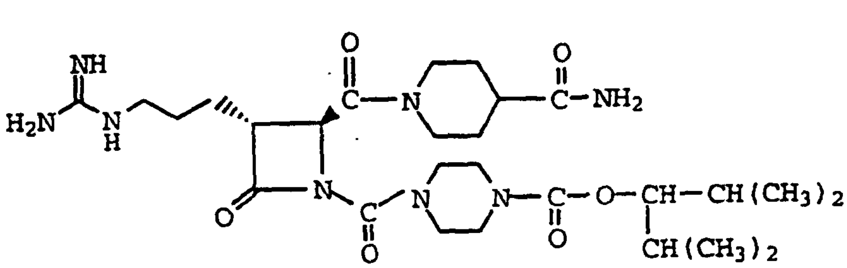
;

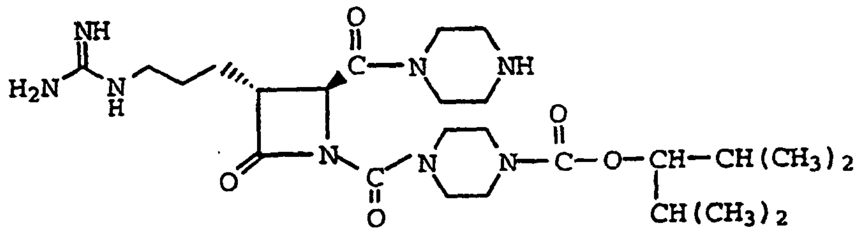

;

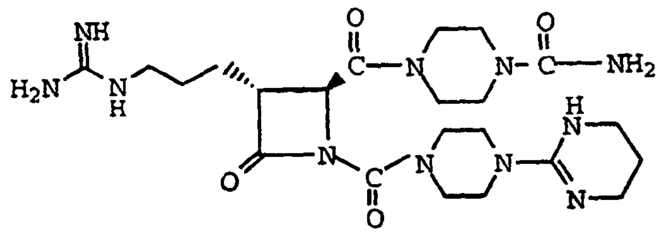

;

and

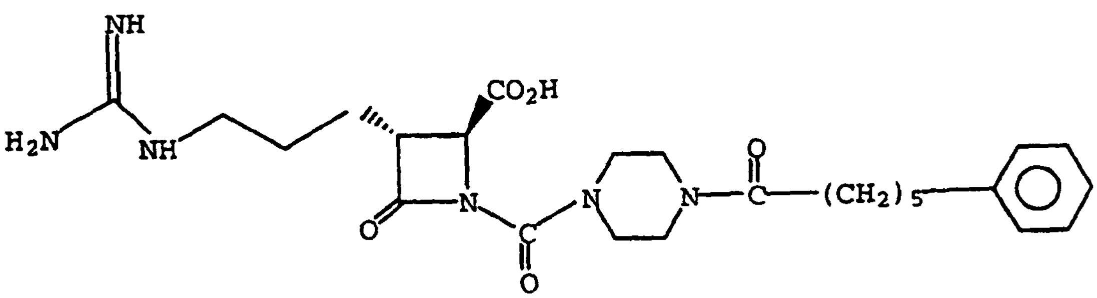

.

Also most preferred are the following compounds of formula I including an inner salt or pharmeceutically acceptable salt thereof:

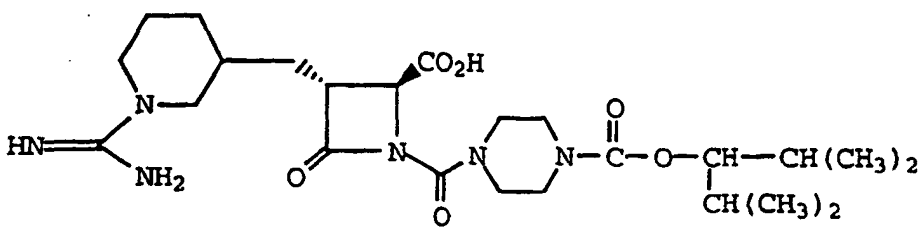

;

and

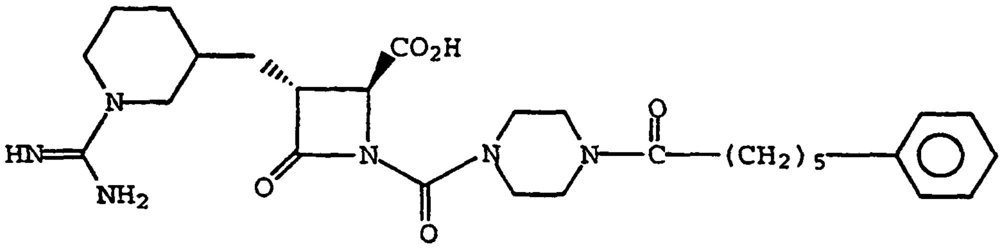

The following compounds of formula IV including an inner salt or a pharmaceutically acceptable salt thereof are also preferred:

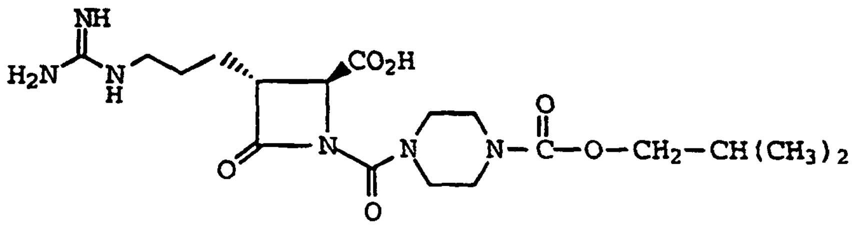

;

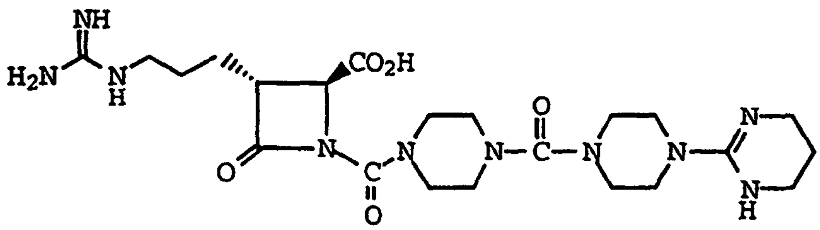

;

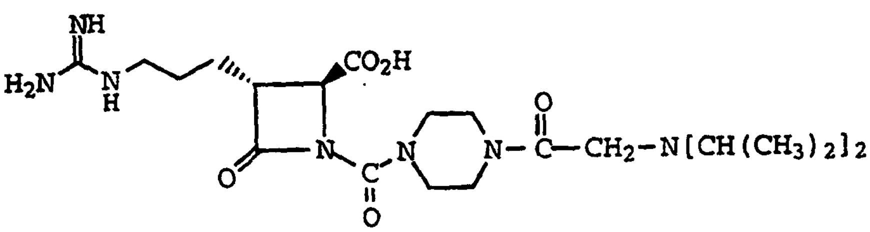

;

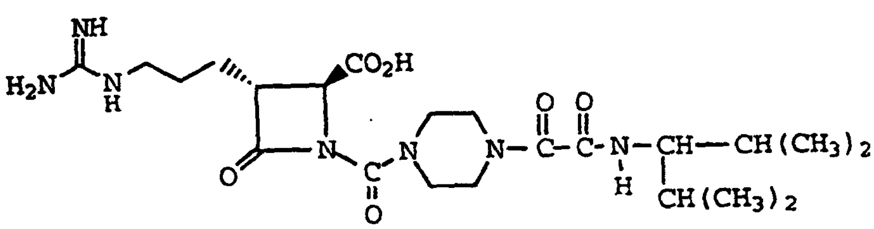

;

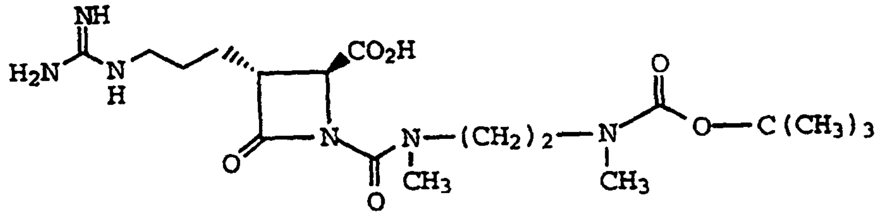

;

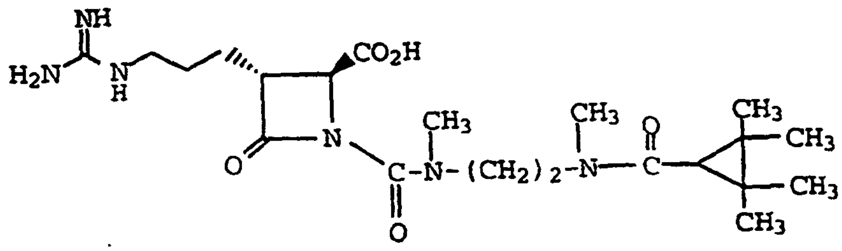

;

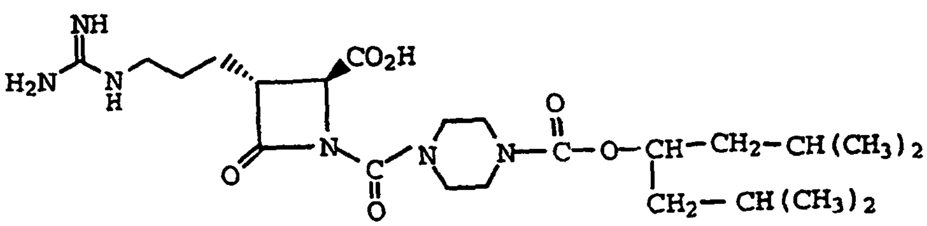

;

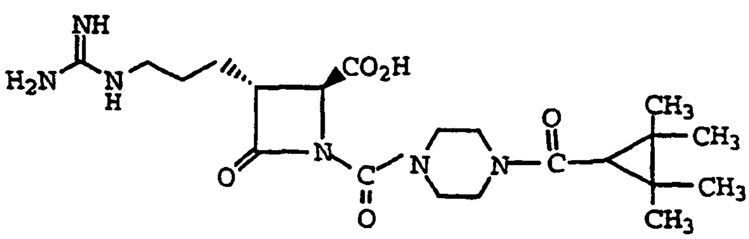

;

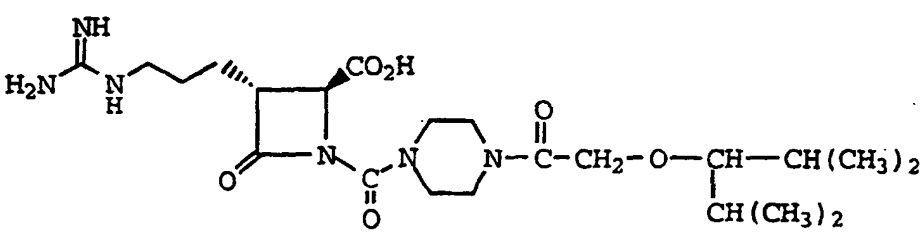

;

and

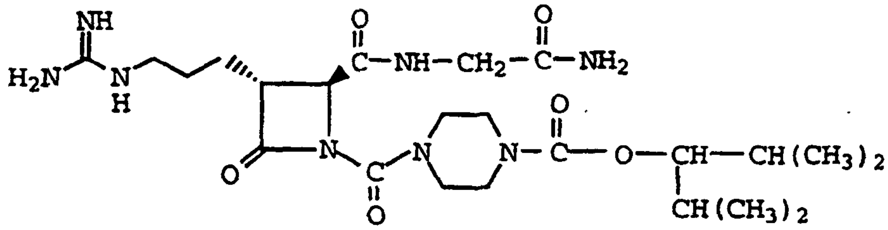

The following examples are illustrative of the invention.

Example 1 (not encompassed in the present invention)

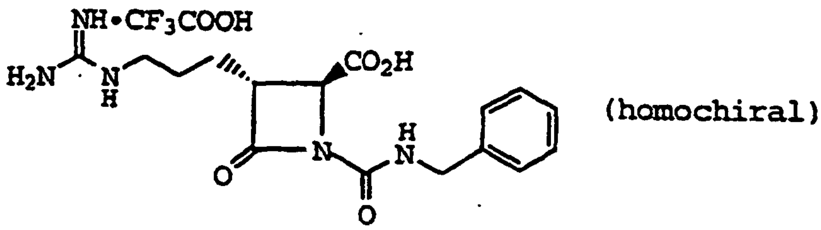

a)

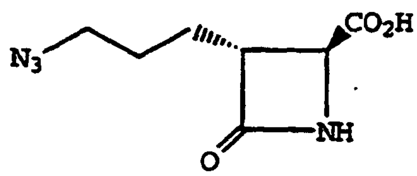

n-Butyl lithium in hexanes (2.5 M, 36.6 ml, 91.5 mmol) was added dropwise over 5 minutes to a solution of diisopropylamine (13.5 ml, 95.9 mmol) in dry tetrahydrofuran (40 ml) under nitrogen at -78°C with mechanical stirring. After warming to 0°C and stirring for 30 minutes, the solution was cooled to -78°C and (4S)-N-(*t*-butyldimethylsilyl)-azetidine-2-one-4-carboxylic acid (10 g, 43.6 mmol) [Baldwin et al, Tetrahedron, Vol. 46, p. 4733-4748, 1990] was added in a single portion. After stirring the reaction mixture - gelatinous suspension at -78°C for 5 minutes, the reaction was warmed to -20°C to -10°C and stirred at this temperature for 30 minutes. 1-Chloro-3-iodopropane (5.7 ml, 53.0 mmol) was added in a single portion and the reaction was stirred at -20°C for 2 hours (gelatinous suspension disappears upon the addition of 1-chloro-3-iodopropane). The reaction mixture was then poured into 1N HCl saturated with sodium chloride (300 ml) and the aqueous phase was extracted with ethyl acetate (1 x 150 ml) which was then washed twice with saturated 1N HCl. The aqueous layers were then extracted twice, in order, with ethyl acetate (2 x 150 ml). The combined organics were then extracted twice with pH 7.5-8 water (2 x 100 ml, adjusted by the dropwise addition of 25% sodium hydroxide). The combined basic aqueous layers were then washed with ethyl acetate (2 x 150 ml). The basic aqueous layers were then acidified with concentrated HCl to pH 3, saturated with sodium chloride (solid) and extracted with ethyl acetate (2 x 150 ml), dried over sodium sulfate, filtered and concentrated. Evaporative drying with toluene then gave

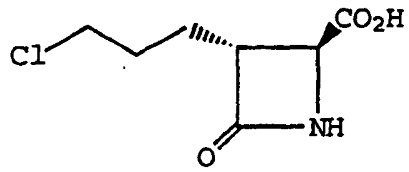

as a crude yellow oil. TLC (silica gel, 1% acetic acid in ethyl acetate) $R_f$ = 0.1, streaks.

Tetrabutylammonium iodide (0.5 g, 1.36 mmol) and tetrabutylammonium azide (15 g, 52.7 mmol) were added to a solution of the crude chloride from above (less than 43.6 mmol) in dry dimethylformamide (40 ml) under nitrogen. After stirring the reaction mixture at room temperature for 72 hours, the majority of the dimethylformamide was removed under vacuum. The product was extracted into ethyl acetate (150 ml) which was washed with 1N HCl saturated with sodium chloride (3 x 150 ml). The aqueous layers were extracted, in order, with ethyl acetate (2 x 150 ml). The combined organics were then extracted into pH 7.5 - 8 water (2 x 100 ml, adjusted by the dropwise addition of 25% sodium hydroxide). The combined basic aqueous layers were then washed with ethyl acetate (3 x 150 ml). The basic aqueous layers were then acidified with concentrated HCl to pH 3, saturated with sodium chloride (solid) and extracted with ethyl acetate, dried over sodium sulfate, filtered and concentrated. Evaporative drying with toluene gave the desired azide as a yellow-brown foam (6.56 g, 33.1 mmol). TLC (silica gel, 1% acetic acid in ethyl acetate) $R_f$ = 0.1

b)

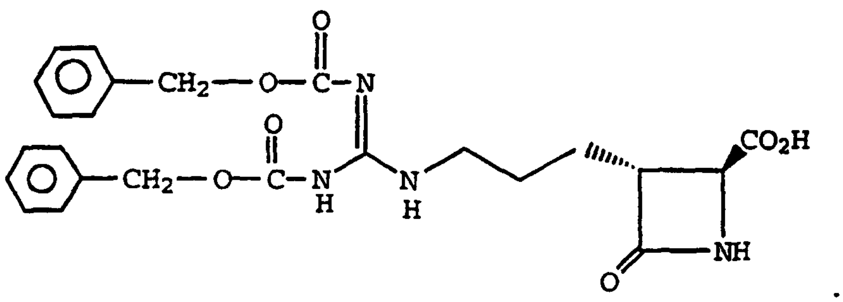

Acetic acid (4 ml, 66 mmol) was added to a solution of the azide from step (a) (6.5 g, 32.8 mmol) in dimethylformamide (40 ml) followed by 10% palladium on carbon (1.3 g). Hydrogen was bubbled through the reaction mixture for 25 minutes and then the reaction was stirred under hydrogen for 6 hours. After degassing the mixture with nitrogen for 25 minutes, triethylamine (15 ml, 108 mmol) and N,N'-bis(benzyl-oxycarbony)-1-guanylpyrazole (15 g, 39.7 mmol) [Wu et al., Synthetic Communications, 23(21), p. 3055 - 3060, (1993)] were added. The reaction was then stirred at room temperature for 18 hours. The reaction mixture was then filtered through Celite® which was then washed with ethyl acetate. Solvents were reduced under vacuum and the resulting residue was dissolved in ethyl acetate (150 ml) and washed with 1N HCl saturated with sodium chloride (3 x 150 ml). The aqueous washes were extracted, in order, with ethyl acetate (2 x 100 ml). The combined organics were then extracted with pH 7.5 - 8 water (2 x 150 ml, adjusted by the dropwise addition of 25% sodium hydroxide). The combined basic aqueous layers were then washed with ethyl acetate (3 x 150 ml) to remove excess N,N'-bis(benzyloxycarbonyl)-1-guanylprazole from the product. The basic aqueous layers were then acidified with concentrated HCl to pH 3, saturated with sodium chloride (solid), extracted with ethyl acetate (2 x 150 ml), dried over sodium sulfate, filtered and concentrated. Evaporative drying with toluene gave a light brown foam. Purification by flash chromatography (silica gel, 1 - 3% acetic acid in ethyl acetate) gave the desired product (8.5 g, 17.6 mmol) as an off-white solid. TLC (silica gel, 1% acetic acid in ethyl acetate) $R_f$ = 0.2.

c)

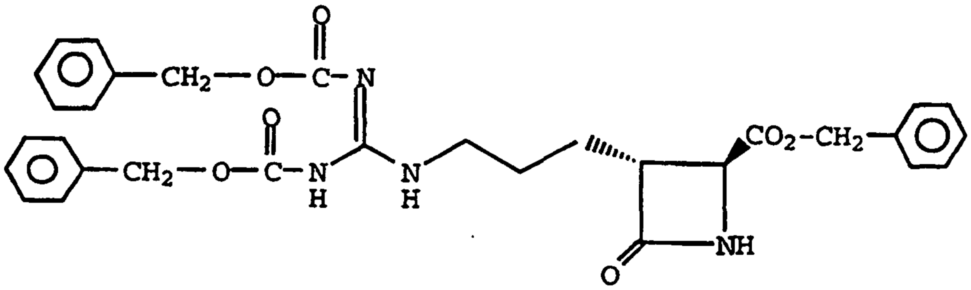

Solid sodium bicarbonate (1.5 g, 17.9 mmol), tetrabutylammonium iodide (200 mg, 0.54 mmol) and lastly benzyl bromide (2.5 ml, 21.0 mmol) were added to a solution of the product from step (b) (1.7 g, 3.52 mmol) in dimethylformamide (20 ml) under nitrogen at room temperature. The reaction mixture was stirred at room temperature for 48 hours. Dimethylformamide was removed under vacuum and the resulting residue was dissolved in ethyl acetate which was then washed twice with saturated aqueous sodium bicarbonate. The organic phase was separated, dried over magnesium sulfate, filtered and reduced to leave a brown oil. Purification by flash chromatography (silica gel, 0 - 10% methanol in methylene chloride) provided the desired product (1.84, 3.21 mmol) as a yellow oil.

d)

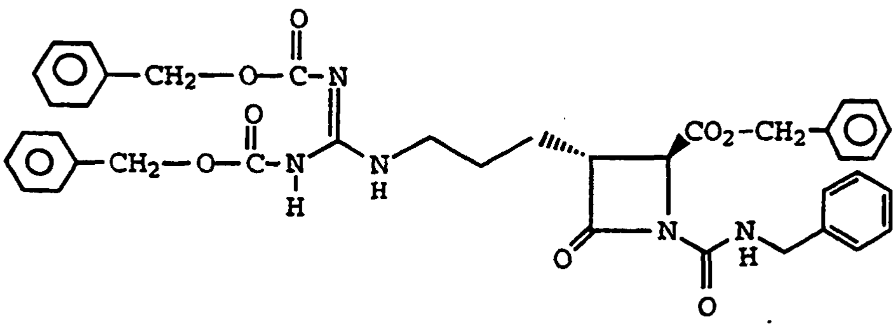

Sodium bis(trimethylsilyl)amide (1.0 M in tetrahydrofuran, 100 μl, 0.1 mmol) was added to a solution of the product from step (c) (46 mg, 0.08 mmol) in dry tetrahydrofuran (1.0 ml) under nitrogen at -78°C. The reaction mixture was stirred at -78°C for 10 minutes and then at -20°C for 10 minutes. After cooling the reaction mixture to -78°C, benzyl isocyanate (100 μl, 0.78 mmol) was added in a single portion. The reaction mixture was stirred at -78°C for 5 minutes and then at -20°C for 15 minutes. 1N HCl (1 ml) was added followed immediately by ethyl acetate (3 ml). The resulting biphasic solution was stirred vigorously while warming to room temperature. The organic phase was separated and washed once with saturated aqueous sodium bicarbonate, dried over magnesium sulfate, filtered and concentrated to leave a light yellow residue. Purification by flash chromatography (silica gel, 0-30% ethyl acetate in hexane) gave the desired product (33 mg, 0.047 mmol).

e)

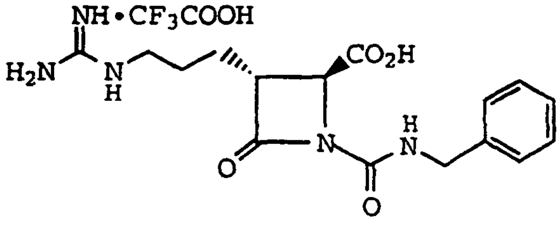

(homochiral)

Concentrated HCl (4 μl, 0.048 mmol) was added to a solution of the product from step (d) (33 mg, 0.047 mmol) in dioxane (2 ml) followed by 10% palladium on carbon catalyst (15 mg). Hydrogen gas was bubbled through the reaction mixture for 1.5 hours. Water (0.5 ml) was added and the reaction was stirred under hydrogen for an

additional 1 hour. The reaction mixture was then filtered through Celite® which was then washed with three portions of water. The combined eluent was lyophilized to give white powder. Purification by preparative HPLC (reverse phase, methanol, water, trifluoroacetic acid) provided after lyophilization the desired product (8.7 mg, 0.019 mmol). IR (film) 1773 $cm^{-1}$; MS 348.0 $(M+H)^+$, 346.3 $(M-H)^-$.

**Example 19** (not encompassed in the scope of the present invention)

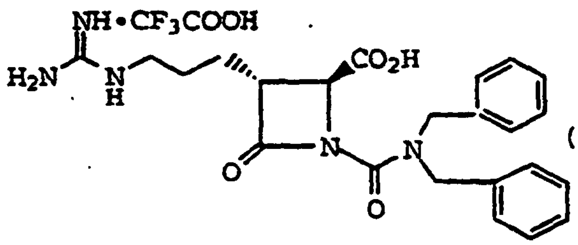

(homochiral)

a)

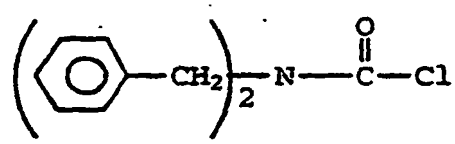

A solution of diphosgene (680 µl, 5.7 mmol) in toluene (5 ml) was added to a mixture of dibenzylamine (2.0 g, 9.8 mmol) and triethylamine (1.2 ml, 85 mmol) in toluene (15 ml). The resultant mixture was stirred at room temperature for 4 hours. The mixture was poured into 2N HCl aqueous solution (50 ml) and extracted with ethyl acetate (3 x 50 ml). The organic layers were combined, dried over magnesium sulfate and concentrated to give 2.60 g of the desired product as a white solid. IR (film) 1731 $cm^{-1}$.

b)

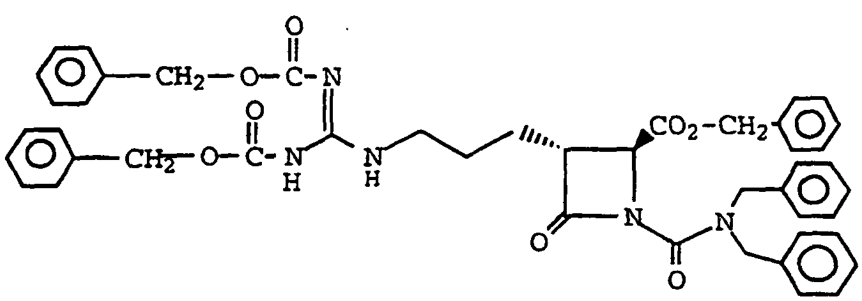

Triethylamine (42 µl), 4-dimethylaminopyridine (30 mg), and a solution of the carbamoyl chloride from step (a) (78 mg, 0.30 mmol) in methylene chloride (2 ml) were added to a solution of the benzyl ester product from Example 1(c) (116 mg, 0.20 mmol) in methylene chloride (2 ml). The mixture was stirred at room temperature for 3 days. Analytical HPLC indicated the reaction was complete. The reaction was quenched by the addition of 1N potassium bisulfate (15 ml). The mixture was extracted with ethyl acetate (100 ml). The organic layer was washed with brine (15 ml), dried over magnesium sulfate, and concentrated to give the crude product. Purifiation by flash chromatography (30% ethyl acetate/hexane) gave the desired product (95 mg). MS $(M+H)^+$ 796.1, $(M-H)^-$ 794.4; IR (film) 1785 $cm^{-1}$, 1732 $cm^{-1}$, 1671 $cm^{-1}$, 1639 $cm^{-1}$.

c)

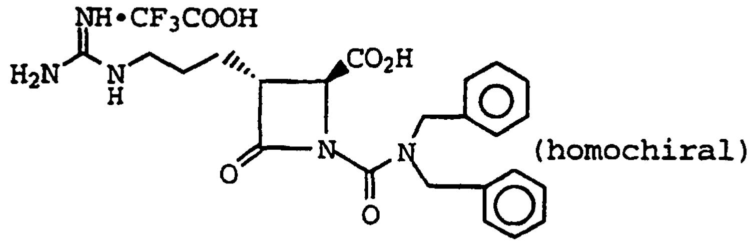

A mixture of the product from step (b) (95 mg, 0.12 mmol), 1N HCl (145 μl), and 10% palladium on carbon catalyst (61 mg) in dioxane (3 ml) was stirred under hydrogen atmosphere (hydrogen balloon) at room temperature for 2 hours. Analytical HPLC indicated the reaction was complete. The reaction mixture was filtered through a Celite® cake and concentrated to give the crude product as a white powder. Purification by reverse phase HPLC using the solvent system described in Example 1(e) gives the desired product (36 mg) as a white powder. MS $(M+H)^+$ 438.1, $(M-H)^-$ 436.3; IR (KBr) 1786 $cm^{-1}$, 1672 $cm^{-1}$.

**Example 20**

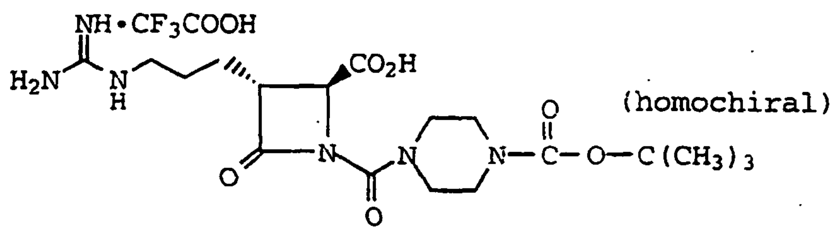

a)

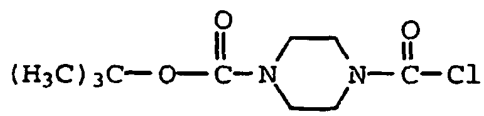

A mixture of tert-butylpiperizine carboxylate (1.0 g) and triethylamine (753 μl) in methylene chloride (5 ml) was added to a solution of diphosgene (326 μl, 20% in toluene) in methylene chloride at 0°C. The resultant mixture was stirred at 0°C for 90 minutes. TLC showed completion of the reaction. The reaction was quenched by the addition of water (20 ml). The organic layer was separated. The aqueous layer was extracted with methylene chloride (2 x 20 ml. The organic layers were combined and washed with water (10 ml) and brine (2 x 10 ml), dried over magnesium sulfate, filtered and concentrated to give the crude product. Purification by flash chromatography (methylene chloride) provided 913 mg of the desired product as a white solid. IR (KBr) 1680 $cm^{-1}$, 1747 $cm^{-1}$.

b)

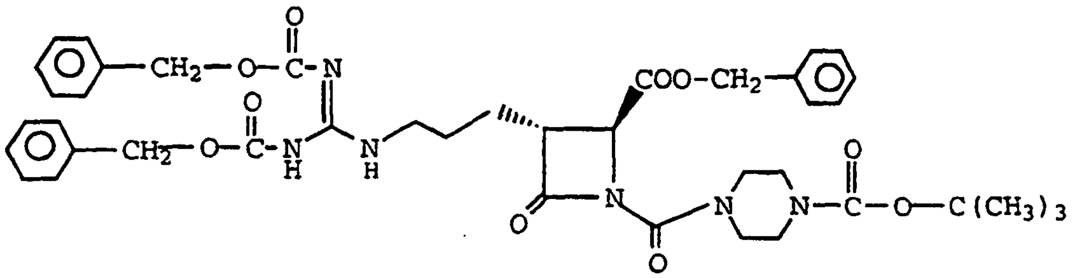

Sodium bis(trimethylsilyl)amide (1.0 M in tetrahydrofuran, 180 µl, 0.18 mmol) was added dropwise to a -78°C solution of the benzyl ester product of Example 1(c) (85 mg, 0.15 mmol) in tetrahydrofuran (3 ml). The mixture was stirred at -78°C for 90 minutes. A solution of the acid chloride product from part (a) (45 mg, 0.18 mmol) in tetrahydrofuran (1 ml) was added. The reaction mixture was stirred at -78°C for 5 hours. The reaction was quenched by the addition of 1N potassium bisulfate (15 ml). The mixture was extracted with ethyl acetate (3 x 30 ml). The organic layers were combined and washed with brine (2 x 15 ml), dried over magnesium sulfate, filtered and concentrated to give the crude product. Purification by flash chromatography (30 - 50% ethyl acetate/hexane) provided 32 mg of the desired product as a colorless oil. MS $(M+H)^+$ 785.4, $(M-H)^-$ 783.7; IR (neat) 1786 $cm^{-1}$, 1732 $cm^{-1}$, 1681 $cm^{-1}$, 1640 $cm^{-1}$.

c)

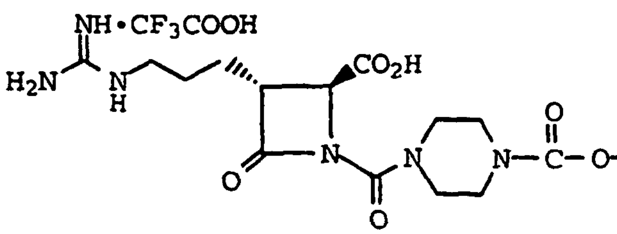

(homochiral)

Deprotection and purification of the product from part (b) (32 mg) according to the procedure of Example 19 (c) gives 17 mg of the desired product as a white fluffy powder. MS $(M+H)^+$ 427.1, $(M-H)^-$ 425.2; IR (KBr) 1792 $cm^{-1}$, 1670 $cm^{-1}$.

**Example 21**

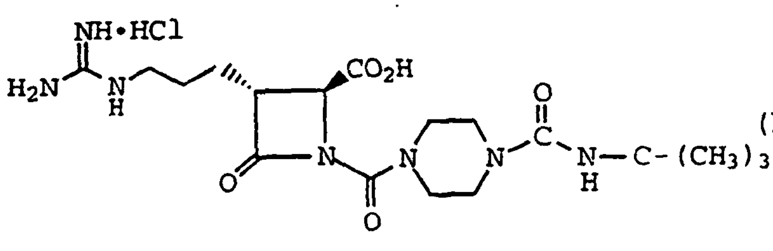

(homochiral)

a)

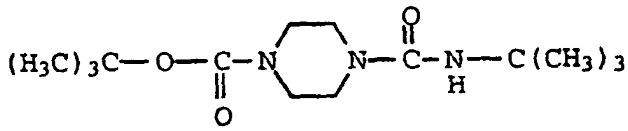

Tert-butyl isocyanate (0.28 g, 2.82 mmol) was added to a solution of a tert-butyl-1-piperazine carboxylate (0.5 g, 2.68 mmol) in methylene chloride (10 ml) and the mixture was stirred at room temperature. After 2 hours, the reaction mixture was evaporated *in vacuo*, suspended in water (50 ml) and extracted with ethyl acetate (2 x 50 ml). The organic phase was washed with saturated sodium chloride (1 x 50 ml) and filtered through a sintered glass funnel. The filtrate was dried over sodium sulfate and condensed to give 0.53 g of the desired product as a white solid.

b)

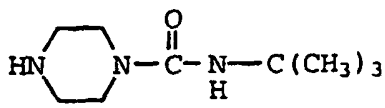

The product from part (a) (0.475 g, 1.67 mmol) was suspended in methylene chloride (4 ml) and trifluoroacetic acid (4 ml) was added over 1 minute. The mixture was stirred at room temperature. After 1 hour, the mixture was evaporated *in vacuo*. The residue was dissolved in water, the pH adjusted to 12 - 13 and extracted into ethyl acetate (2 x 25 ml). The organic phase was washed with saturated sodium chloride (1 x 50 ml), filtered, dried over sodium sulfate, and condensed to obtain 0.113 g of the desired product as a white solid.

c)

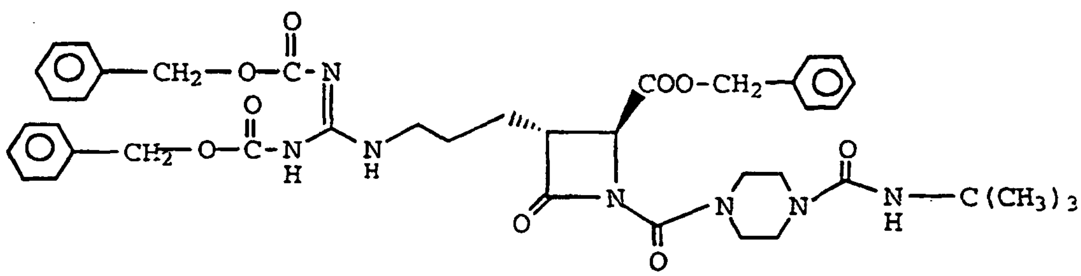

A solution of the benzyl ester product from Example 1(c) (65 mg, 0.114 mmol) in methylene chloride (1 ml) was cooled to -10°C and triethylamine (24 µl, 0.17 mmol) was added, followed by the addition of 20% phosgene in toluene (0.15 ml, 0.285 mmol). After 90 minutes at -10°C, the mixture was evaporated *in vacuo*. The residue was taken up in methylene chloride (1 ml) and cooled to -10°C. Triethylamine (24 µl, 0.17 mmol) was added, followed by the addition of the piperazine product from part (b) (21 mg, 0.114 mmol). The mixture was stirred at -10°C. After 1 hour, the mixture was quenched with 10% monobasic potassium phosphate (15 ml) and extracted with ethyl acetate (2 x 20 ml). The organic phase was washed with saturated sodium chloride (1 x 30 ml), dried over sodium sulfate, and concentrated to obtain a pale yellow oil. Purification by preparative HPLC (reverse phase, methanol, water, trifluoroacetic acid) gave 27 mg of the desired product as a white foam. MS 784.2 $(M+H)^+$; IR (film): 1787.1, 1741.9, 1636.6 $cm^{-1}$.

d)

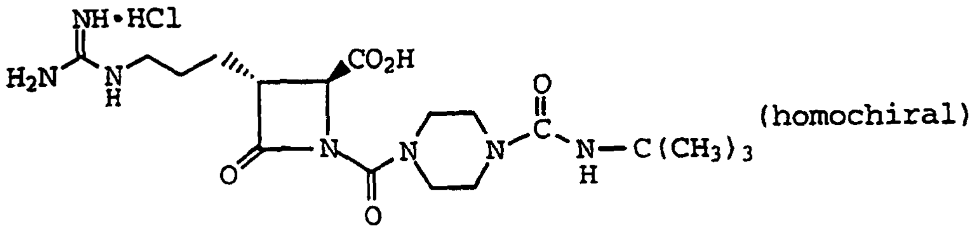

A solution of the product from part (c) (25 mg, 0.032 mmol) in 1,4-dioxane (7 ml) was treated with 1N HCl (40 µl, 0.038 mmol) and 10% palladium on carbon catalyst (15 mg). Hydrogen gas was bubbled through the mixture for 2 hours. The reaction mixture was filtered through a pad of Celite® which was then repeatedly washed with 1,4-dioxane. The combined eluents were lyophilized to give 15 mg of the desired product as a white lyophillate. MS 426.1 $(M+H)^+$, 424.3 $(M - H)^-$;IR (KBr) 1780 $cm^{-1}$.

**Example 22**

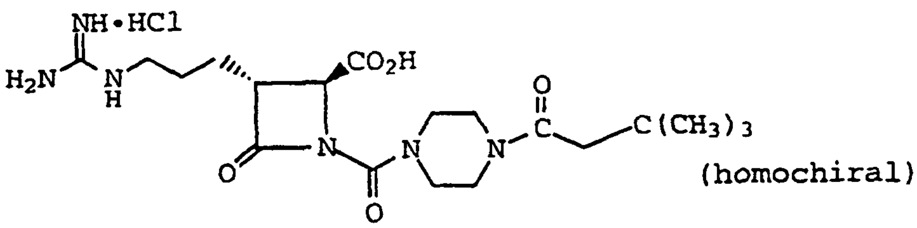

a)

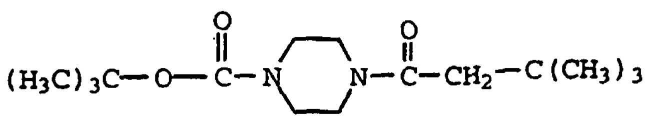

A solution of tert-butyl-1-piperazine carboxylate (0.5 g, 2.58 mmol) in methylene chloride (5 ml) was cooled to 0°C. N,N-Diisopropylethylamine (0.42 g, 3.22 mmol) and 4-dimethylaminopyridine (30 mg) were added, followed by the addition of tert-butyl acetyl chloride (0.36 g, 2.68 mmol) over 1 minute. The mixture was stirred at 0°C for 2 hours. After two hours, the mixture was partitioned between water (20 ml) and ethyl acetate (2 x 20 ml). The organic layer was washed with brine (1 x 75 ml), dried over sodium sulfate and condensed to give 0.763 g of the desired product as a white solid. MS 285.0 $(M + H)^+$.

b)

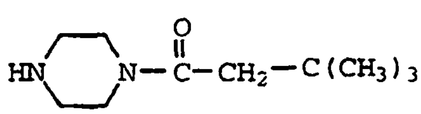

The product from part (a) (0.7 g, 2.46 mmol) was dissolved in methylene chloride (7 ml) and trifluoroacetic acid (4 ml) was added over 1 minute. The mixture was stirred at room temperature. After 1 hour, the mixture was evaporated *in vacuo*. The residue was dissolved in water, the pH was adjusted to 12 - 13 and extracted with ethyl acetate. The organic phase was washed with brine, dried over sodium sulfate and condensed to give 0.218 g of the desired product as a pale yellow oil. MS 184.9 $(M + H)^+$.

c)

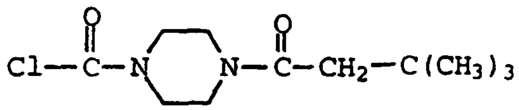

A solution of the product from part (b) (109 mg, 0.59 mmole) in methylene chloride (0.5 ml) was added to a mixture of phosgene (0.79 ml of 20% phosgene in toluene solution, 1.48 mmol) in methylene chloride (2 ml) at 0°C followed by the addition of triethylamine (82 µl, 0.59 mmol). The mixture was stirred at 0°C for 1 hour. The reaction mixture was then partitioned between water (25 ml) and ethyl acetate (2 x 25 ml). The organic phase was washed with 1N HCl (40 ml), brine (50 ml), dried over sodium sulfate and condensed to give a brown oil. Purification by flash chromatography (silica gel, 0 - 30% ethyl acetate/Hexane) gave 70 mg of the desired product.

d)

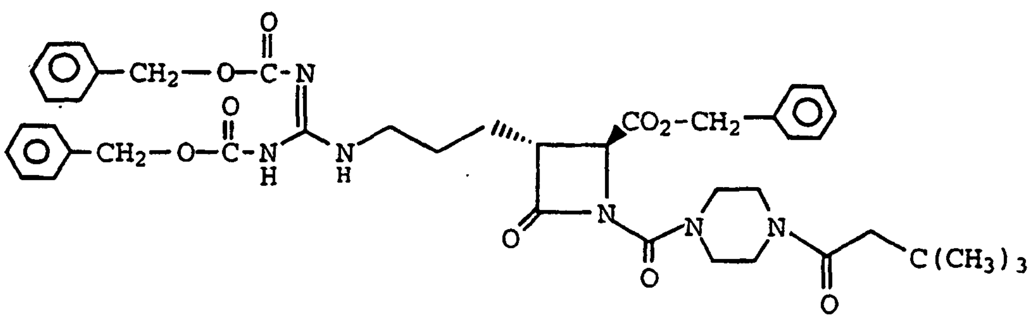

The product from part (c) (70 mg, 0.122 mmol) was dissolved in tetrahydrofuran (1 ml) and cooled to -78°C. Sodium bis (trimethylsilyl)amide (0.15 ml, 0.146 mmol) was added over one minute and the mixture was stirred at -78°C for 1 hour. A solution of the benzyl ester product from Example 1(c) (36 mg, 0.146 mmol) in tetrahydrofuran (0.5 ml) was added and the reaction mixture was stirred at -78°C. After 2.5 hours, the reaction mixture was quenched with 0.5 N potassium bisulfate solution (25 ml) and extracted with ethyl acetate (2 x 25 ml). The organic phase was washed with brine (1 x 50 ml), dried over sodium sulfate and concentrated to a yellow oil. Purification by preparative HPLC (reverse phase, methanol, water, trifluoroacetic acid) gave 21 mg of the desired product as a colorless oil. MS 783.4 $(M + H)^+$, 781.3 $(M - H)^-$.

e)

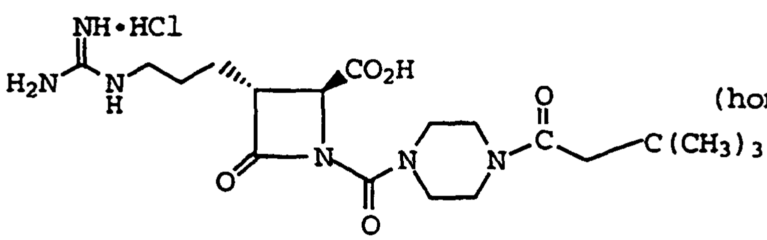

(homochiral)

The product from part (d) was deprotected and worked-up as described in Example 21(d) to give 12 mg of the desired product as a white lyophilate. MS 425.1 $(M+H)^+$, 423.3 $(M - H)^-$; IR (KBr) 1786, 1736 $cm^{-1}$.

**Example 23**

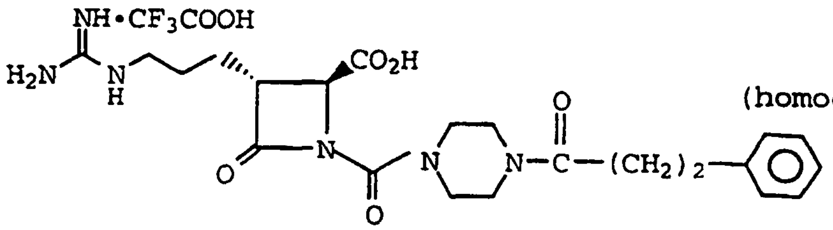

(homochiral)

a)

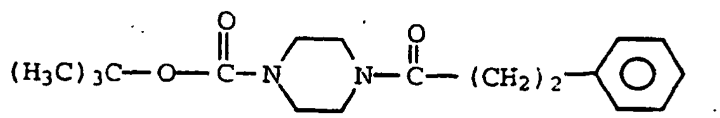

N,N-Diisopropylethylamine (560 µl), 4-dimethylaminopyridine (33 mg) and a solution of 3-phenylpropanoic acid chloride (400 µl, 2.69 mmol) in methylene chloride (2 ml) were added to a 0°C solution of tert-butyl-1-piperazine carboxylate (500 mg, 2.68 mmol) in methylene chloride (4 ml). The mixture was stirred at 0°C for 2 hours. The reaction was quenched with the addition of water (20 ml). The mixture was extracted with ethyl acetate (2 x 50 ml). The organic layers were combined and washed with brine (2 x 10 ml), dired over magnesium sulfate, and concentrated to give 872 mg of the desired product (crude) as a yellow solid. MS $(M+H)^+$ 319.1.

b)

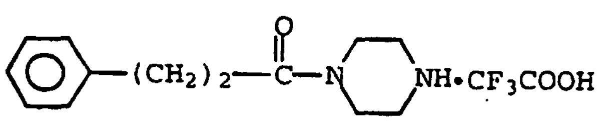

A mixture of the crude product from part (a) (860 mg, 2.68 mmol), trifluoroacetic acid (10 ml) and methylene chloride (10 ml) was stirred at room temperature for 1 hour. TLC showed the reaction was complete. The solvent was removed and 1N sodium hydroxide solution (15 ml) was added. The mixture was extracted with ethyl acetate (100 ml). The combined organic solution was washed with brine (10 ml), dried over magnesium sulfate, filtered and concentrated to give 238 mg of the desired product as a yellow oil which was used without further purification. IR(film) 1633 $cm^{-1}$.

c)

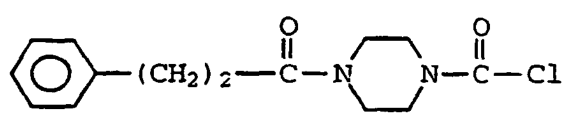

A mixture of the product from part (b) (200 mg, 0.92 mmol) and triethylamine (154 µl) in methylene chloride (4 ml) was added to a solution of phosgene in toluene (584 µl. 20%) at 0°C. The resulting mixture was stirred at 0°C for 20 minutes. TLC showed the completion of the reaction. The solvent was removed, and anhydrous ether (50 ml) was added. The mixture was filtered and the filtrate was concentrated to give the crude product as a yellow oil. Purification of the crude product by flash chromatography (50% ethyl acetate/hexanes) provided 235 mg of the desired product as a yellow oil. IR(film) 1741 $cm^{-1}$, 1703 $cm^{-1}$.

d)

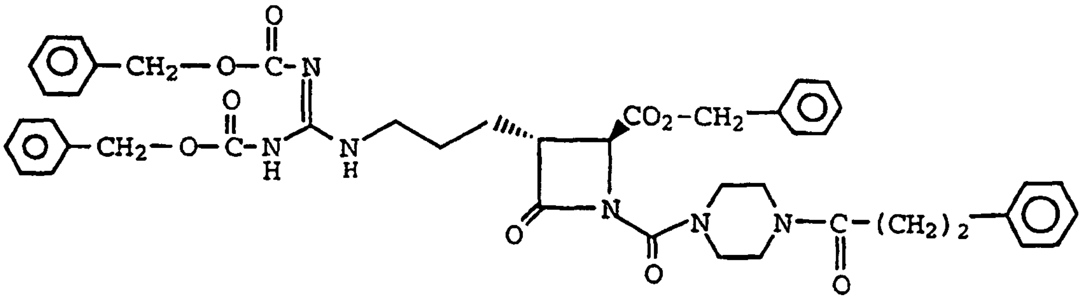

Sodium bis(trimethylsilyl)azide (1.0M in tetrahydrofuran, 210 µl, 0.21 mmol) was added dropwise to a -78°C solution of the benzyl ester product from Example 1(c) (100 mg, 0.17 mmol) in tetrahydrofuran (2 ml). The mixture was stirred at -78°C for 1 hour. A solution of the product from part (c) (58 mg, 0.21 mmol) in tetrahydrofuran (1 ml) was added. The reaction mixture was stirred at -78°C for 2.5 hours. Analytical HPLC indicated that the starting material was not completely consumed. This was quenched with the addition of 1N potassium bisulfate (20 ml). The mixture was extracted with ethyl acetate (2 x 30 ml). The organic layers were combined and washed with brine (2 x 15 ml) dried (magnesium sulfate), filtered and concentrated to give the crude product. Purification of the crude product by reverse phase HPLC provided 32 mg of the desired product as a colorless oil. MS $(M+H)^+$ 817.1, $(M-H)^-$ 815.4.

e)

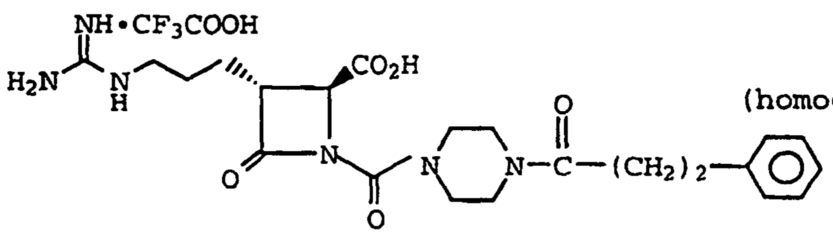

(homochiral)

Deprotection and purification of the product from part (d) according to the procedure of Example 19(c) gave 10 mg of the desired product as a white powder. MS $(M+H)^+$ 459.2, $(M-H)^-$ 457.4; IR (KBr) 1790 $cm^{-1}$, 1680 $cm^{-1}$.

## Example 24

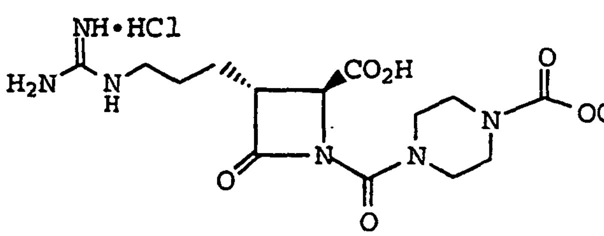

(homochiral)

a)

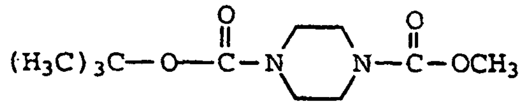

A solution of *tert*-butyl-1-piperazine carboxylate (0.5 g, 2.68 mmol) in methylene chloride (5 ml) was cooled to 0°C. N,N-Diisopropylethylamine (0.42 g, 3.22 mmol) and 4-dimethylaminopyridine (30 mg) were added, followed by addition of methyl chloroformate (0.25 g, 2.69 mmol) over 1 minute. The mixture was stirred at 0°C for 1 hour. The mixture was partitioned between water (20 ml) and ethyl acetate (2 x 20 ml). The organic phase was washed with brine (1 x 75 ml), dried over sodium sulfate and condensed to give the desired product as a cream colored solid (0.636 g).

b)

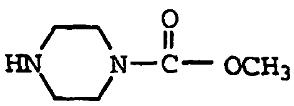

The product from part (a) (0.3 g, 1.23 mmol) was dissolved in methylene chloride (3 ml) and cooled to 0°C. Trifluoroacetic acid (3 ml) was added and the mixture was warmed to room temperature and stirred for 1 hour. The mixture was then evaporated *in vacuo*. The residue was dissolved in water, the pH adjusted to 12 - 13 with 6 N sodium hydroxide, and extracted with ethyl acetate. The organic phase was washed with brine, dried over sodium sulfate and condensed to give 91 mg of the desired " free amine product. IR(film) 1696.2 $cm^{-1}$; MS 144.9 $(M+H)^+$.

c)

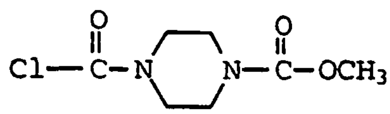

A mixture of the product from part (b) (89 mg, 0.617 mmol) and triethylamine (86 µl, 0.59 mmol) in methylene chloride (2 ml) was added to a mixture of phosgene (0.82 ml of a 20% phosgene in toluene solution, 1.54 mmol). The mixture was stirred at 0°C for 1 hour. The reaction mixture was then partitioned between water (25 ml) and ethyl acetate (2 x 25 ml). The organic phase was washed with 1N HCl (40 ml), brine (50 ml), dried over sodium sulfate and concentrated to give 108 mg of the desired product as a brown oil. IR (film) 1738.8, 1704.7 $cm^{-1}$.

d)

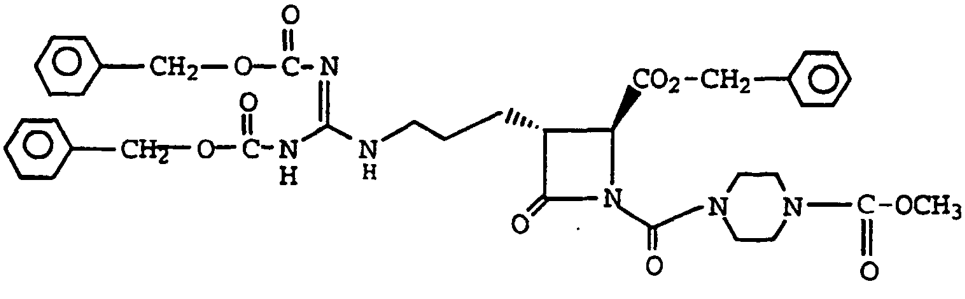

The benzyl ester product from Example 1(c) (111 mg, 0.194 mmol) was dissolved in tetrahydrofuran (2 ml)

and cooled to -78°C. Sodium bis(trimethylsilyl)amide (0.23 ml, 0.234 mmol) was added over 1 minute and the mixture was stirred at -78°C for 1 hour. A solution of the product from part (c) (48 mg, 0.234 mmol) in tetrahydrofuran (1 ml) was added and the reaction mixture was stirred at -78°C. After 1 hour, the mixture was quenched with 0.5 N potassium bisulfate solution (25 ml) and extracted with ethyl acetate (2 x 25 ml). The organic phase was washed with brine (1 x 50 ml), dried over sodium sulfate and concentrated to give a pale yellow oil. Purification by preparative HPLC (reverse phase, methanol, water, trifluoroacetic acid) gave 25 mg of the desired product as a colorless oil/ foam. MS 743.1 $(M+H)^+$, 741.4 $(M-H)^-$; IR(film) 1786.6 $cm^{-1}$.

e)

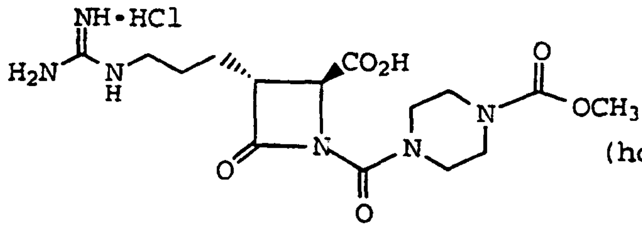

(homochiral)

Deprotection of the product from part (d) (22 mg, 0.027 mmol) and work-up as described in Example 21(d) gave 12 mg of the desired product as a white lyophilate. MS 385.1 $(M+H)^+$, 383.2 $(M-H)^-$; IR(film) 1786 $cm^{-1}$.

**Example 25**

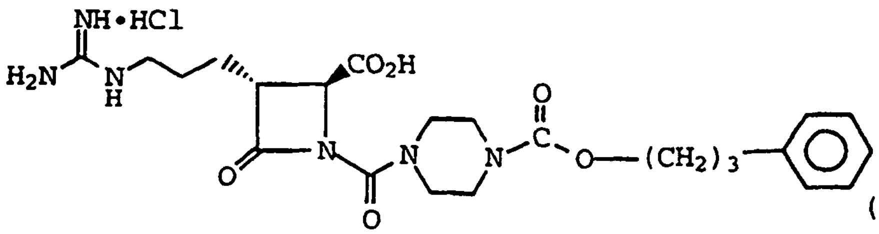

(homochiral)

a)

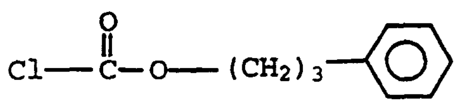

3-Phenyl-1-propanol (1.09 g, 7.34 mmol) was added to a mixture of phosgene (5.5 ml of 20% phosgene in toluene solution, 11.01 mmol) in methylene chloride (5 ml) at 0°C. The mixture was stirred at 0°C for 5 hours. The reaction mixture was evaporated *in vacuo* to give a colorless oil. Purification by flash column chromatography (silica gel, 0 - 5% ethyl acetate/Hexane gave 1.33 g of the desired product.

b)

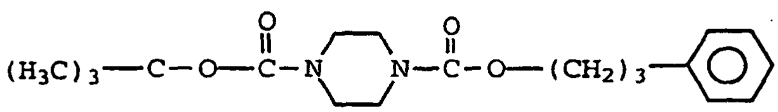

A solution of *tert*-butyl-1-piperazine carboxylate (0.2, 1.07 mmol) in methylene chloride (3 ml) was cooled to

0°C. N,N-Diisopropylethylamine (0.25 g, 1.93 mmol) and 4-dimethylaminopyridine (5 - 7 crystals) were added, followed by addition of a solution of the product from part (a) (0.2 g, 1.07 mmol) over 1 minute. The mixture was stirred at 0°C for 1 hour. The mixture was then partitioned betweeen water (20 ml) and ethyl acetate (2 x 20 ml). The organic layer was washed with brine (1 x 75 ml), dried over sodium sulfate and concentrated to give 0.35 g of the desired product as a yellow oil.

c)

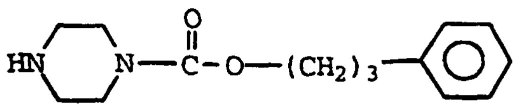

The product from part (b) (0.35 g, 1.03 mmol) was dissolved in methylene chloride (4 ml) and cooled to 0°C. Trifluoroacetic acid (4 ml) was added over 1 minute and the mixture was warmed to room temperature and stirred for 1 hour. The mixture was then evaporated *in vavuo*. The residue was dissolved in water, the pH was adjusted to 12 - 13 using 6 N sodium hydroxide and extracted with ethyl acetate. The organic phase was washed with brine, filtered over sodium sulfate and concentrated to give 0.23 g of the desired product as a white solid. MS 248.9 $(M + H)^+$.

d)

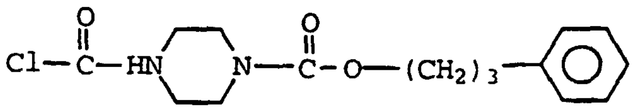

A solution of the product from part (c) (100 mg, 0.403 mmol) in methylene chloride (1 ml) was added to a mixture of phosgene (0.53 ml of a

20% phosgene in toluene solution, 1.01 mmol) followed by the addition of triethylamine (60 µl, 0.403 mmol). The mixture was stirred at 0°C for 1

hour. The reaction mixture was then partitioned between water (25 ml)

and ethyl acetate (2 x 25 ml). The organic phase was washed with 1N HCl (40 ml), brine (50 ml), dried over sodium sulfate and concentrated to give 115 mg of the desired product.

e)

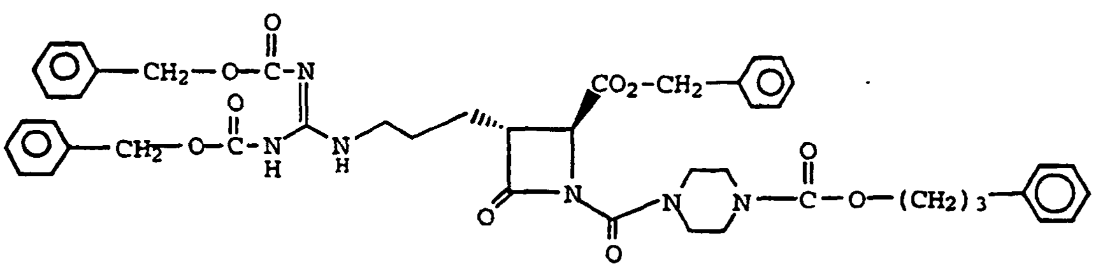

A solution of the benzyl ester product from Example 1(c) (43 mg, 0.075 mmol) in methylene chloride (1 ml) was cooled to 0°C and triethylamine (11 mg, 0.113 mmol) and 4-dimethylaminopyridine (6-8 crystals) were added. A solution of the product from part (d) (58 mg) in methylene chloride (0.5 ml) was added and the mixture was stirred at 0°C for 45 minutes followed by stirring at room temperature for 3 hours. The mixture was then evaporated *in vacuo*. Purification of the residue by flash column chromatography (silica gel, 30% ethyl acetate/hexane) gave 40 mg of the desired product as a pale yellow oil. MS 847.1 $(M + H)^+$, 845.4 (M - H)-; IR (film) 1784 $cm^{-1}$.

f)

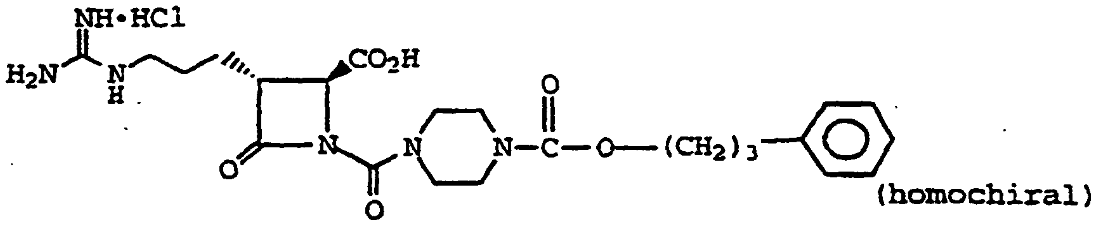

Deprotection of the product from part (e) (40 mg, 0.027 mmol) and work-up as described in Example 21(d) gave 21 mg of the desired product as a white lyophilate. MS 489.1 $(M + H)^+$, 487.4 $(M - H)^-$; IR (KBr) 1784, 1667 $cm^{-1}$.

**Example 28**

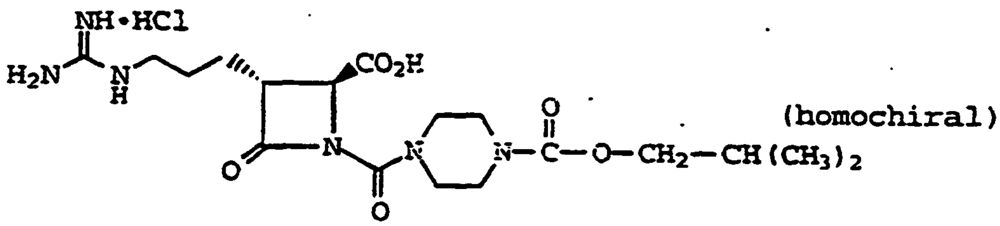

a)

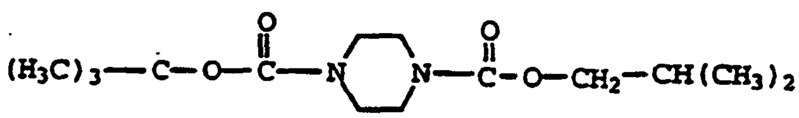

Diisopropylethylamine (30 mg), 4-dimethylaminopyridine (30 mg) and a solution of isobutyl chloroformate (366 µl, 268 mmol) in methylene chloride (2 ml) were added to a 0°C solution of *tert*-butyl-1-piperazine carboxylate (500 mg, 2.68 mmol) in methylene chloride (2 ml). The mixture was stirred at 0°C for 1 hour. The reaction was quenched with the addition of water (20 ml). The mixture was extracted with ethyl acetate (2 x 50 ml). The organic layers were combined and washed with brine (2 x 10 ml), dried over magnesium sulfate, and concentrated to give 819 mg of the desired product as a yellow solid; IR(film) 1701 $cm^{-1}$, 1688 $cm^{-1}$.

b)

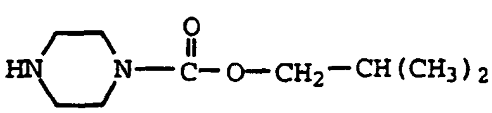

A mixture of the crude product from part (a) (800 mg, 2.79) mmol), trifluoroacetic acid (10 ml) and methylene chloride (10 ml) was stirred at room temperature for 1 hour. TLC showed the completion of the reaction. The solvent was removed and 1N sodium hydroxide solution (15 ml) was added. The mixture was extracted with ethyl acetate (100 ml). The combined organic solution was washed with brine (10 ml), dried over magnesium sulfate, filtered and concentrated to give 511 mg of the desired product as a yellow oil which was used without further purification. IR(film) 1692 $cm^{-1}$.

c)

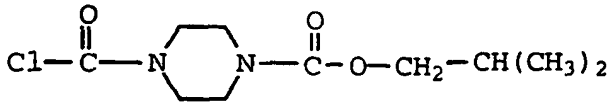

A mixture of the product from part (b) (477 mg, 2.56 mmol) and triethylamine (432 µl) in methylene chloride (5 ml) was added to a solution of phosgene in toluene (1.6 ml, 20%) at 0°C. The resultant mixture was stirred at 0°C for 2 hours. TLC showed the completion of the reaction. The solvent was removed, and anhydrous ether (50 ml) was added. The mixture was filtered and the filtrate was concentrated to give the crude product (481 mg) as an orange oil. Purification of the crude product provided 453 mg of the desired product as a yellow oil. IR (film) 1741 $cm^{-1}$, 1703 $cm^{-1}$.

d)

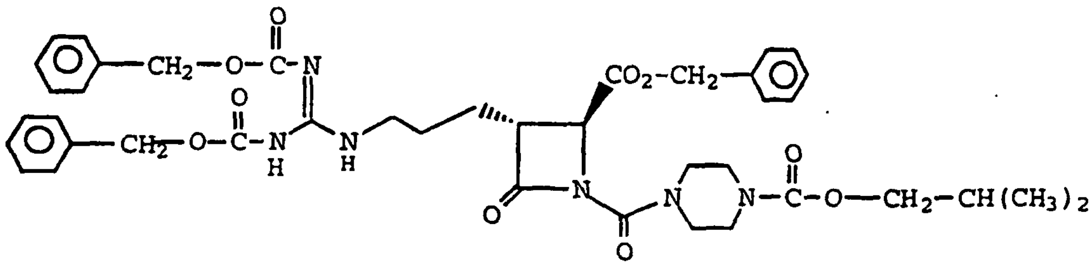

Triethylamine (36 µl), 4-dimethylaminopyridine (30 mg) and a solution of the product from step (c) (65 mg, 0.24) in methylene chloride (1 ml) were added to a solution of the benzyl ester product from Example 1(c) (100 mg, 0.17 mmol) in methylene chloride (1 ml). The mixture was stirred for 4 hours at room temperature. Analytical HPLC indicated that the reaction was complete. The reaction was quenched by the addition of 1N potassium bisulfate (15 ml). The mixture was extracted with ethyl acetate (100 ml). The organic layer was washed with brine (15 ml), dried over magnesium sulfate, and concentrated to give the crude product as a yellow oil. Purification by flash chromatography (50% ethyl acetate/hexane) gave 81 mg of the desired product. MS 785.2 $(M+H)^+$, 783.4 $(M-H)^-$.

e)

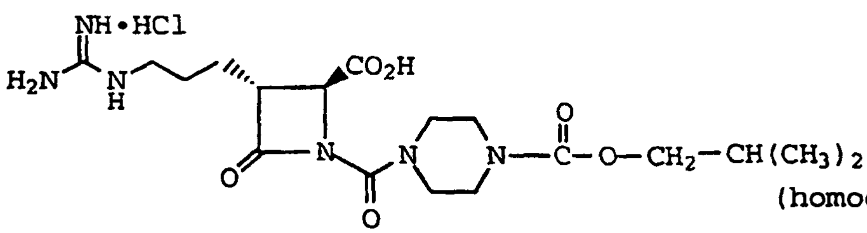

(homochiral)

The product from part (d) (80 mg, 0.10 mmol) was deprotected and worked-up as described in Example 21 (d) to give 41 mg of the desired product as a white solid. MS $(M+H)^+$ 427.1, (M-H)- 425.3; IR (KBr) 1786 $cm^{-1}$, 1653 $cm^{-1}$.

**Example 29**

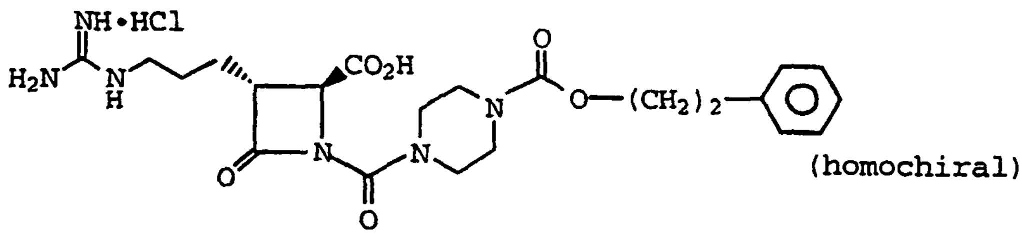

a)

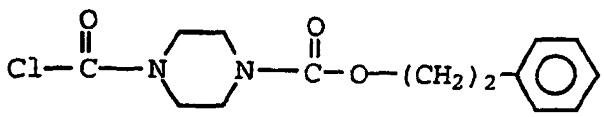

Following the procedure of Example 25(a) through (d) but substituting phenethyl alcohol for the 3-phenyl-1-propanol in (a), the desired product was obtained as an orange oil.

b)

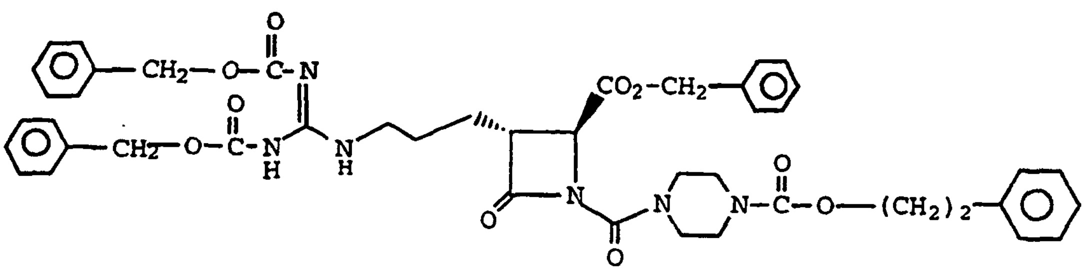

A solution of the benzyl ester product from Example 1(c) (69 mg, 0.121 mmol) in methylene chloride (1 ml) was cooled to 0°C and triethylamine (18 mg, 0.181 mmol) and 4-dimethylaminopyridine (6 - 8 crystals) were added. A solution of the product from part (a) (54 mg) in methylene chloride (1 ml) was added and the mixture was stirred at 0°C for 45 minutes followed by stirring at room temperature for 2.5 hours. The mixture was then evaporated *in vacuo*. Purification of the residue by flash chromatography (silica gel, 0 - 30% ethyl acetate/Hexane) gave 90 mg of the desired product as a colorless oil. MS 833.1 $(M+H)^+$, 831.4 $(M-H)^-$; IR (film) 1786, 1737.7 $cm^{-1}$.

c)

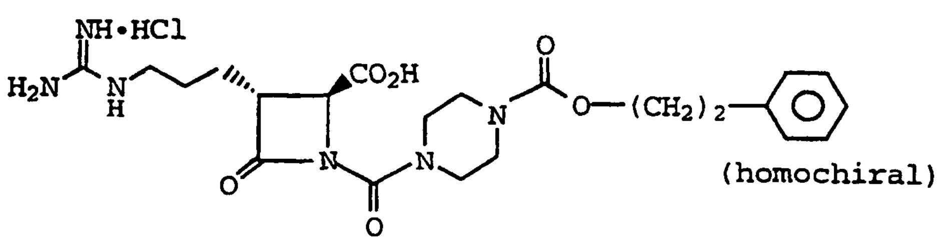

The product from part (b) (85 mg, 0.108 mmol) was deprotected and worked-up as described in Example 21 (d) to give 34 mg of the desired product as a white lyophilate. MS 475.1 $(M+H)^+$, 473.4 $(M-H)^-$; IR (film) 1783 $cm^{-1}$. 1665 $cm^{-1}$.

**Example 30**

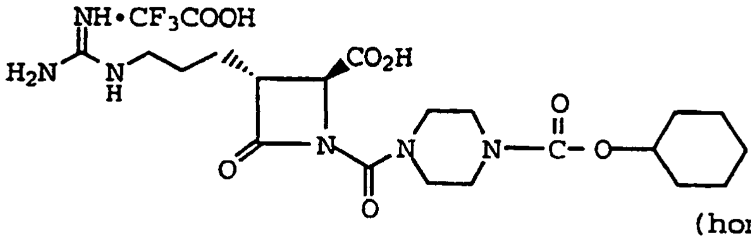

(homochiral)

a) Cyclohexyl chloroformate

A mixture of cyclohexanol (500 mg, 5.0 mmol) and triethylamine (836 µl) in methylene chloride (4 ml) was added to a 0°C solution of phosgene in toluene (5.3 ml, 20%). The resultant mixture was stirred at 0°C for 2.5 hours. TLC showed completion of the reaction. The solvent was removed and anhydrous ether (50 ml) was added. The mixture was filtered and the filtrate was concentrated to give 726 mg of the desired product as a colorless oil which was used without further purification. IR (film) 1776 $cm^{-1}$.

b)

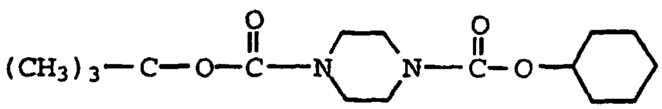

Diisopropylethylamine (560 µl), 4-dimethylaminopyridine (30 mg) and a solution of cyclohexyl chloroformate (473 mg, 2.68 mmol) in methylene chloride (2 ml) were added to a 0°C solution of *tert*-butyl-1-piperazine carboxylate (500 mg, 2.68 mmol) in methylene chloride (3 ml). The mixture was stirred at 0°C and warmed to room temperature over 4 hours. The reaction was quenched with the addition of water (15 ml). The mixture was extracted with ethyl acetate (100 ml). The organic layer was washed with brine (2 x 10 ml), dried over magnesium sulfate, and concentrated to give 832 mg of the desired product as a white solid. IR(film) 1692 $cm^{-1}$.

c)

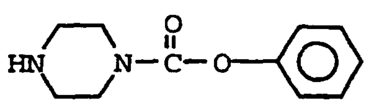

A mixture of the crude product from part (b) (832 mg, 2.68 mmol), trifluoroacetic acid (2 ml) and methylene chloride (2 ml) was stirred at room temperature for 3 hours. TLC showed completion of the reaction. The solvent was removed and 1N sodium hydroxide solution (15 ml) was added. The mixture was extracted with ethyl acetate (100 ml). The combined organic solution was washed with brine (10 ml), dried over magnesium sulfate, filtered and concentrated to give 535 mg of the desired product as a light yellow oil which was used without further purification.

d)

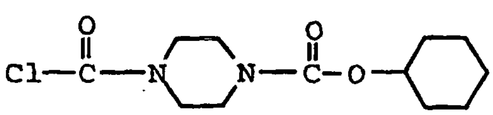

A mixture of the product from part (c) (459 mg) and triethylamine (448 µl) in methylene chloride (2 ml) was added to a solution of phosgene in toluene (2.1 ml, 20%) at 0°C. The resultant mixture was stirred at 0°C for 1 hour. TLC showed the completion of the reaction. The solvent was removed and anhydrous ether (50 ml) was

added. The mixture was filtered and the filtrate was concentrated to give the crude product (626 mg) as an orange oil. Purification of the crude product provided 626 mg of the desired product as a yellow solid. IR (film) 1740 $cm^{-1}$ 1697 $cm^{-1}$.

e)

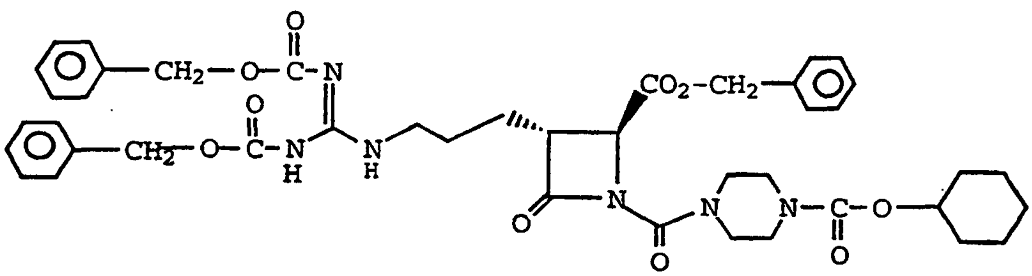

Triethylamine (34 µl), 4-dimethylaminopyridine (20 mg) and a solution of the product from part (d) (65 mg, 0.24 mmol) in methylene chloride (1 ml) was added to solution of the benzyl ester product from Example 1(c) (113 mg, 0.14 mmol) in methylene chloride (1 ml). The mixture was stirred at room temperature for 2 hours. Analytical HPLC showed the reaction was complete. The reaction was quenched with the addition of 1N potassium bisulfate (15 ml). The mixture was extracted with ethyl acetate (100 ml). The organic layer was washed with brine (15 ml), dried (magnesium sulfate) and concentrated to give the crude product as a colorless oil. Purification by flash chromatography (50% ethyl acetate/hexane) gave 113 mg of the desired product. IR (film) 1786 $cm^{-1}$, 1734 $cm^{-1}$, 1683 $cm^{-1}$, 1639 $cm^{-1}$.

f)

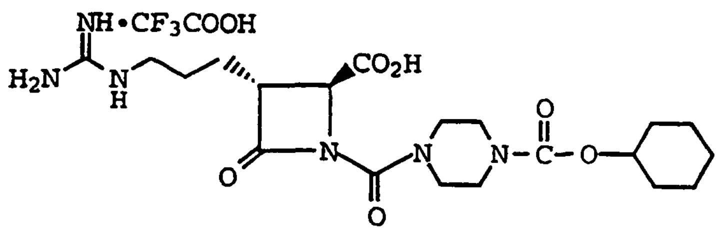

(homochiral)

Deprotection and purification of the product from part (e) (113 mg, 0.14 mmol) according to the procedure of Example 19(c) gives 33 mg of the desired product as a white solid. MS $(M+H)^+$ 453.3, $(M-H)^-$ 451.5; IR (KBr) 1790 $cm^{-1}$, 1674 $cm^{-1}$.

**Example 31**

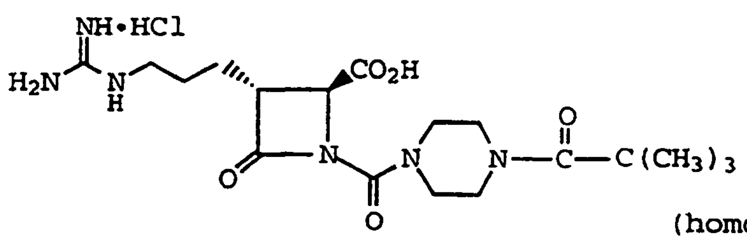

(homochiral)

a)

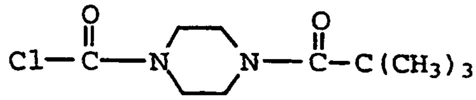

Following the procedure of Example 22(a) through (c) but substituting *tert*-butylcarbonyl chloride for the *tert*-butyl acetylchloride in part (a), the desired product was obtained as a pale brown solid. IR (film) 1733.2, 1616.4 $cm^{-1}$.

b)

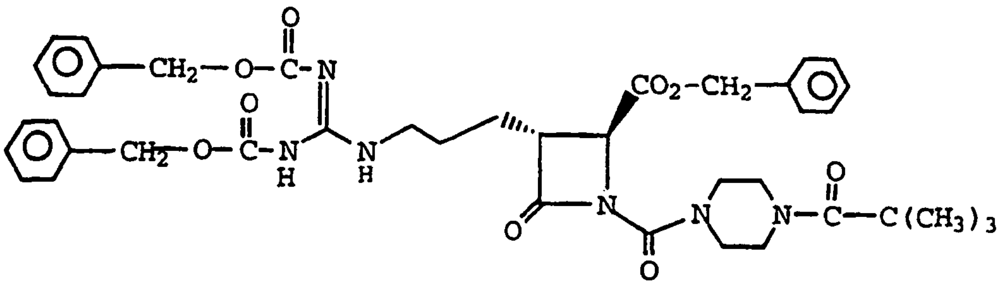

Triethylamine (24 mg, 0.236 mmol) and 4-dimethylaminopyridine (8 - 10 crystals) were added to a solution of the benzyl ester product from Example 1(c) (90 mg, 0.157 mmol) in methylene chloride (1 ml). A solution of the product from part (a) (59 mg, 0.236 mmol) in methylene chloride (1 ml) was added and the mixture was stirred at 0°C for 30 minutes followed by stirring at room temperature for 6 hours. The mixture was then evaporated *in vacuo*. Purification of the residue by flash chromatography (silica gel, 0 - 30% ethyl acetate/Hexane) gave 89 mg of the desired product as a colorless. MS 769.4 $(M+H)^+$, 767.6 $(M-H)^-$; IR (film) 1785.4, 1733.4, 1679.4, 1635.9 $cm^{-1}$.

c)

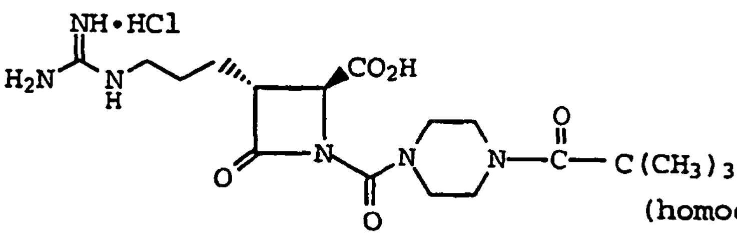

(homochiral)

The product from part (b) (87 mg, 0.11 mmol) was deprotected and worked-up as described in Example 21(d) to give 10 mg of the desired product as a white solid lyophilate. MS 411.2 $(M+H)^+$, 409.5 $(M-H)^-$; IR (KBr) 1788.0, 1742.0 $cm^{-1}$.

**Example 32**

The product of Example 21 was also prepared as follows:

a)

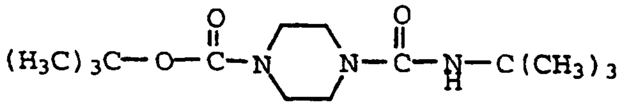

A solution of tert-butylisocyanate (10.28 g, 103 mmol) in methylene chloride (20 ml) was added over 2 minutes to a solution of tert-butyl-1-piperazine carboxylate (9.17 g, 49.2 mmol) in methylene chloride (40 ml) at 0°C under nitrogen. After stirring the reaction mixture at room temperature for 2 hours, the reaction mixture was poured into

hexane (60 ml). The resulting precipitate was collected by filtration and washed with hexane/methylene chloride (2:1) (2 x 50 ml). The combined eluent was concentrated to approximately a 20 ml volume and the precipitate that formed was collected by filtration, washed as above and combined with previously collected solid. The solid was dried under vacuum to give 14.1 g of the desired product. MS 286.2 $(M+H)^+$.

b)

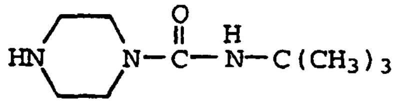

Trifluoroacetic acid (25 ml) was added dropwise over 5 minutes to a solution of the product from part (a) (14.1 g, 49 mmol) in methylene chloride (25 ml) at 0°C under nitrogen. The reaction mixture was stirred at 0°C for 20 minutes and then at room temperature for 2 hours. The reaction mixture was transferred to a beaker (100 ml) and diluted with ethyl acetate (200 ml) and water (200 ml). While vigorously stirring the biphasic mixture sodium hydroxide (25% aqueous) was added dropwise until the pH of the aqueous phase was about 12. The organic phase was separated with an additional portion of ethyl acetate (200 ml). The combined organics were dried over sodium sulfate, filtered and concentrated to give 11 g of the desired product as a white solid.

c)

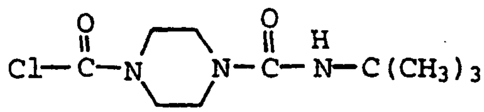

A solution of the product from part (b) (about 11 g) in methylene chloride: acetonitrile (1:1, 40 ml) was added dropwise over 10 minutes to a solution of phosgene (20% in toluene, 70 ml, 132 mmol) at 0°C under nitrogen. Triethylamine (30 ml) was then added dropwise over 5 minutes. The reaction mixture was then stirred at room temperature for 2 hours. The reaction mixture was transferred to a separatory funnel, diluted with ethyl acetate (150 ml) and washed with 2N HCl (2 x 150 ml). The organics were dried over sodium sulfate, filtered and concentrated to a yellow oil. Purification by flash chromatrography (silica gel, 0 to 50% ethyl acetate in hexane) provided 7.8 g of the desired product.

d)

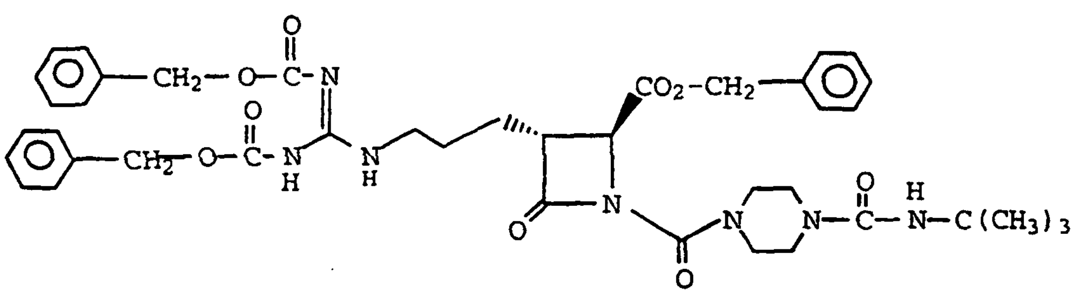

The carbamoyl chloride product from part (c) (4.5 g, 18.2 mmol), triethylamine (2.6 ml, 18.2 mmol) and dimethylaminopyridine (225 mg) were added to a solution of the benzyl ester product from Example 1(c) (6.94 g, 12.13 mmol) in methylene chloride (50 ml) at room temperature under nitrogen. After stirring the reaction mixture at room temperature for 4 hours, additional portions of the acid chloride product from part (c) (1g, 4 mmol) and triethylamine (1 ml, 7 mmol) were added. The reaction was stirred for an additional 3 hours. The reaction was diluted with hexane (5 ml) and the crude reaction mixture was loaded onto a silica column (wetted with hexane) for purification by flash chromatography (0 to 60% ethyl acetate in hexane) to provide 7.46 g of the desired product. MS 784.4 $(M+H)^+$, 782.2 $(M-H)^-$.

e)

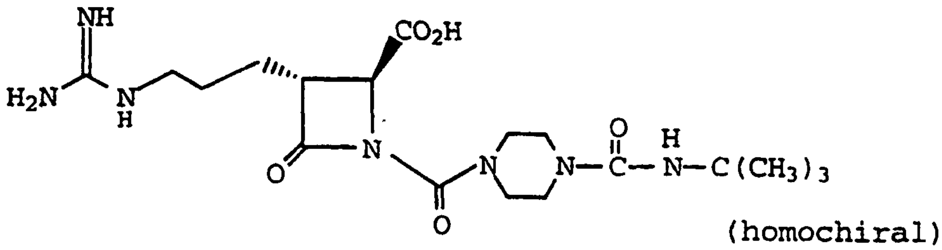

Water (50 ml), concentrated HCl (0.8 ml, 9.6 mmol) and 10% palladium on carbon catalyst (7.5 g, 50% water content) were added to a solution of the product from part (d) (7.46 g, 9.57 mmol) in dioxane (125 ml) at room temperature under nitrogen. Hydrogen was bubbled through the solution for 30 minutes and then the reaction was stirred under hydrogen (1 atmosphere) for 11 hours. The reaction was filtered through a Celite® pad which was washed with water (about 100 ml) until no product could be detected in the eluent. The solution was frozen and lyophilized to give 4.5 g of a white solid. Purification by HPLC (reverse phase, methanol, water, trifluoroacetic acid), subsequent lyophilization, filtration through polyvinylpyridine with a water mobile phase, and final lyophilization proved 3.3 g of the desired product as a voluminous white solid. MS 426.2 $(M + H)^+$, 424.4 $(M - H)^-$; IR (KBr) 1777 $cm^{-1}$.

| Anal. calc'd for $C_{18}H_{31}N_7O_5 \cdot 1.56\ H_2O$: | | | | |
|---|---|---|---|---|
| | C, 47.66; | H, 7.58; | N, 21.62; | O, 23.14 |
| Found: | C,47.58, | H,7.37; | N, 21.41. | |

**Example 33**

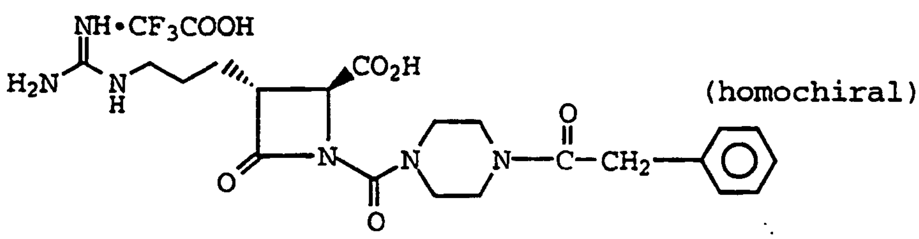

a)

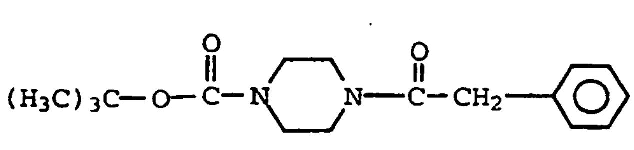

Following the procedure of Example 23(a) but substituting phenylacetyl chloride for the 3-phenylpropanoic acid chloride, the desired compound was obtained as a yellow solid.

b)

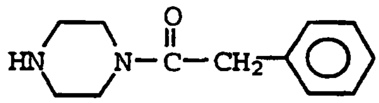

A mixture of the product from part (a) (2.68 mmol), trifluoroacetic acid (10 ml) and methylene chloride (10 ml) was stirred at room temperature for 90 minutes. TLC showed the completion of the reaction. The solvent was removed and 1N sodium hydroxide solution (10 ml) was added. The mixture was extracted with ethyl acetate (100 ml). The organic solution was washed with brine (20 ml), dried (magnesium sulfate), filtered and concentrated to give 536 mg of desired product as a colorless oil which was used without further purification. IR (film) 1630 $cm^{-1}$.

c)

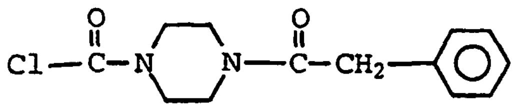

A mixture of the product from part(b) (113 mg, 0.55 mmol) and triethylamine (92 μl) in methylene chloride (1 ml) was added to a solution of phosgene (351 μl, 20% in toluene) in methylene chloride (1 ml) at 0°C. The resultant mixture was stirred at 0°C for 2 hours and worked-up according to the procedure of Example 23(c) to give 74 mg of the desired product as a yellow solid. IR(film) 1735 $cm^{-1}$, 1645 $cm^{-1}$.

d)

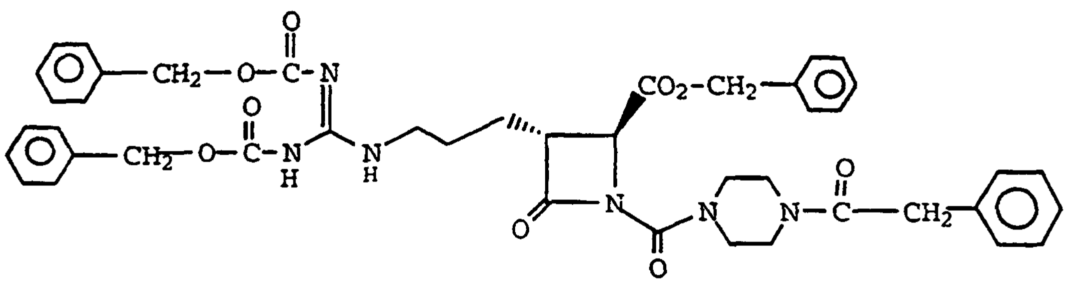

Triethylamine (34 μl), 4-dimethylaminopyridine (30 mg) and a solution of the product from part (c) (74 mg, 0.28 mmol) in methylene chloride (2 ml) were added to a solution of the benzyl ester product from Example 1(c) (113 mg, 0.20 mmol) in methylene chloride (1 ml). The mixture was stirred at room temperature for 2 hours. Analytical HPLC indicated that the reaction was complete. The reaction was quenched with the addition of 1N potassium sulfate (10 ml). The mixture was extracted with ethyl acetate (100 ml). The organic layer was washed with brine (15 ml), dried (magnesium sulfate) and concentrated to give the crude product as a colorless oil. Purification using flash chromatography (30 - 50% ethyl acetate/hexane) gave 73 mg of the desired product. MS $(M+H)^+$ 803.4, $(M-H)^-$ 801.5; IR (film) 1785 $cm^{-1}$, 1733 $cm^{-1}$, 1733 $cm^{-1}$, 1677 $cm^{-1}$, 1640 $cm^{-1}$.

e)

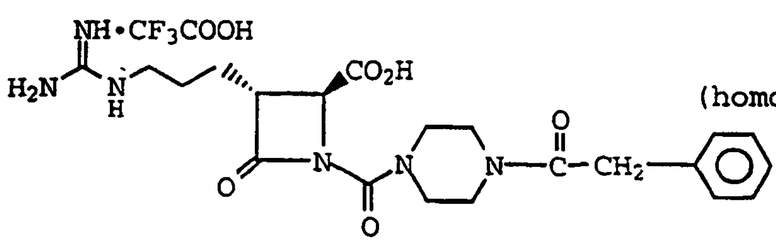

(homochiral)

The product from part (d) (67 mg, 0.83 mmol) was deprotected and worked-up according to the procedure of

Example 19(c) to give 5 mg of the desired product as a white solid. MS $(M+H)^+$ 445.2, (M-H)- 443.4; IR (film) 1782 $cm^{-1}$, 1677 $cm^{-1}$.

**Example 34**

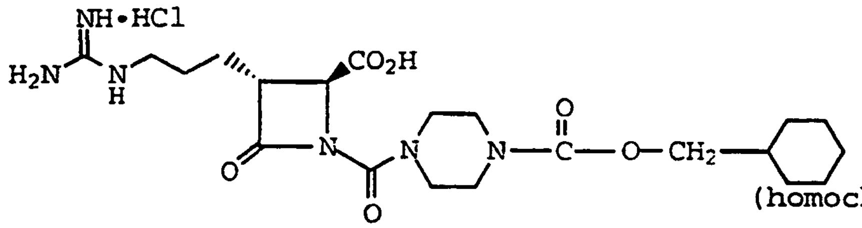

(homochiral)

a)

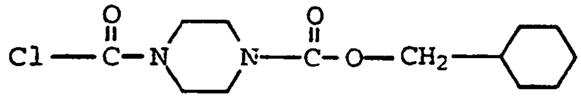

Following the procedure of Example 30 (a) through (d) but substituting cyclohexylmethanol for the cyclohexanol in step (a), the desired compound was obtained as a yellow oil. IR (film) 1743 $cm^{-1}$, 1702 $cm^{-1}$.

b)

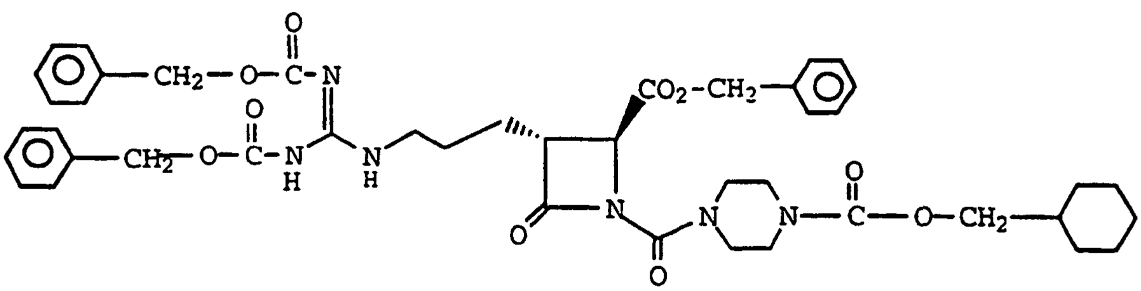

The product from part (a) is reacted with the benzyl ester product from Example 1(c) according to the procedure of Example 30 (e) to give the desired product as a colorless oil. IR (film) 1786 $cm^{-1}$, 1732 $cm^{-1}$, 1680 $cm^{-1}$, 1639 $cm^{-1}$.

c)

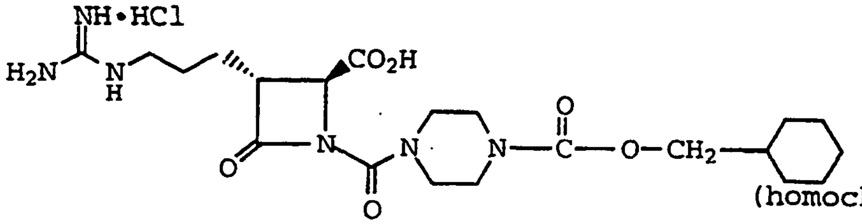

(homochiral)

The product from part (b) was deprotected and worked-up as described in Example 21(d) to give the desired product as a white solid. MS $(M+H)^+$ 467.3, $(M-H)^-$ 465.5; IR (KBr) 1778 $cm^{-1}$, 1541 $cm^{-1}$.

**Example 35**

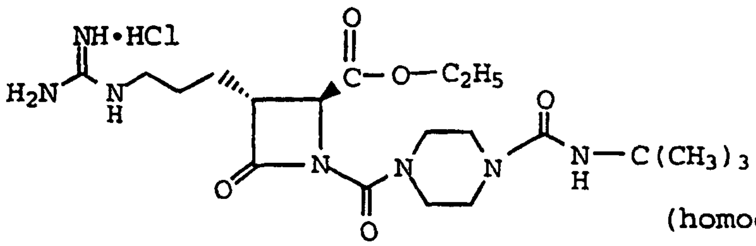

(homochiral)

a)

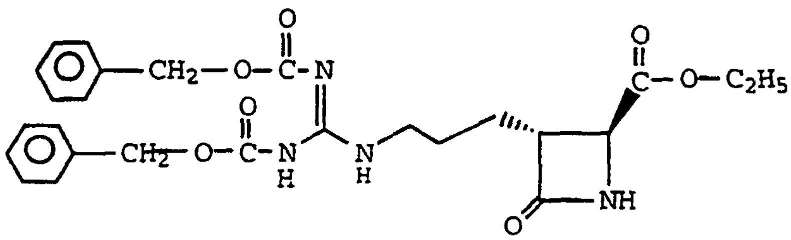

Cesium carbonate (14 mg, 0.042 mmol) was added to a stirred solution of the azetidinone product of Example 1(b) (40 mg, 0.083 mmol) and iodoethane (27 µl, 0.332 mmol) in dimethylformamide (200 µl) at room temperature. After 3 hours, the reaction mixture was partitioned between ethyl acetate and water containing a small amount of sodium thiosulfate. The organic phase was isolated, washed with saturated sodium chloride, dried over magnesium sulfate, and concentrated. The residue was purified by silica gel chromatography to afford 33 mg of the desired product.

b)

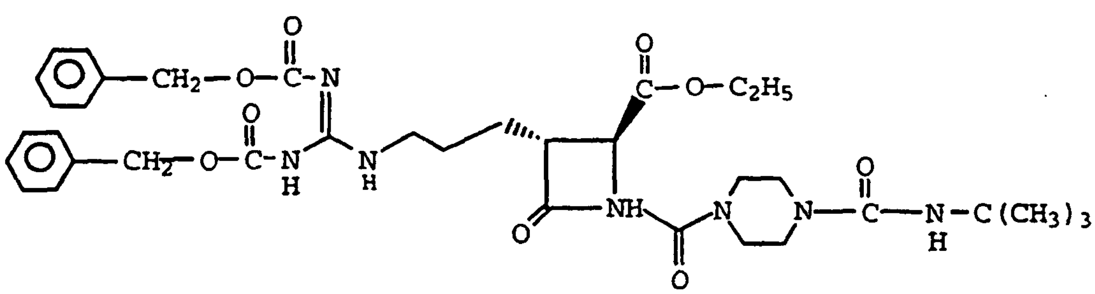

The product from part (a) (86 mg, 0.168 mmol) and the piperazinyl carbamoyl chloride of the formula

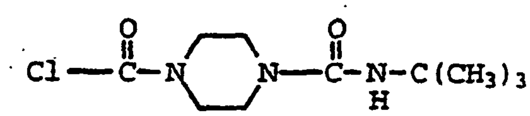

(56 mg, 0.227 mmol) [prepared as described in Example 32(c)] were dissolved in methylene chloride (1.80 ml) and tetrahydrofuran (0.20 ml). Triethylamine (35 µl, 0.252 mmol) was added followed by 4-dimethylaminopyridine (4.0 mg, 0.034 mmol). After 48 hours the reaction was concentrated and the crude product was purified by silica

gel chromatography to give 71 mg of the desired product.

c)

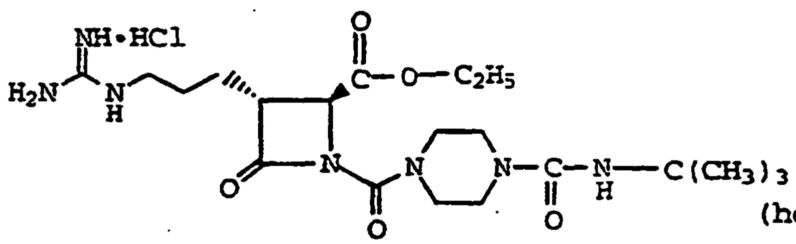

(homochiral)

The product from part (b) was deprotected and worked-up according to the procedure described in Example 21(d) to give the desired product as a lyophilate. IR(KBr) 1788 $cm^{-1}$.

**Example 54**

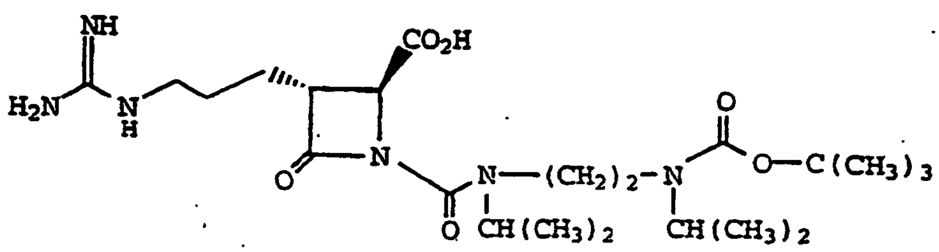

(homochiral)

a)

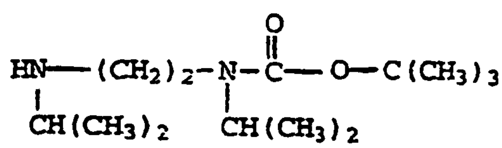

A mixture of di-tert-butyl dicarbonate (1.51 g, 6.9 mmol) and triethylamine (0.7 g, 6.9 mmol) in anhydrous tetrahydrofuran (3 ml) was added over 20 minutes to a solution of N,N'-diisopropylethylenediamine (1.0g, 6.9 mmol) in anhydrous tetrahydrofuran (2 ml). The mixture was stirred at room temperature for 2 hours. The mixture was then filtered and washed with methylene chloride. The filtrate and washings were condensed to obtain a colorless oil. Purification by flash column chromatography (silica gel, 1 - 5% 2M ammonia in methanol/methylene chloride) gave 50 mg of the desired product as a colorless oil. MS 245.2 $(M+H)^+$.

b)

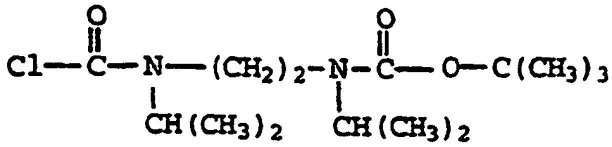

The product from part (a) (44 mg, 0.18 mmol) was added to a mixture of phosgene (0.24 ml of a 20% phosgene

in toluene solution, 0.45 mmol) in methylene chloride (1 ml) at 0°C followed by the addition of triethylamine (25 µl, 0.18 mmol). The mixture was stirred at 0°C for 1 hour. The reaction mixture was evaporated *in vacuo*. The residue was purified by flash chromatography (silica gel, 0 - 10% ethyl acetate/hexane) to give about 30 mg of the desired product as a colorless oil. IR(neat) 1732.0, 1694.5 $cm^{-1}$.

c)

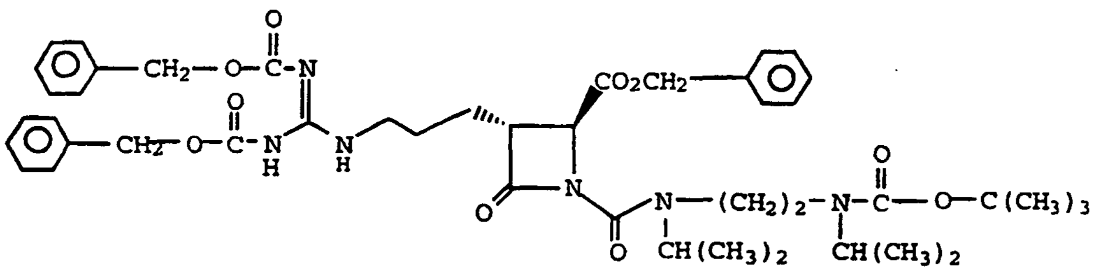

Triethylamine (15 µl, 0.104 mmol) and dimethylaminopyridine (10 - 12 crystals) were added to a solution of the benzyl ester product from Example 1(c) (40 mg, 0.07 mmol) in methylene chloride (1 ml) followed by the addition of the acid chloride product from part (b) (25 mg, 0.084 mmol). The mixture was stirred for 48 hours and then evaporated in *vacuo* and purified by flash chromatography (silica gel, 0-30% ethyl acetate/hexane) to give 21 mg of the desired product as a colorless oil.

MS 843.5 $(M+H)^+$, 841.8 $(M-H)^-$; IR (film) 1785.1, 1733.1, 1681.7, 1640.9 $cm^{-1}$.

d)

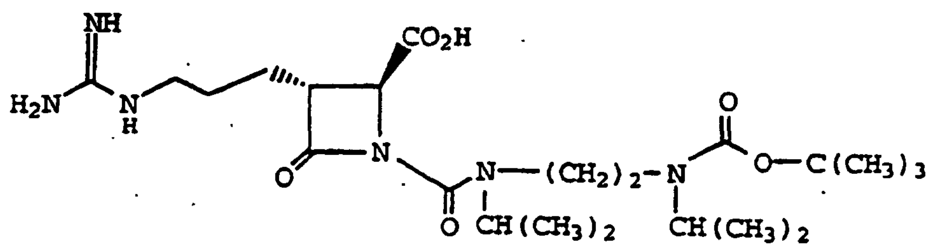

(homochiral)

10% Palladium on carbon catalyst (20 mg, wet type) was added to a solution of the product from part (c) (21 mg, 0.025 mmol) in 1,4-dioxane (5 ml) containing 1N HCl (25 µl, 0.025 mmol). Hydrogen gas was bubbled through the solution for 4 hours. The reaction mixture was filtered through a pad of Celite® which was then repeatedly washed with 1.4-dioxane (10 ml) and water (15 ml). The combined eluents were lyophilized. The white lyophilate was dissolved in water and passed through a plug of polyvinylpyrrolidone eluting with water. The eluents were lyophilized to give 12 mg of the desired product as a white lyophilate. MS 485.3 $(M+H)^+$, 483.5 $(M-H)^-$. IR (KBr) 1778.0, 1665.0 $cm^{-1}$.

**Example 56**

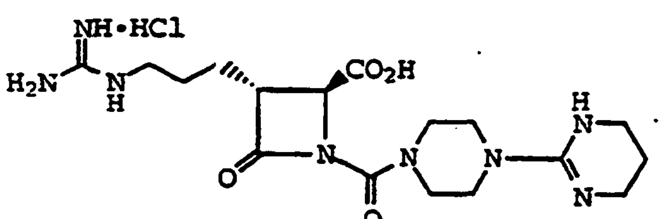

(homochiral)

a)

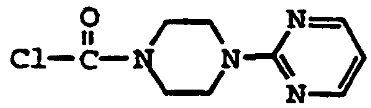

A solution of 1-(2-pyrimidyl)piperazine dihydrochloride (4.0 g, 16.9 mmol) in 1N sodium hydroxide saturated with sodium chloride (40 ml) was extracted with ethyl acetate (2 x 30 ml). The organic layer was dried over sodium sulfate, filtered and concentrated to give 2.6 g of 1-(2-pyrimidyl)piperazine.

A solution of 1-(2-pyrimidyl)piperazine (2.6 g) in methylene chloride (5 ml) was added dropwise over 3 minutes to a solution of phosgene (25 ml, 20% in toluene, 47.3 mmol) in methylene chloride (15 ml) over solid sodium bicarbonate (3g) under nitrogen at room temperature. The resulting solution was stirred vigorously for 10 minutes, filtered through a fritted funnel, and the remaining solids were washed with methylene chloride (2 x 5 ml). The combined eluent was concentrated under vacuum to give a white solid. The solid was then recrystallized from methylene chloride/hexane to give 3g of the desired product as a white solid. IR(film) 1735 $cm^{-1}$.

b)

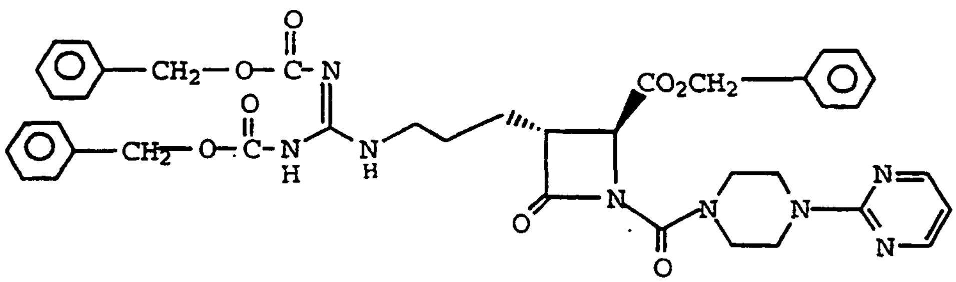

The acid chloride from part (a) (1.11 g, 4.8 mmol), triethylamine (700 µl, 5.0 mmol), dimethylaminopyridine (200 mg, 1.64 mmol) were added to a solution of the benzyl ester product from Example 1(c) (1.56 g, 3.23 mmol) in methylene chloride (15 ml) under nitrogen at room temperature. After stirring at room temperature for 7 hours, the reaction mixture was diluted with hexane (5 ml) and was then added to the top of a silica gel column (wetted with methylene chloride) for purification by flash chromatography (0 to 30% ethyl acetate/methylene chloride) to give 1.6 g of the derived product as a white foam. MS 763.2 $(M+H)^+$, 761.7 $(M - H)^-$; IR (KBr) 1788 $cm^{-1}$.

c)

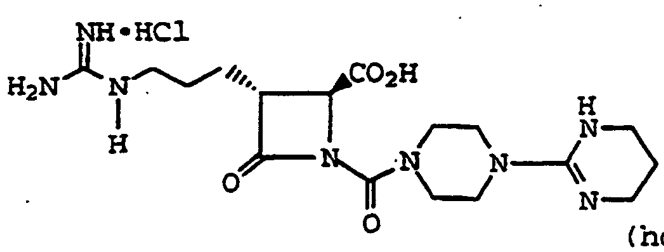

(homochiral)

The product from part (b) (1.6 g, 2.1 mmol) was deprotected and worked-up as described in Example 21(d) to give 854 mg of the desired product as white solid lyophilate. MS 409.2 $(M+H)^+$, 407.5 $(M-H)^-$; IR (KBr) 1777 $cm^{-1}$.

| Anal. calc'd for $C_{17}H_{28}N_8O_4 \cdot$ 1.0 HCl · 1.54 $H_2O$: | | | | | |
|---|---|---|---|---|---|
| | C. 43.20; | H, 6.84; | N. 23.71: | O, 18.75: | Cl, 7.50 |
| Found | C, 43.31; | H, 6.59; | N, 23.09: | O (not calculated) ; | Cl, 7.06. |

**Example 58**

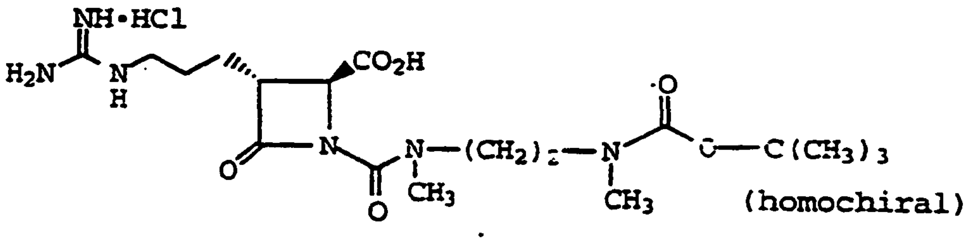

a)

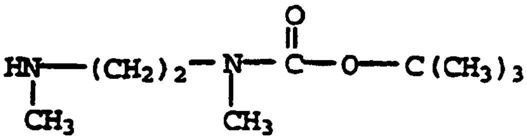

A solution of di-tert-butyl dicarbonate (1.24 g, 5.67 mmol) and triethylamine (790 µl, 5.67 mmol) in tetrahydrofuran (15 ml) was added dropwise to a solution of N,N'-dimethylethylene diamine (500 mg, 5.67 mmol) in tetrahydrofuran (35 ml). The reaction mixture was stirred at room temperature for 4 days. The mixture was then filtered and the filtrate was concentrated to give the crude product as a colorless oil. Purification by flash chromatography (10% 2N ammonia in methanol/methylene chloride) provided 362 mg of the desired product as a colorless oil. IR (film) 1694 $cm^{-1}$.

b)

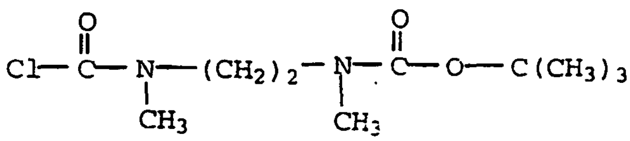

A mixture of the product from part (a) (250 mg) and triethylamine (278 µl) in methylene chloride (3 ml) was added to a solution of phosgene in toluene (1.4 ml, 20%) at 0°C. The resultant mixture was stirred at 0°C for 5 hours. Anhydrous ether (10 ml) was added and the solid was filtered off. The filtrate was evaporated to give the crude product as an orange oil which was purified by flash chromatography (20 - 30% ethyl acetate/hexane) to give 313 mg of the desired product as a colorless oil. IR (film) 1740 $cm^{-1}$, 1694 $cm^{-1}$.

c)

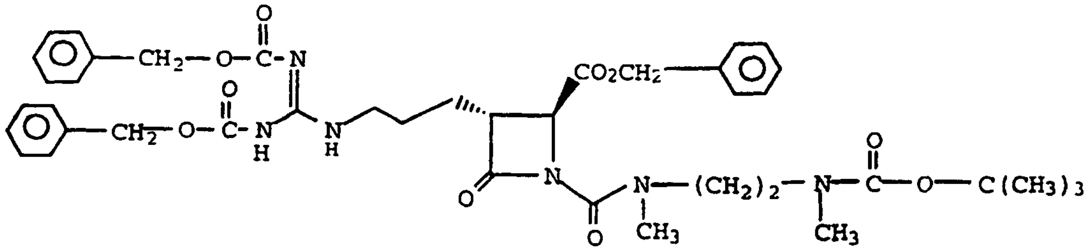

Triethylamine (25 µl), dimethylaminopyridine (15 mg) and a solution of the acid chloride product from part (b) (45 mg, 0.18 mmol) in methylene chloride (1 ml) were added to a solution of the benzyl ester product from Example

1(c) (70 mg) in methylene chloride (2 ml). The mixture was stirred overnight at room temperature. The reaction was quenched with the addition of 1N potassium bisulfate (15 ml). The mixture was extracted with ethyl acetate (2 x 30 ml). The organic layers were combined and washed with brine (10 ml), dried over magnesium sulfate and concentrated to give 101 mg of the crude product as a yellow oil. Purification using flash chromatography (30 - 50% ethyl acetate/hexane) gave 77 mg of the desired product as a colorless oil. IR (film) 1786 $cm^{-1}$, 1733 $cm^{-1}$, 1681 $cm^{-1}$, 1639 $cm^{-1}$.

d)

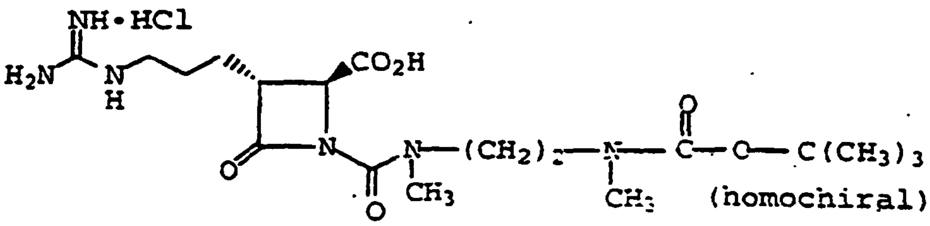

A mixture of the product from part (c) (74 mg. 0.094 mmol), 1N HCl (94 µl), and palladium on carbon catalyst (10%, 19 mg) in dioxane (2 ml) was stirred under hydrogen atmosphere (hydrogen balloon) at room temperature for 1 hour. The reaction mixture was filtered through a Celite® cake and lyophilized to give 44 mg of the desired product as a white solid. MS 429.2 $(M+H)^+$, 427.5 $(M-H)^-$; IR (KBr) 1784, 1663 $cm^{-1}$.

## Example 65

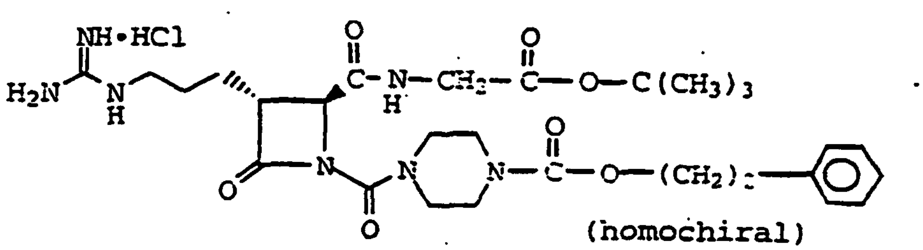

a)

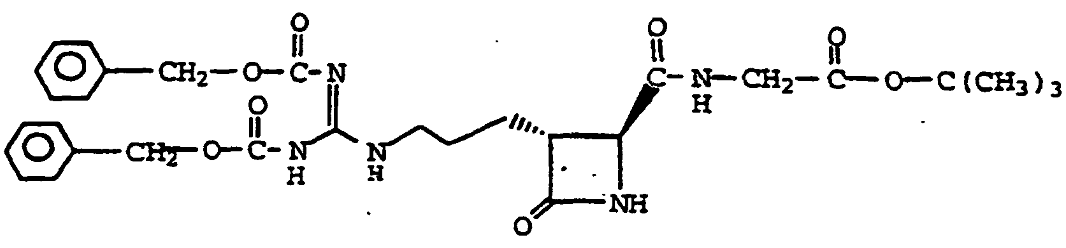

A solution of the carboxylic acid azetidinone product of Example 1(b) (482 mg, 1.0 mmol) in tetrahydrofuran (5 ml) was cooled to -20°C under an argon atmosphere and N-methylmorpholine (223 mg, 2.2 mmol) was added. 1.1 Equivalents of a 0.5 M solution of tert-butylglycine ester, hydrochloride (184 mg, 1.1 mmol) was added followed by the addition of benzotriazol-1-yloxytris(dimethylamino)phosphonium hexafluorophosphate (486 mg, 1.1 mmol). The reaction was stirred at -20°C for 24 hours, poured into 5% potassium bisulfate solution and extracted with ethyl acetate. The ethyl acetate extract was washed with water and brine, and dried over sodium sulfate. The solvents were evaporated and the crude residue was purified by silica gel chromotography eluting with ethyl acetate to give 396 mg of the desired product as a colorless solid. MS 596 $(M+H)^+$.

b)

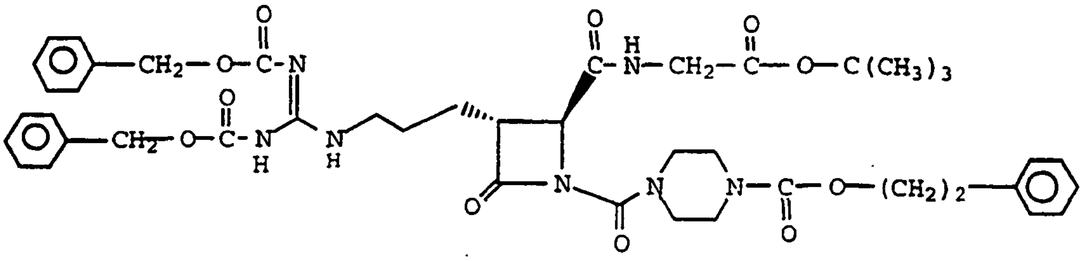

A solution of the product from part (a) (200 mg, 0.336 mmol) and triethylamine (38 mg, 0.37 mmol) in methylene chloride (4 ml) was stirred at room temperature and 1.1 equivalents of 1-phenethyloxypiperazine-4-carbonylchloride (110 mg, 0.37 mmol) was added. Dimethylaminopyridine (10 mg) was added and the reaction mixture was stirred for 30 hours. The reaction was diluted with methylene chloride, washed with brine, and dried over anhydrous sodium sulfate. The crude product was purified by flash chromatography on silica gel eluting with ethyl acetate yielding 210 mg of the desired product as a colorless glass-like residue. MS 856 $(M+H)^+$.

c)

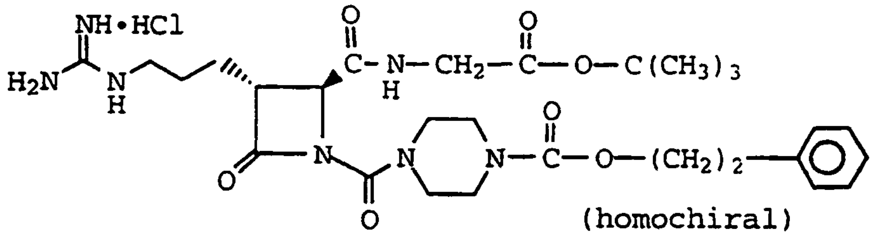

(homochiral)

A solution of the product from part (b) (200 mg, 0.234 mmol) in dioxane (5 ml) containing 1.1 equivalents of HCl was stirred under a hydrogen atmosphere with 10% palladium on carbon catalyst (75 mg) for 2 hours. The reaction was filtered and the solvents lyophilized to yield 122 mg of the desired product as a colorless solid. MS 588 $(M+H)^+$.

## Example 66

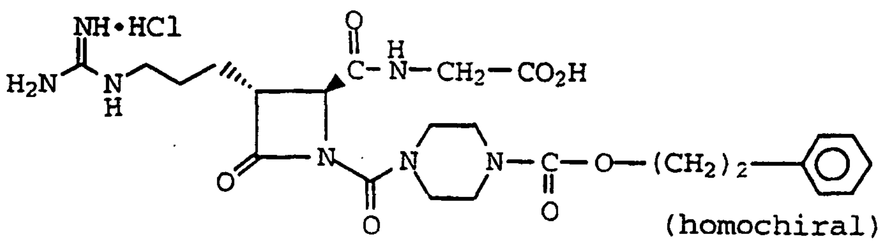

(homochiral)

The product of Example 65 (30 mg, 0.05 mmol) was added to trifluoroacetic acid (1 ml) at 0°C and the mixture was stirred for 30 minutes. The trifluoroacetic acid was evaporated and the residue was dissolved in water/dioxane (1:1) (1 ml) and lyophilized to give 22 mg of the desired product as a colorless solid. MS 532 $(M+H)^+$.

Example 67

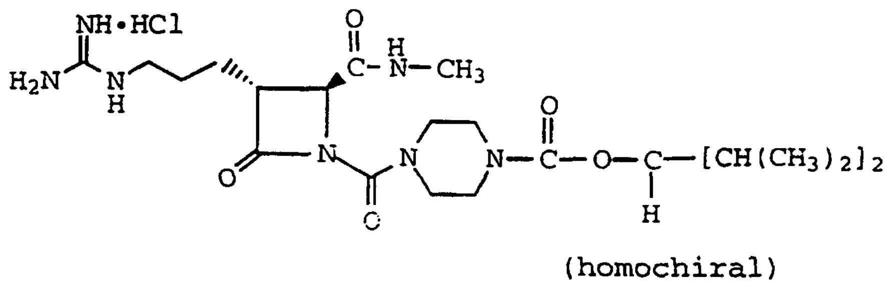

a)

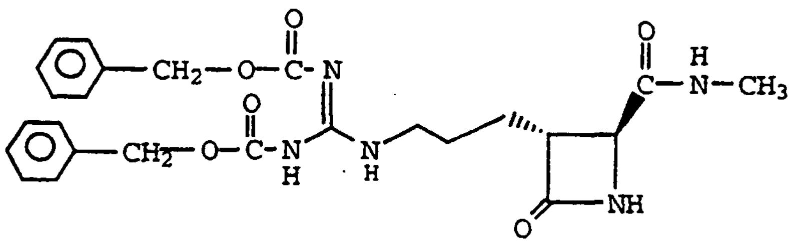

A solution of the carboxylic acid azetidinone product of Example 1(b) (150 mg, 0.311 mmol) in tetrahydrofuran (3 ml) was cooled to -20°C under an argon atmosphere and N-methylmorpholine (34.6 mg, 0.342 mmol) was added. 1.1 Equivalents of a 2 M solution on monomethylamine in tetrahydrofuran was added followed by the addition ofbenzotriazol-1-yloxytris(dimethylamino)phosphonium hexafluorophosphate (151 mg, 0.341 mmol). The reaction was stirred at -20°C for 48 hours, poured into 5% potassium bisulfate solution, and extracted with ethyl acetate. The ethyl acetate extract was washed with water and brine, and dried over sodium sulfate. The solvents were evaporated and the crude residue was purified by silica chromatography eluting with ethyl acetate to give 124 mg of the desired product as a colorless solid. MS 496 $(M+H)^+$.

b)

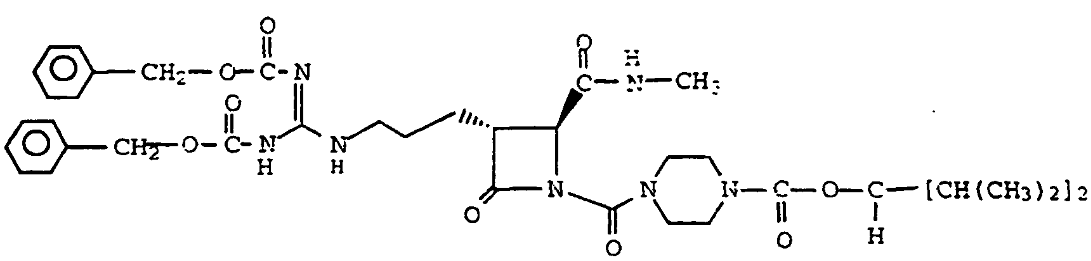

A solution of the product from part (a) (100 mg, 0.2 mmol) and triethylamine (23 mg, 0.225 mmol) in methylene chloride (2 ml) was stirred at room temperature and 1.1 equivalents of 1-diisopropylmethoxy-carbonylpiperazine-4-carbonylchloride (65 mg. 0.225 mmol) was added. Dimethylaminopyridine (8 mg) was added and the reaction mixture was stirred for 16 hours. The reaction was diluted with methylene chloride, washed with brine, and dried over anhydrous sodium sulfate. The crude product was purified by flash chromatography on silica eluting with ethyl acetate to give 58 mg of the desired product as a colorless glass-like residue. MS 750 $(M+H)^+$.

c)

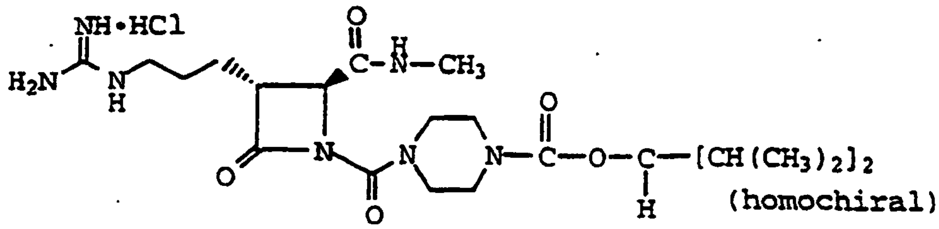

A solution of the product from part (b) (53 mg, 0.07 mmol) in dioxane (3 ml) containing 1.1 equivalents of 1N HCl was stirred under a hydrogen atmosphere with 10% palladium on carbon catalyst (20 mg) for 2 hours. The reaction was filtered and the solvents lyophilized to yield 32 mg of the desired product as a colorless solid. MS 482 $(M+H)^+$.

**Example 70**

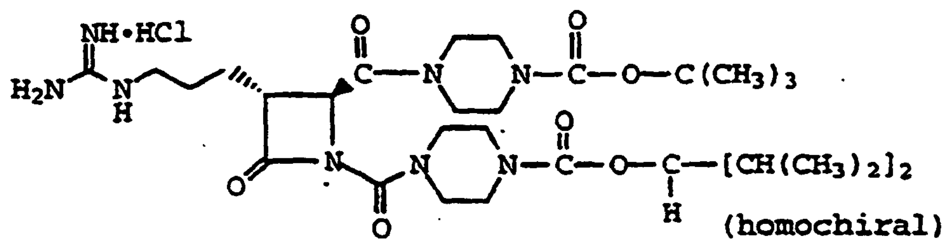

a)

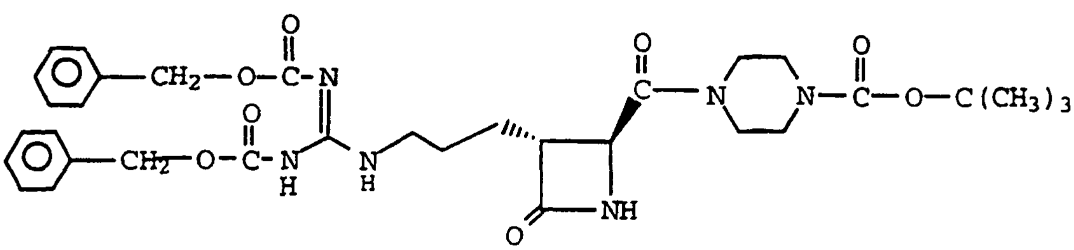

Following the procedure of Example 67(a) but substituting 1-*tert*-butoxycarbonylpiperazine for the monomethylamine, the desired product was obtained as a colorless solid. MS 651 $(M+H)^+$.

b)

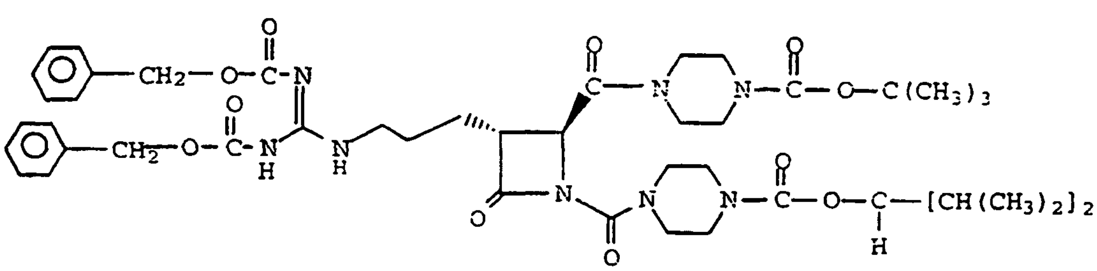

A solution of the product from part (a) (100 mg, 0.2 mmol) and triethylamine (23 mg, 0.225 mmol) in methylene chloride (2 ml) was stirred at room temperature and 1.1 equivalents of 1-diisopropyl-methyloxycarbonylpiperazine-4-carbonylchloride (65 mg, 0.225 mmol) was added. Dimethylaminopyridine (8 mg) was added and the reaction mixture was stirred for 48 hours. The reaction was diluted with methylene chloride, washed with brine, and dried over anhydrous sodium sulfate. The crude product was purified by flash chromatography on silica eluting with ethyl acetate to give 68 mg of the desired product as a colorless glass-like residue. MS 906 $(M+H)^+$.

c)

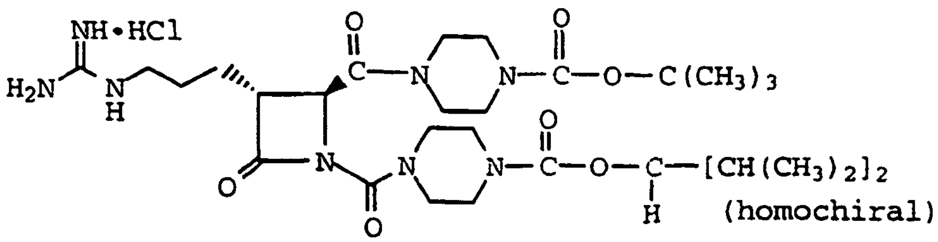

**Example 75**

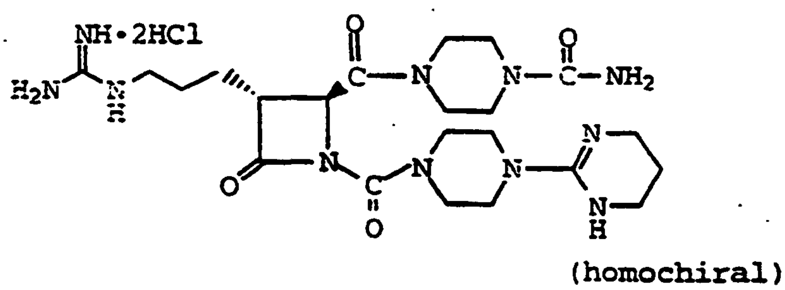

a)

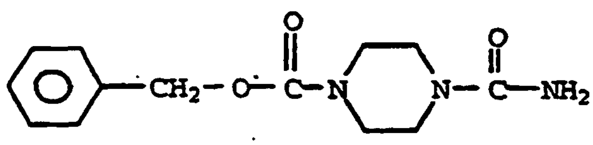

Trimethylsilyl isocyanate (3.8 ml, 3.22 g, 28 mmol) was added dropwise over 15 minutes to a solution of N-carbobenzyloxypiperazine (5.5 g, 25 mmol) and diisopropylethylamine (9.6 ml, 7.1 g, 55 mmol) in tetrahydrofuran (100 ml) at room temperature under an argon atomsphere. The reaction was stirred overnight at room temperature. The reaction was poured into water and extracted with ethyl acetate, washed with water and brine, and dried over sodium sulfate. The crude product was purified by column chromatography eluting with 20% ethyl acetate/hexane to give 5.1 g of the desired product. MS $(M+H)^+$ 264.

b)

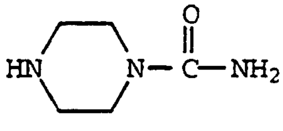

A mixture of the product from step (a) (3.96 g, 15 mmol) and palladium on carbon catalyst (10%, 2 g) in methanol (100 ml) was stirred under hydrogen atmosphere (hydrogen balloon) at room temperature for 1.25 hours. Filtration and concentration of the reaction gave 2.0 of the desired product.

c)

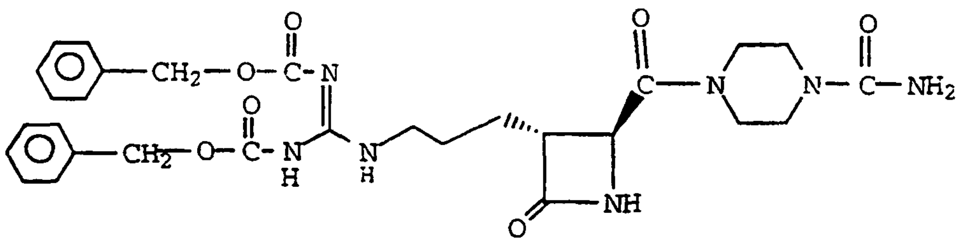

A mixture of the carboxylic acid azetidinone product of Example 1(b) (180 mg., 0.37 mmol), the product from step (b) (62 mg, 0.48 mmol), diisopropylethylamine (84 μl, 0.48 mmol), ethyl-3-(dimethylamino)propyl carbodiimide, hydrochloride salt (92 mg, 0.48 mmol) and 1-hydroxy-7-azabenzotriazole (65 mg, 0.48 mmol) in tetrahydrofuran (10 ml) was heated at 60°C overnight. The mixture was diluted with methylene chloride, washed with water, dried over magnesium sulfate, and concentrated to give the crude product. Purification of the crude product by flash column chromatography (silica, 5 - 10% methanol/methylene chloride) gave 128 mg of the desired product as a white solid. MS 594.3 $(M+H)^+$, 592.3 $(M-H)^-$.

d)

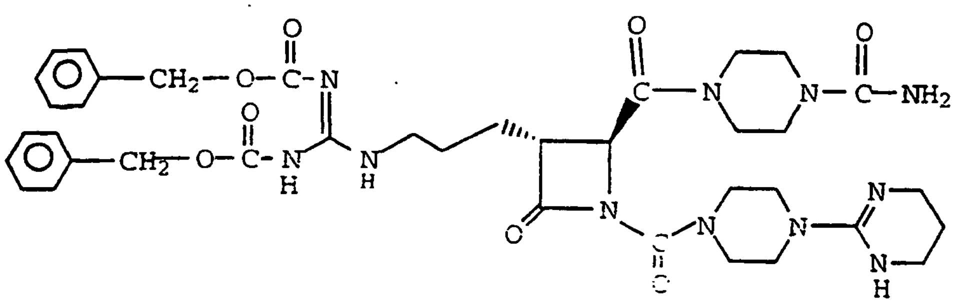

A mixture of the product from step (c) (110 mg. 0.185 mmol), the acid chloride from Example 56(a) (63 mg, 0.28 mmol), diisopropylethylamine (96 μl), and 4-dimethylaminopyridine (18 mg) in methylene chloride (4 ml) was stirred at room temperature for 18 hours. The reaction was quenched by the addition of saturated sodium chloride solution and extracted with ethyl acetate (3 x 50 ml). The extracts were combined, dried over magnesium sulfate, and concentrated. The crude product was purified by flash chromatography (0 - 15% methanol/methylene chloride) to give 114 mg of the desired product as a white solid. MS 784.5 $(M+H)^+$, 782.5 $(M-H)^-$ ; IR (KBr) 1782 $cm^{-1}$, 1732 $cm^{-1}$, 1643 $cm^{-1}$, 1586 $cm^{-1}$.

e)

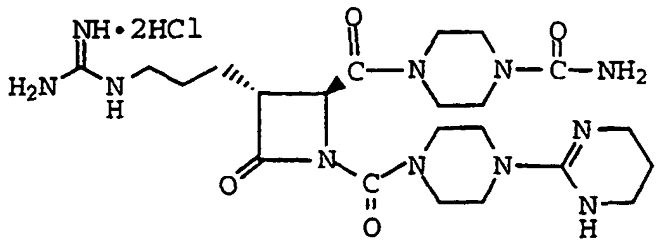

(homochiral)

A mixture of the product from step (d) (1.39 g, 1.77 mmol), 1N HCl (3.54 ml, 3.54 mmol) and palladium on carbon catalyst (10%, 750 mg) in dioxane (30 ml) was stirred under a hydrogen atomsphere (hydrogen balloon) at room temperature for 3.5 hours. Analytical HPLC indicated completion of the reaction. The reaction mixture was filtered through a , Celite® cake and lyophilized to give 1.01 g as a white solid. MS 260.6 $(M+2H)^{2+}$; 1R 1780 $cm^{-1}$, 1632 $cm^{-1}$.

The following additional compounds of formula IV were also prepared:

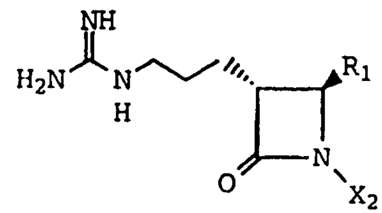

| Ex | $X_2$ | $R_1$ | salt | stereochemistry | $(M+H)^+$ |
|---|---|---|---|---|---|
| 76 | 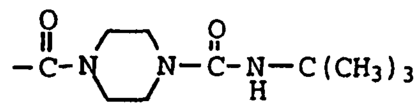 | 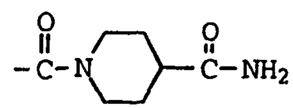 | 1.0 HCl | homochiral | 536 |
| 77 | 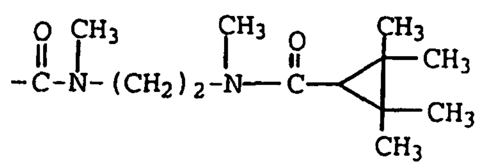 | $-CO_2H$ | ---- | homochiral | 453 |
| 78 | 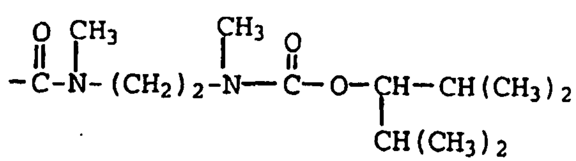 | $-CO_2H$ | ---- | homochiral | 471 |

| Ex | $X_2$ | $R_1$ | salt | stereochemistry | $(M+H)^+$ |
|---|---|---|---|---|---|
| 79 | 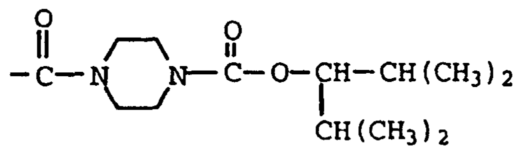 | 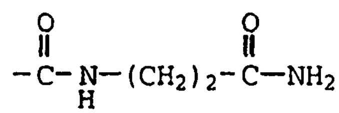 | 1.0 HCl | homochiral | 539 |
| 80 | 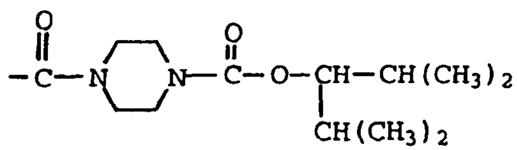 | 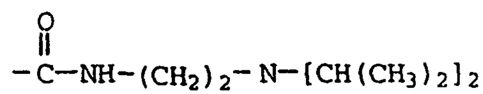 | 2.0 $CF_3CO_2H$ | homochiral | 595 |

| Ex | $X_2$ | $R_1$ | salt | stereochemistry | $(M+H)^+$ |
| --- | --- | --- | --- | --- | --- |
| 81 | 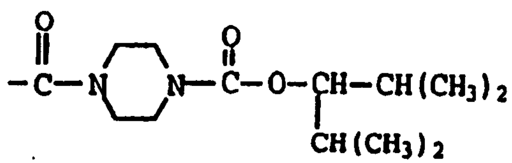 | 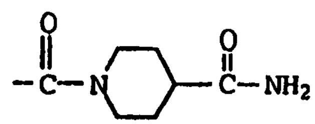 | 1.0 HCl | homochiral | 579 |

| Ex | $X_2$ | $R_1$ | salt | stereochemistry | $(M+H)^+$ |
|---|---|---|---|---|---|
| 84 | 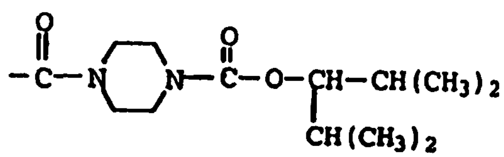 | 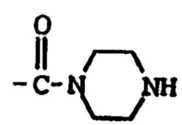 | 2.0 $CF_3CO_2H$ | homochiral | 537 |

| Ex | $X_2$ | $R_1$ | salt | stereochemistry | $(M+H)^+$ |
|---|---|---|---|---|---|
| 87 | 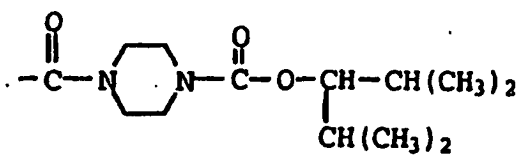 | 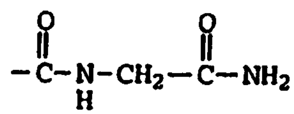 | 1.0 HCl | homochiral | 525 |

| Ex | $X_2$ | $R_1$ | salt | stereochemistry | $(M+H)^+$ |
|---|---|---|---|---|---|
| 89 | 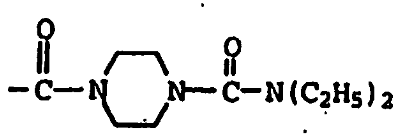 | $-CO_2H$ | ---- | homochiral | 426 |

| Ex | $X_2$ | $R_1$ | salt | stereochemistry | $(M+H)^+$ |
|---|---|---|---|---|---|
| 90 | 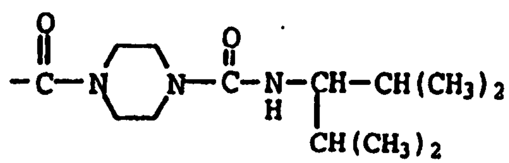 | $-CO_2H$ | ---- | homochiral | 468 |
| 91 | 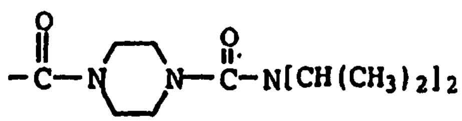 | $-CO_2H$ | ---- | homochiral | 454 |

| Ex | $X_2$ | $R_1$ | salt | stereochemistry | $(M+H)^+$ |
|---|---|---|---|---|---|
| 94 | 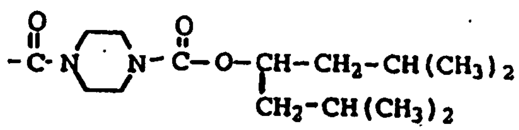 | $-CO_2H$ | 1.0 $CF_3CO_2H$ | homochiral | 497 |

| Ex | $X_2$ | $R_1$ | salt | stereochemistry | $(M+H)^+$ |
|---|---|---|---|---|---|
| 99 | 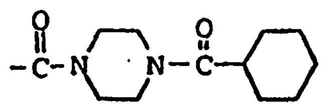 | $-CO_2H$ | 1.0 HCl | homochiral | 437 |
| 100 | 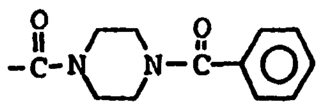 | $-CO_2H$ | 1.0 HCl | homochiral | 431 |

| Ex | X2 | R1 | salt | stereochemistry | (M+H)+ |
|---|---|---|---|---|---|
| 101 | 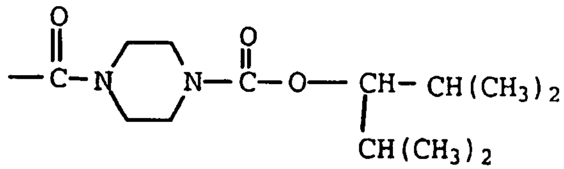 | $-CO_2H$ | 0.02 $CF_3CO_2H$ | homochiral | 469 |
| 102 | 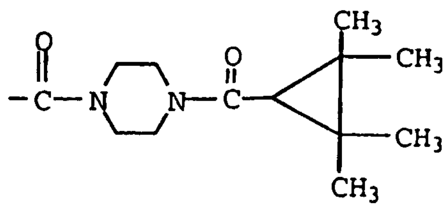 | $-CO_2H$ | 1.0 $CF_3CO_2H$ | homochiral | 451 |
| 103 | 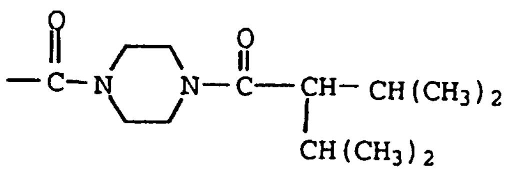 | $-CO_2H$ | ---- | homochiral | 453 |
| 104 | 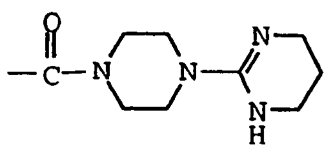 | 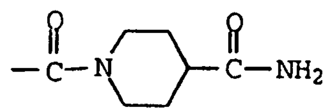 | 2.0 HCl | homochiral | 519 |

| Ex | $X_2$ | $R_1$ | salt | stereochemistry | $(M+H)^+$ |
|---|---|---|---|---|---|
| 108 | 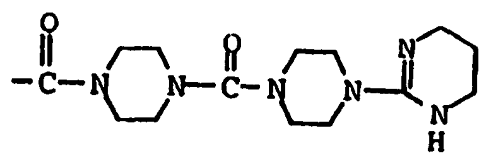 | $-CO_2H$ | 1.0 HCl | homochiral | 521 |
| 110 | 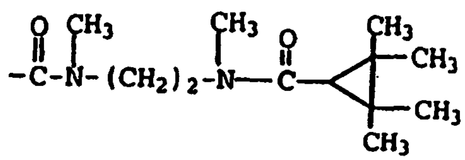 | 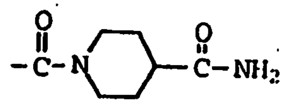 | 1.0 HCl | homochiral | 563 |

| Ex | $X_2$ | $R_1$ | salt | stereochemistry | $(M+H)^+$ |
|---|---|---|---|---|---|
| 115 | 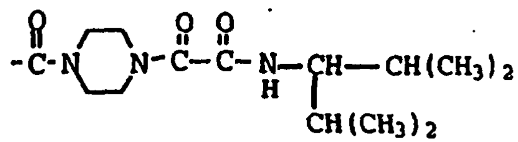 | $-CO_2H$ | --- | homochiral | 496 |
| 117 | 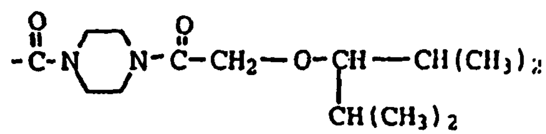 | $-CO_2H$ | --- | homochiral | 483 |
| 118 | 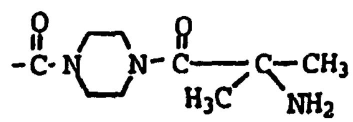 | $-CO_2H$ | 2.0 $CF_3CO_2H$ | homochiral | 434 |

| Ex | $X_2$ | $R_1$ | salt | stereochemistry | $(M+H)^+$ |
|---|---|---|---|---|---|
| 120 | 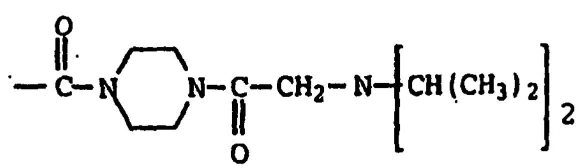 | 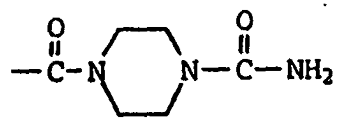 | 2.0 $CF_3CO_2H$ | homochiral | 579 |

| Ex | $X_2$ | $R_1$ | salt | stereochemistry | $(M+H)^+$ |
|---|---|---|---|---|---|
| 128 | 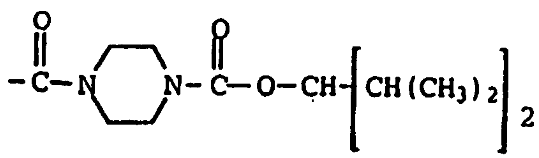 | 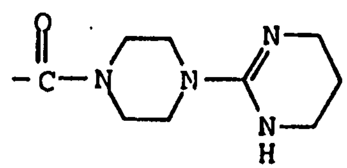 | $2.0\ CF_3COOH$ | homochiral | 619 |

| Ex | $X_2$ | $R_1$ | salt | stereochemistry | $(M+H)^+$ |
|---|---|---|---|---|---|
| 130 | 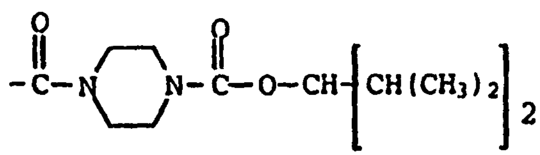 | 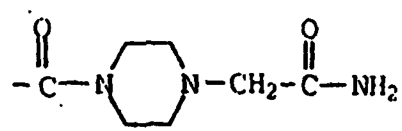 | 2.0 $CF_3COOH$ | homochiral | 594 |

The following additional compounds of formula IV were also prepared

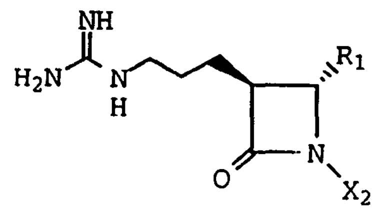

| Ex | $X_2$ | $R_1$ | salt | stereochemistry | $(M+H)^+$ |
| --- | --- | --- | --- | --- | --- |
| 131 | 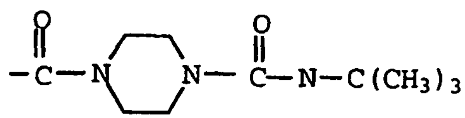 | $-CO_2H$ | | homochiral | 426 |
| 132 | 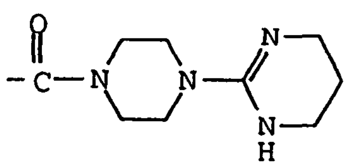 | 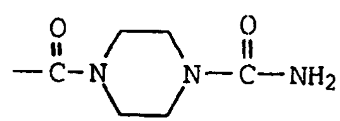 | 2.0 HCl | homochiral | 520 |

| Ex | $X_2$ | $R_1$ | salt | stereochemistry | $(M+H)^+$ |
|---|---|---|---|---|---|
| 133 | 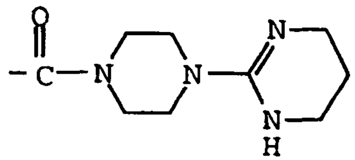 | $-CO_2H$ | 1.0 HCl | homochiral | 409 |
| 134 | 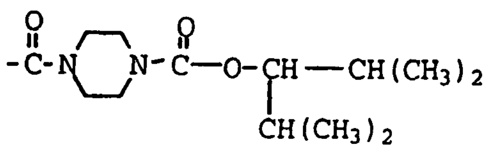 | 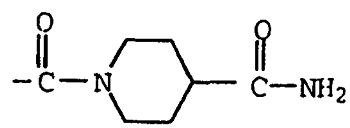 | 1.0 HCl | homochiral | 579 |

**Example 171** (not encompassed in the scope of the present invention

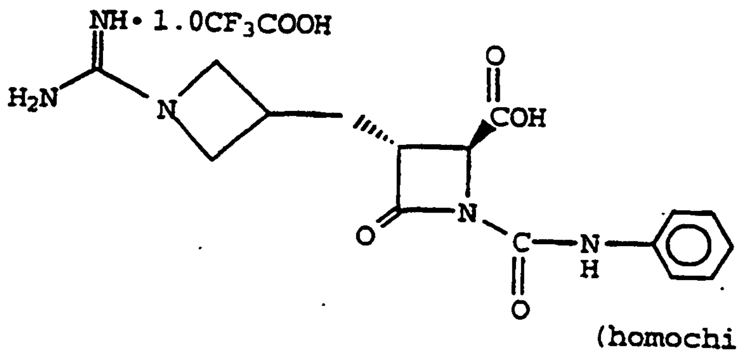

(homochiral)

a)

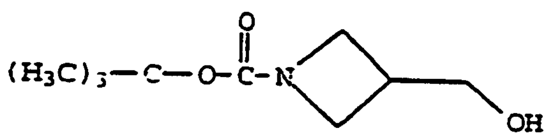

The azetidine carboxylic acid

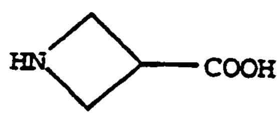

was prepared from epichlorohydrin according to the procedure of A.G. Anderson Jr. and R. Lok, J. Org. Chem., VoL 37, p. 3953, (1972). This azetidine carboxylic acid was then converted to the desired product according to the procedure of T.L. Hansen et al., WO97/23508.

b)

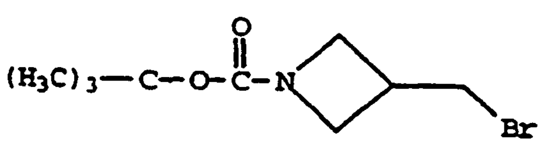

A solution of triphenylphosphine (1.57 g, 6 mmol) in methylene chloride (6 ml) was added dropwise to a stirred solution of the product from part (a) (936 mg, 5 mmol) and carbon tetrabromide (2.32 g, 7 mmol) in methylene chloride 910 ml) at 0°C under argon. The reaction was then stirred at room temperature for 16 hours. The reaction was concentrated in *vacuo* and the residue was triturated with ethyl ether. Filtration and evaporation of the filtrate gave 3.34 g of an oily residue which was chromatographed over silica gel by eluting with methylene chloride and then methylene chloride:ethyl acetate (19:1) to give 1.02 g of the desired product as an oily residue.

c)

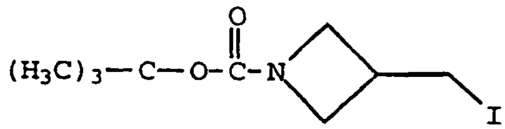

A mixture of the product from part (b) (1.00 g, 4 mmol) and sodium iodide (1.80 g, 12 mmol) in dry acetonitrile (10 ml) under argon was stirred at 65°C for 2.5 hours, cooled to room temperature and concentrated *in vacuo*. The residue was taken up in ethyl acetate and water and the ethyl acetate layer was washed with water (twice), dilute sodium thiosulfate, and water (twice), dried over sodium sulfate, and concentrated to give 1.18 g of the desired product as an oily residue.

d)

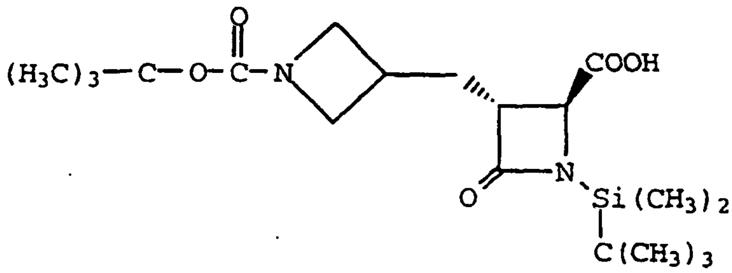

To diisopropylamine (0.63 ml, 4.5 mmol) in tetrahydrofuran (3.5 ml) at -20°C under argon was added 1.6 ml of 2.5 M n-butyl lithium in hexane (4 mmol). The mixture was stirred for 15 minutes and cooled to -70°C. A solution of (4S)-N-(t-butyldimethylsilyl) azetidine-2-one-4-carboxylic acid (459 mg, 2.0 mmol) [Baldwin et al, Tetrahedron, Vol. 46, p. 4733 - 4748, 1990] in tetrahydrofuran (2.5 ml) was added over 3 minutes and the reaction was warmed to -20°C over 15 minutes. A solution of the product from part (c) (1.19 g, 4 mmol) in tetrahydrofuran (3 ml) was added and the reaction was stirred between -20°C and -30°C for 1.5 hours and then at -20°C for 16 hours. The reaction was warmed to 0°C and quenched by the addition of 5% potassium bisulfate and then ethyl acetate. After extraction with ethyl acetate (three times), the ethyl acetate extracts were combined, washed with brine, dried over sodium sulfate, and concentrated to an oily residue. The residue was dissolved in ethyl ether and washed with saturated sodium bicarbonate (twice). The combined sodium bicarbonate extract was washed with ethyl ether and then layered with ethyl acetate. The pH was adjusted to 2.2 (10% potassium bisulfate) and after extraction with ethyl acetate (three times), the acidic ethyl acetate extract was washed with brine. dried over sodium sulfate, and concentrated to give 624 mg of the desired product as a crude oil.

e)

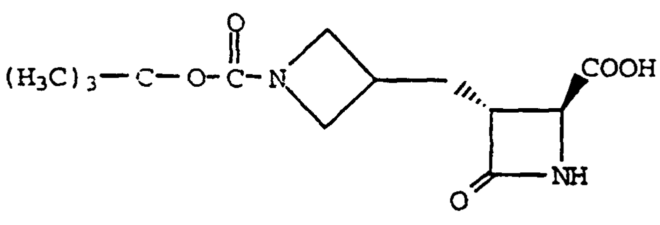

To the crude product from part (d) in tetrahydrofuran (3 ml) at 0 - 5°C under nitrogen was added 2.7 ml of 1M tetrabutylammonium fluoride in tetrahydrofuran. The reaction was stirred for 1.5 hours at room temperature and then the solvent was removed *in vacuo* and the residue was taken up in ethyl acetate, water and 10% potassium bisulfate (11 ml). After extraction with ethyl acetate (three times), the extracts were combined, washed with small amounts of water (twice), and brine, dried over sodium sulfate, and concentrated to give 470 mg of crude desired product as an amorphous residue.

f)

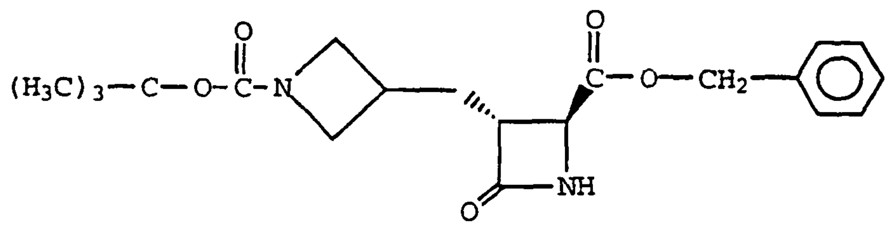

A solution of the product from part (e) (466 mg), benzyl bromide (0.84 ml, 7.1 mmol) and sodium bicarbonate (239 mg, 2.84 mmol) in dry dimethylformamide (4 ml) was stirred at room temperature under nitrogen for 16 hours. The reaction was diluted with ethyl acetate and water, and the aqueous layer was extracted with ethyl acetate (twice). The ethyl acetate extracts were combined, and washed with dilute potassium bisulfate, water (twice) and brine, dried over sodium sulfate, and concentrated to give 563 mg of an oil. This oil was chromatographed over silica gel by eluting with methylene chloride and then methylene chloride:ethyl acetate (1:1) to give 407 mg of the desired product as an amorphous residue.

g)

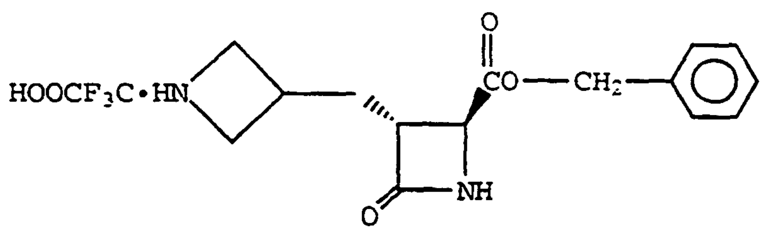

Trifluroacetic acid (1 ml) was added to a stirred solution of the product from step (f) (300 mg, 0.80 mmol) in methylene chloride (3 ml) at 0 - 5°C. After 5 minutes, the reaction was stirred at room temperature for 1 hour and then concentrated *in vacuo* to give the desired product as a residue.

h)

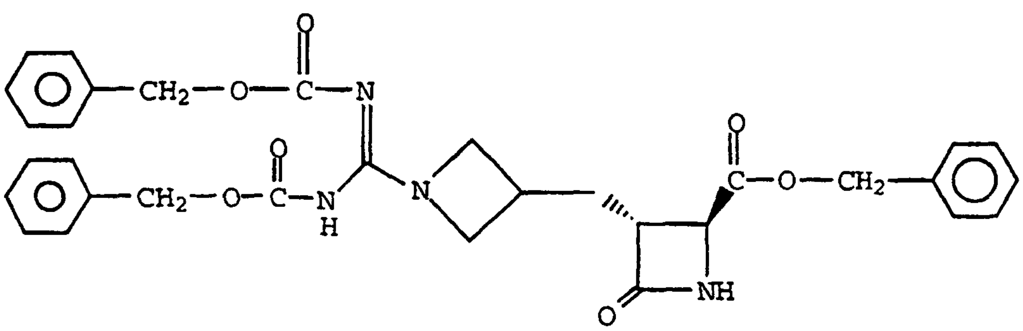

To a solution of the product from part (g) (0.80 mmol) in dry dimethylformamide (3 ml) under argon was added sequentially N,N'-dicarbobenzyloxy-S-methylisothiourea (430 mg, 1.20 mmol), triethylamine (0.45 ml, 3.23 mmol), and mercuric chloride (326 mg, 1.20 mmol). The reaction was stirred at room temperature for 2.5 hours and then filtered through Celite® using ethyl acetate. The filtrate was washed with dilute aqueous potassium bisulfate (twice) and brine, dried over sodium sulfate, and concentrated to an oily residue (767 mg). Purification by chromatography over silica gel eluting with methylene chloride:ethyl acetate (6:4) and methylene chloride: ethyl acetate (3:7) gave 342 mg of the desired product as an oily residue.

i)

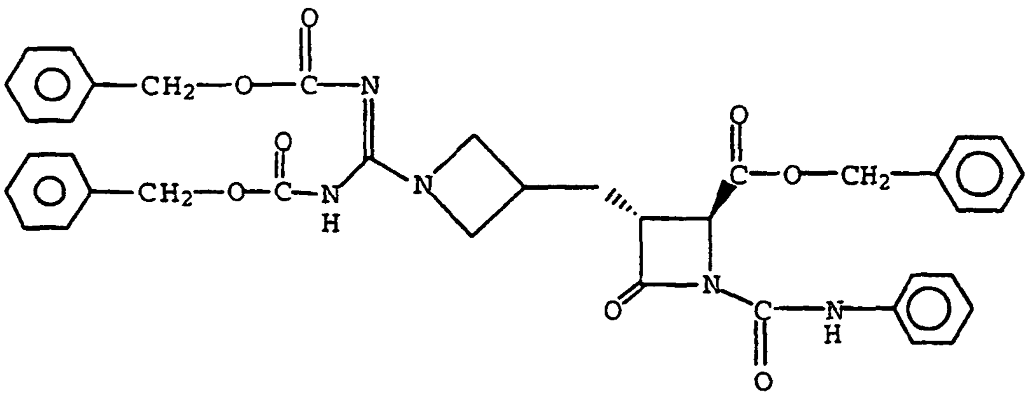

To the product from part (h) (136 mg, 0.23 mmol), previously dried by azetroping from tetrahydrofuran and toluene, in tetrahydrofuran (2 ml) at -78°C under argon was added 0.30 ml of 1.0M sodium bis(trimethylsilyl)amide (0.30 mmol). The mixture was stirred for 10 minutes and then phenylisocyanate (31 µl,0.28 mmol) was added. The reaction was warmed to 0°C over 40 minutes and poured into 2 ml of 10% potassium bisulfate and water. After extraction with ethyl acetate (three times), the ethyl acetate extracts were combined, washed with water (three times), dried over sodium sulfate, and concentrated to give 193 mg of an oil. Purification by chromatography over silica gel eluting with methylene chloride: ethyl acetate (98:2) and then methylene chloride:ethyl acetate (95: 5) gave 121 mg of the desired product as an oily residue.

j)

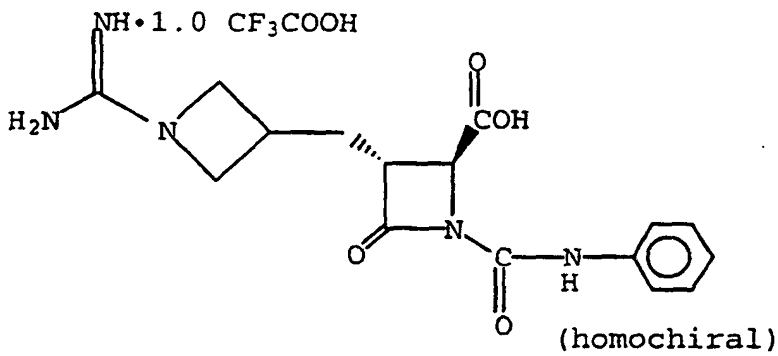

(homochiral)

The product from part (i) (115 mg, 0.163 mmol) was hydrogenated in dioxane (4 ml) and 1.0N HCl (163 µl, 0.163 mmol) in the presence of 10% palladium on carbon catalyst (35 mg) at 1 atmosphere for 1 hour. After filtration using aqueous dioxane, the filtrate was concentrated to a residue. This residue was lyophilized from aqueous dioxane to give 89 mg of crude product. Purification by preparative HPLC [YMC S5 ODS 30 x 250 mm, 25 ml/min, using a gradient (10 - 40%) of solvent A (10% methanol + 90% water + 0.1% trifluoroacetic acid) and solvent B (90% methanol + 10% water + 0.1% trifluoroacetic acid)] gave after lyophilization 31 mg of desired product as a white hydroscopic solid, IR (KBr) 1780 $cm^{-1}$; $(M+H)^+$ = 346.

**Example 172**

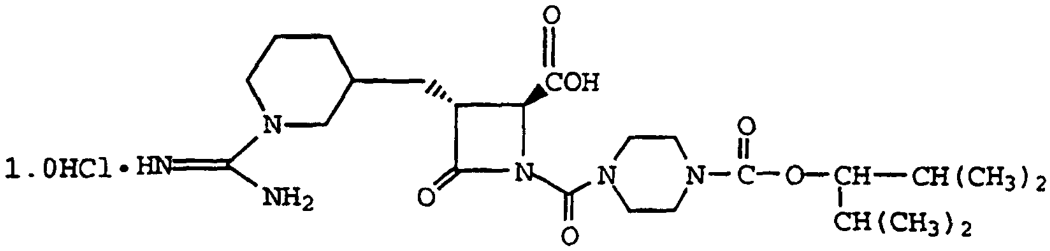

(diastereomer mixture)

a)

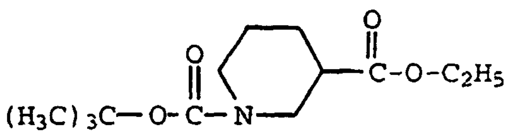

D-*tert*-butyl dicarbonate (10.9 g, 50 mmol) was slowly added to a stirred solution of ethyl nipecotate (6.2 ml, 40 mmol) and N,N-diisopropylethylamine (7.0 ml, 40 mmol) in methylene chloride (80 ml) at 0°C under argon. The cooling bath was removed, dimethylaminopyridine (0.49 g, 4 mmol) was added, and the reaction was stirred overnight at room temperature. The reaction was concentrated *in vacuo* and the residue was taken up in ethyl acetate. The ethyl acetate was washed with dilute HCl(2 x) and brine (2 x), dried over magnesium sulfate and concentrated to an oil, which was pased through a column of silica gel using hexanes-ethyl acetate (8:2) to provide 10.2 g of the desired product as a colorless oil.

b)

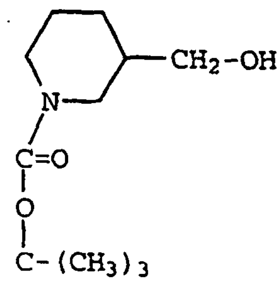

A solution of 1M lithium aluminum hydride in tetrahydrofuran (40.4 ml, 40.4 mmol) was added over 10 minutes to a stirred solution of the product from part (a) (9.92 g, 38.5 mmol) in tetrahydrofuran (120 ml) at 0°C under argon. After 45 minutes, the ice water cooled reaction was decomposed by the cautious dropwise addition of 5N sodium hydroxide (10 ml). The mixture was stirred for 10 minutes and the semigranular mixture was filtered. The filtrate was concentrated to an oil, which was taken up in ether. The ether was washed with brine, dried over sodium sulfate and concentrated to an oil, which solidified *in vacuo* to give 7.73 g of the desired product.

c)

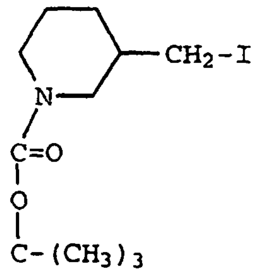

A solution of the product from part (b) (4.1 g, 20 mmol) in 30 ml of methylene chloride was added over 10 minutes to a stirred solution of triphenylphosphine (7.34 g, 28 mmol), imidazole (1.91 g, 28 mmol) and iodine (7.1 g, 28 mmol) in 70 ml of methylene chloride at 0°C under argon. The cooling bath was removed and the reaction was stirred at room at room temperature for 1 hour and filtered. Concentration of the filtrate gave an oil which was stirred with ethyl acetate for 15 minutes. After filtration, the filtrate was washed with 5% sodium thiosulfate (3x) and then brine, dried over magnesium sulfate, and concentrated to give 11.4 g of crude product, which was chromatographed over silica gel using methylene chloride to afford 6.1 g of the desired product as a colorless oil.

d)

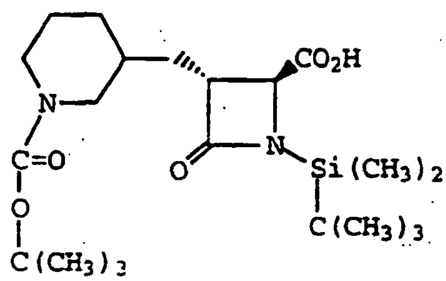

Reaction of (4S)-N-(t-butyldimethylsilyl)azetidine-2-one-4-carboxylic acid (1.15 g, 5 mmol) and the product from part (c) (3.25 g, 10 mmol) according to the procedure of Example 171 step (d) gave 1.98 g of the crude desired product as a foam.

e)

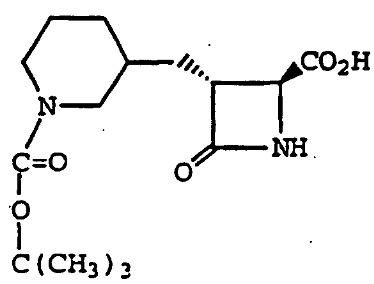

Treatment of the product from part (d) (1.98 g) with tetrabutylammonium fluoride in tetrahydrofuran according to the procedure of Example 171 step (e) gave 1.64 of the crude desired product as an oil.

f)

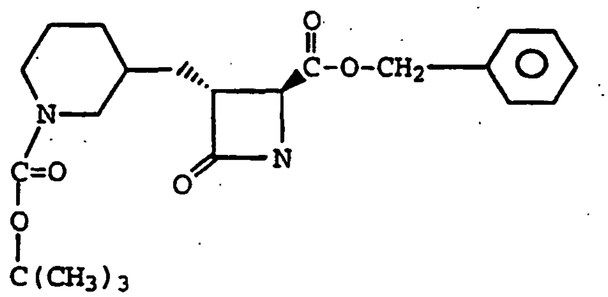

Treatment of the product from part (e) (1.64 g) with benzyl bromide according to the procedure of Example 171 step (f) gave 1.23 g of the desired product as an oil after silica gel chromatography by eluting with methylene chloride and then methylene chloride/ethyl acetate (6:4).

g)

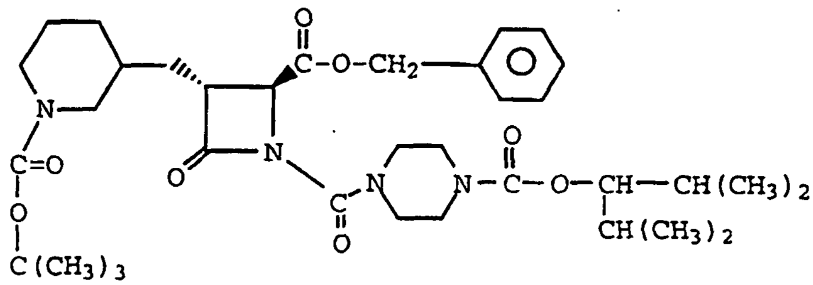

A solution of the product from part (f) (402 mg, 1 mmol), triethylamine (0.28 ml, 2.0 mmol), 1-diisopropylmethoxycarbonyl-piperazine-4-carbonylchloride (436 mg, 1.50 mmol) and dimethylaminopyridine (31 mg, 0.25 mmol) in methylene chloride (4.5 ml) was stirred at room temperature under argon for 8 hours and stored at 0°C overnight. The reaction was concentrated *in vacuo* and the residue was taken up in ethyl acetate, 10% potassium bisulfate (4 ml) and water. The ethyl acetate layer was washed again with dilute potassium bisulfate, water (2 x), and brine, dried over sodium sulfate and concentrated to a viscous oil (779 mg). Chromatography of the oil over silica gel using 10% and then 20% ethyl acetate in methylene chloride provided 618 mg of the desired product as an oil.

h)

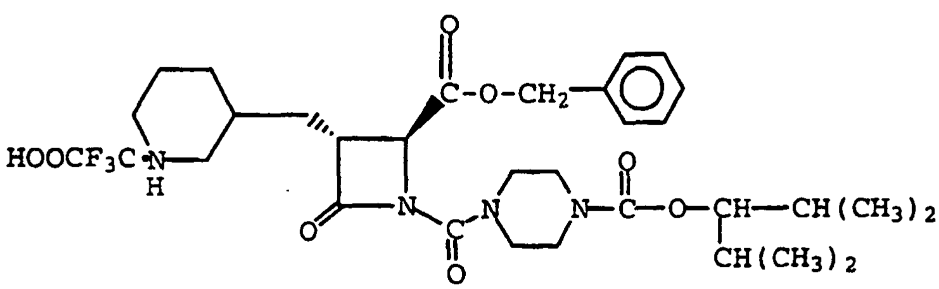

Trifluoroacetic acid (0.75 ml) was added to a stirred solution of the product from part (g) (436 mg, 0.66 mmol) in methylene chloride (3 ml) at 0 - 5°C. After 5 minutes, the reaction was stirred at room temperature for 2 hours and concentrated *in vacuo* to a residue, which was further concentrated from methylene chloride (5 x) and then chloroform (2 x) to give 648 mg of compound of the desired product as an oil.

i)

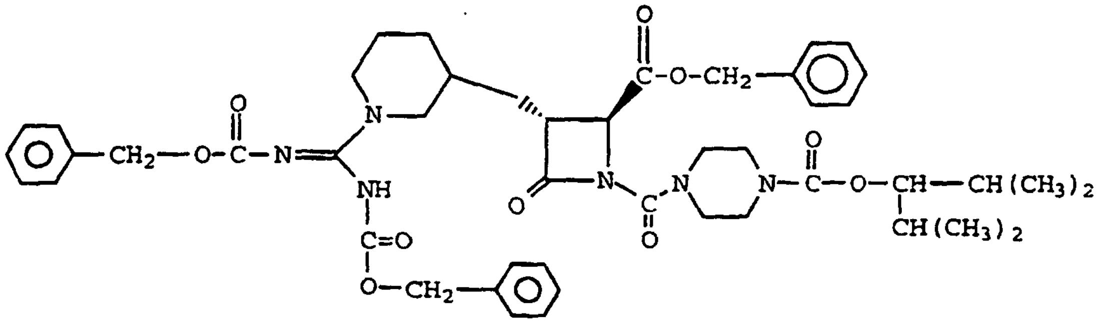

To a solution of the product from part (h) (0.25 ml) in dimethylformamide (1.5 ml) under argon were added sequentially N,N'-dicarbobenzyloxy-S-methylisothiourea (136 mg, 0.38 mmol), triethylamine (0.21 ml, 1.5 mmol) and mercuric chloride (103 mg, 0.38 mmol). The reaction was stirred at room temperature for 3 hours, diluted with ethyl acetate and filtered through Celite. The filtrate was washed with dilute aqueous potassium bisulfate (2 x) and brine (2x), dried over sodium sulfate and concentrated to an oil (280 mg), which was purified by chromatography over silica gel by eluting with 15% and then 20% ethyl acetate in methylene chloride to give 146 mg of the desired product as an oily residue.

i)

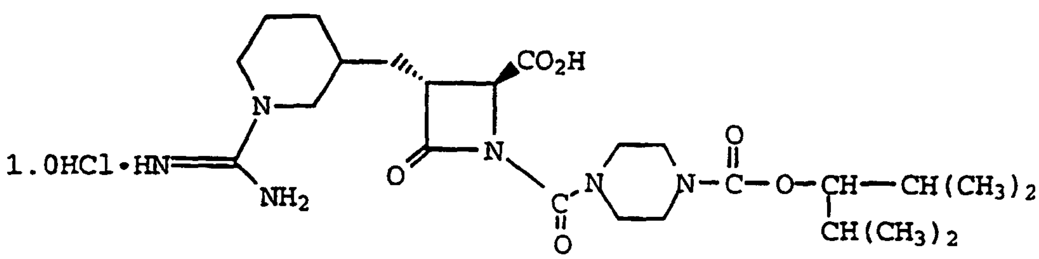

(diastereomer mixture)

The product from part (i) (141 mg, 0.163 mmol) was hydrogenated in dioxane (5 ml) and 1.0 N HCl (0.163 mmol) in the presence of 10% palladium on carbon catalyst (42 mg) at 1 atmosphere for 1 hour. After filtration using aqueous dioxane, the filtrate was concentrated to remove dioxane, filtered, and lyophilized to give 47 mg of the desired product as a white solid; IR(KBr) 1787 $cm^{-1}$, consisting of a mixture (52:48) of diastereomers as determined by HPLC.

The following compounds of formula I' were also prepared

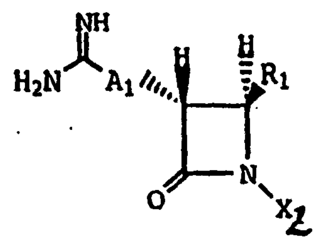

| Ex | $-A_1-$ | $X_2$ | $R_1$ | salt | stereochemistry | $(M+H)^+$ |
|---|---|---|---|---|---|---|

| | | | | | | |
|---|---|---|---|---|---|---|
| 177 | 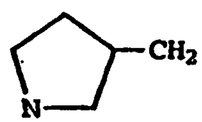 | 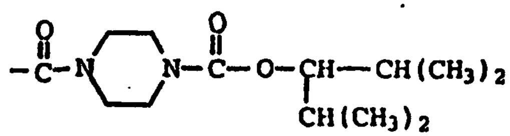 | $\cdot CO_2H$ | 1.0 HCl | diastereomer mixture | 495 |

**Example 192**

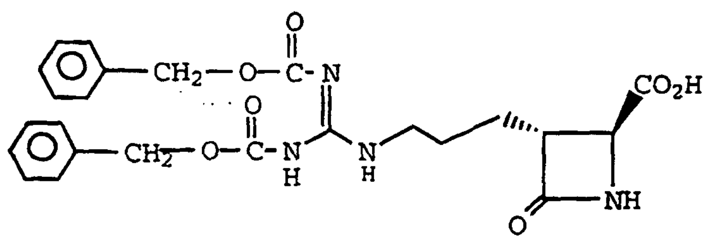

The intermediate of Example 1(b) was also prepared as follows:

a)

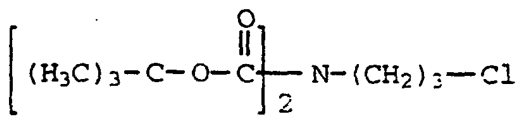

Potassium *t*-amylate (25% wt in toluene, 164.0 g, 322.6 mmol) was added to a solution of di-*t*-butyl iminodicarboxylate (70.1 g, 322.6 mmol) in dimethylformamide (500 ml) over a 15 minute period under nitrogen. The resulting white creamy solution was stirred at 0°C for 40 minutes. 1-Chloro-3-iodopropane (60.0 g, 31. ml, 293.3 mmol) was added and the mixture was stirred at 0° for 3 hours. Hexane (500 ml) and water (300 ml) were added to the mixture. The aqueous layer was separated and extracted with hexane (300 ml). The combined hexane extracts were washed with 1N sodium hydroxide (3 x 300 ml), saturated sodium hydrogen phosphate (300 ml), half-saturated brine (300 ml), and brine (500 ml) and dried over sodium sulfate. Removal of the sodium sulfate by filtration followed by concentration gave 84.2 g of the desired product as a light yellow oil which was dried under vacuum overnight.

b)

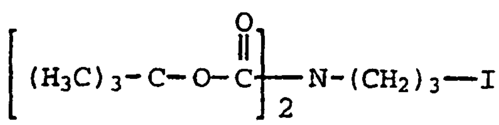

The product from part (a) (55.0 g, 187.2 mmol) was dissolved in acetone (550 ml). Sodium iodide (84.2 g, 561.6 mmol) and sodium bicarbonate (7.9 g, 93.6 mmol) were added. The mixture was stirred at 58°C (oil bath) under nitrogen for 6 hours and additional sodium iodide (14 g, 93.4 mmol) was added). The reaction mixture was stirred for 12 hours and the acetone was evaporated. Hexane (400 ml) and water (300 ml) were added to the resulting solid. The hexane layer was separated, washed with 5% sodium thiosulfate (300 ml), half -saturated brine (300 ml) and brine (300 ml), dried over sodium sulfate, and filtered through 20 g of a silica gel pad. The silica gel pad was washed with hexane (400 ml). Concentration of the filtrate gave 68.2 g of the desired product as a light yellow oil which was dried under vacuum overnight.

c)

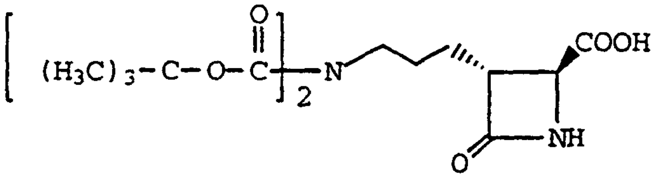

*n*-Butyl lithium (2.5 M in hexane, 46 ml, 115.0 mmol) was added to a solution of diisopropylamine (11.7 g, 16.2 ml, 115.3 mmol) in tetrahydrofuran (150 ml) at 0°C under nitrogen. The resulting solution was stirred at 0°C for 30 minutes then cooled to -30°C. A solution of (4S)-N-(*t*-butyldimethylsilyl)-azetidine-2-one-4-carboxylic acid (12.0 g, 52.4 mmol) [Baldwin et al, Tetrahedron, Vol. 46, p. 4733 - 4748, 1990] in tetrahydrofuran (60 ml) was added and the mixture was stirred at -20°C for 30 minutes. A solution of the iodo product from part (b) (24.0 g, 62.3 mmol) in tetrahydrofuran (30 ml) was added dropwise over a 20 minute period and the resulting mixture was stirred at -20°C for 2 hours. The reaction mixture was allowed to warm to 0°C and water (300 ml) was added. The mixture, adjusted to pH 12.5 with 10% sodium bisulfate, was stirred at 0°C for 30 minutes and then washed with hexane (2 x 100 ml). The aqueous layer was cooled in an ice-bath and acidified to pH 3.0 by the dropwise addition of 6N HCl. This solution was saturated with sodium chloride and extracted with ethyl acetate (3 x 150 ml). The combined ethyl acetate extracts were washed with brine (200 ml), dried over sodium sulfate, filtered and concentrated to give a yellow oil. This oil was dissolved in acetonitrile (20 ml) and evaporation of the acetonitrile gave 16.4 g of the desired product as a yellow foam.

d)

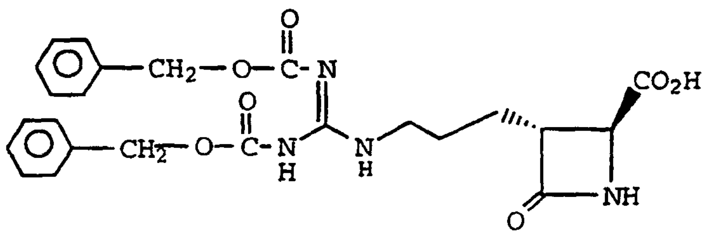

The product from part (c) (4.25 g, 11.4 mmol) was added to a 1:2 mixture of trifluroacetic acid/methylene chloride (42 ml). The resulting mixture was stirred at room temperature for 30 minutes under a nitrogen atmosphere. Toluene was added (80 ml) and the mixture was concentrated to a small volume (approximately 15 ml). Additional toluene (80 ml) was added and the mixture was concentrated to dryness to afford a yellow oil.

A mixture of triethylamine (3.97 ml, 28.5 mmol) and methanol (42 ml) was added into the above oil at 0°C. Additional triethylamine (0.85 ml, 5.7 mmol) and N,N'-bis(benzyloxycarbonyl)-1-guanylpyrazole (4.31 g, 11.4 mmol) [Wu et al, Synthetic Communications, 23(21), p. 3055-3060 (1993)] were added. The mixture was stirred at room temperature for 11 hours and then concentrated *in vacuo* at 25°C to afford a yellow oil.

Ethyl acetate (30 ml) and water (10 ml) were added to this oil followed by acidification to pH 3.2 at 0°C by the addition of 2M potassium bisulfate which was saturated with sodium chloride. The acidic mixture was poured into a separatory funnel. The layers were separated, and the aqueous layer was washed with ethyl acetate (2 x 25 ml) while ensuring that the pH of the aqueous solution was in the range of 2.9 to 3.2. The combined ethyl acetate solutions were washed with saturated sodium chloride solution (25 ml)and the product was extracted with saturated sodium bicarbonate (3 x 25 ml). The combined sodium bicarbonate solutions were washed with ethyl acetate (2 x 25 ml), acidified to pH 3.2 with concentrated HCl at 0°C, treated with saturated sodium chloride (solid), and finally extracted with ethyl acetate (3 x 25 ml). The combined ethyl acetate solution was dried over anhydrous magnesium sulfate, filtered, and concentrated *in vacuo* to afford 4.61 (9.55 mmol) of the desired product as a pale yelllow foam.

**Example 193**

The product of Examples 21 and 32 was also prepared as follows:

a)

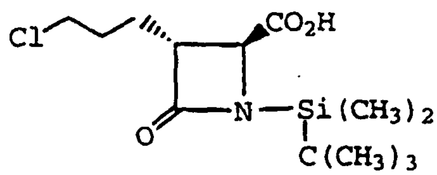

Diisopropylamine (14.05 ml, 0.10 mmol) was added to a dry, three-necked flask equipped with mechanical stirrer and maintained under an argon atmosphere. Tetrahydrofuran (anhydrous, 33 ml) was charged to the flask and while the mixture was stirred and cooled to -20°C, a solution of *n*-butyl lithium (38.3 ml of a 2.5 M solution in hexanes, 0.096 mol) was added dropwise and the solution was stirred at -20°C for 10 minutes. A solution of (4S) -N-(t-butyldimethylsilyl)azetidine-2-one-4-carboxylic acid (10.0 g, 43.6 mmol) [Baldwin et al, Tetrahedron, Vol. 46, p. 4733 - 4748, 1990] was added slowly while maintaining the temperature at -20°C and allowed to stir for 30 minutes. A solution of 1-chloro-3-iodopropane (5.6 ml, 52 mmol, 1.2 eq.) in tetrahydrofuran (30 ml) was added over approximately 10 minutes. After stirring at -20°C for approximately 2 hours. 2.6M potassium bisulfate (75 ml), water (100 ml) and ethyl acetate (100 ml) were added and the mixture was transferred to a separatory funnel. The aqueous layer (pH 2 -3) was drawn off and back extracted with ethyl acetate (2 x 50 ml). The organic solutions were combined and washed sequentially with water (2 x 50 ml), 10% sodium thiosulfate (1 x 50 ml) and then with saturated sodium chloride to neutral pH. The organic solution was dried over sodium sulfate (15 g), filtered and concentrated to an oil. The oil was seeded with a few crystals of the desired product and placed under high vacuum overnight to dry. The resultant crystalline solid was slurried with hexane (100 ml), filtered, washed with hexane (100 ml) and dried under high vacuum to give 11.5 g of the desired product.

b)

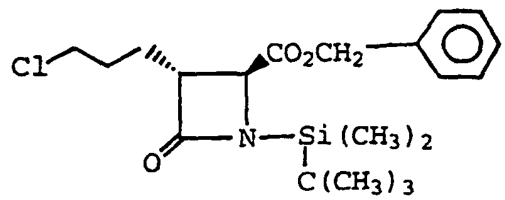

The product from part (a) (7.02 g, 22.9 mmol) was dissolved in methylene chloride (anhydrous, 40 ml) under an argon atmosphere. The solution was stirred and cooled to 0°C, and triethylamine (3.5 ml, 25.2 mmol) was added slowly while maintaining approximately 0°C. Benzylchloroformate (3.6 ml, 25.2 mmol) was added along with an additional 10 ml of methylene chloride to aid stirring. 4-Dimethylaminopyridine (2.8 g, 22.9 mmol) was added as a solid in one portion with considerable gas evolution. The solution was allowed to stir at 0°C for 30 minutes. Additional benzylchloroformate (0.3 ml, 2.5 mmol) was added and the reaction was stirred for an additional 20 minutes. The reaction was quenched with 1M potassium bisulfate (30 ml). The mixture was transferred to a separatory funnel and the layers were separated. The organic layer was washed with 2N potassium bisulfate (20 ml), and the aqueous washes were combined and back extracted with methylene chloride (25 ml). The organic solutions were combined, washed sequentially with water (25 ml), saturated sodium bicarbonate (25 ml), and saturated sodium chloride (2 x 25 ml), and dried over sodium sulfate (15 g). The methylene chloride solution was filtered through silica (15 g) and the silica was washed with 200 ml 3:1 (volume:volume) hexane/ethyl acetate. The filtrate was concentrated to an oil, evaporated under reduced pressure from toluene (2 x 25 ml) and dried under vacuum overnight to give 8.12 of the desired product.

c)

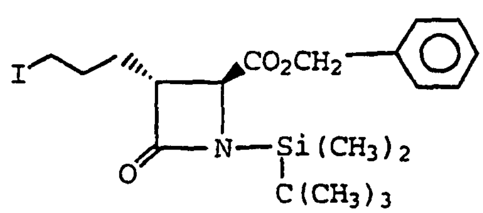

A solution of the product from part (b) (10.55 g, 26.6 mmol) in 4-methyl-2-pentanone (40 ml) was stirred under an argon atmosphere. Sodium iodide (20 g, 133 mmol) was added and the mixture was heated at approximately 110°C, protected from light, for 7 hours. The mixture was cooled to ambient temperature, diluted with hexane (100 ml) and filtered through a plug of Celite®. The Celite® was washed with hexane (2 x 50 ml). The filtrates were combined, washed with sodium thiosulfate (50 ml) and then with water (50 ml). The aqueous washes were back extracted with ethyl acetate (100 ml). The organic solutions were combined and concentrated to an oil, dissolved in hexane (50 ml) and filtered through a plug of silica. The silica was washed with hexane (200 ml) and then with 4:1 (volume:volume) hexane/ethyl acetate (500 ml). The hexane/ethyl acetate solution was concentrated to give 11.84 g of the desired product as an oil.

d)

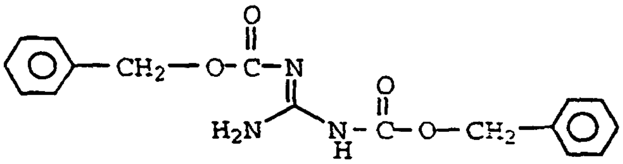

The compound of the formula

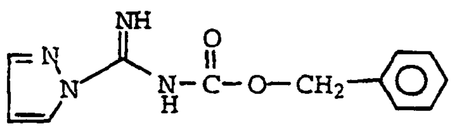

(5 g, 20.5 mmol) and tetrahydrofuran (27 ml, anhydrous) were charged to a dry flask under nitrogen. The solution was stirred and cooled to 0°C. Sodium hydride (2.62 g, 65.5 mmol, 3.2 equivalents of a 60% dispersion in mineral oil) was charged to the flask slowly (exotherm). The suspension was stirred at 0°C. N-(Benzyloxycarbonyloxy) succinimde (8.2 g, 32.8 mmol, 1.6 eq.) was added portionwise maintaining a temperature of approximately 0°C. The cooling was removed and the reaction was allowed to warm to room temperature. After 1 hour, an additional amount of N-(benzyloxycarbonyloxy)succinimide (1g, 0.2 eq) was added and the reaction was stirred at room temperature overnight. The reaction was worked up by cooling to approximately 0°C and quenched slowly by the addition of 13% aqueous ammonium chloride. The layers were separated, and the aqueous layer was back extracted with ethyl acetate (3 x 20 ml). The combined organic solution was washed sequentially with 13% aqueous ammonium chloride (5 ml), water (15 ml) and saturated sodium chloride (2 x 15 ml). The organic solution was dried over sodium sulfate, filtered and concentrated. The resulting crude oil was charged to a flask with a 2M solution of ammonia in methanol (51 ml, 101 mmol, 5 eq) and allowed to stir at ambient temperature overnight. The reaction was worked up by concentration under reduced pressure, followed by coevaporation with hexanes (2 x 25 ml). The resultant material was dissolved in methylene chloride (50 ml) and washed with water (50 ml). The aqueous layer was back extracted with methylene chloride (2 x 50 ml). The organic extracts were combined and washed with water (25 ml) and saturated sodium chloride (25 ml), dried over sodium sulfate, filtered and concentrated to a solid. The solid was crystallized from ethyl acetate to give 4.65 g of the desired product as white crystals.

e)

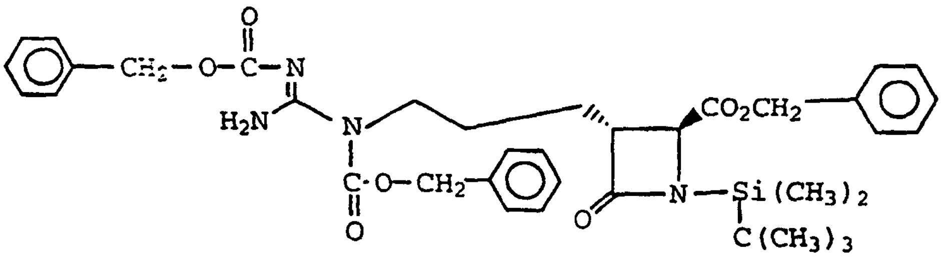

The product from part (d) (6.01 g, 18.37 mmol) was dissolved in 1-methyl-2-pyrrolidinone (anhydrous, 10 ml)

and warmed to 35 - 40°C under an argon atmosphere. Potassium carbonate (finely ground and dried, 12.7 g, 92 mmol) was added and the mixture was allowed to stir for 25 minutes. A solution of the product from part (c) (9.5 g, 18.37 mmol)in 1-methyl-2-pyrrolidone (10 ml) was added and the reaction mixture was allowed to stir for 8 hours at 35 - 40°C. The reaction mixture was cooled to ambient temperature, diluted with ethyl acetate (100 ml) and filtered into a separatory funnel. The mixture was washed with 1M potassium bisulfate (84 ml) and the layers were separated. The aqueous layer was back extracted with ethyl acetate (50 ml). The organic solutions were combined, washed sequentially with water (50 ml), 10% sodium thiosulfate (50 ml) and saturated sodium chloride (50 ml), dried over sodium sulfate, filtered and concentrated to an oil. The oil was dissolved in 50 ml. 1:1 (volume: volume) hexane/ethyl acetate and filtered through silica. The filtrate was concentrated to give 12.88 g of the desired product as a crude oil that was used without further purification.

f)

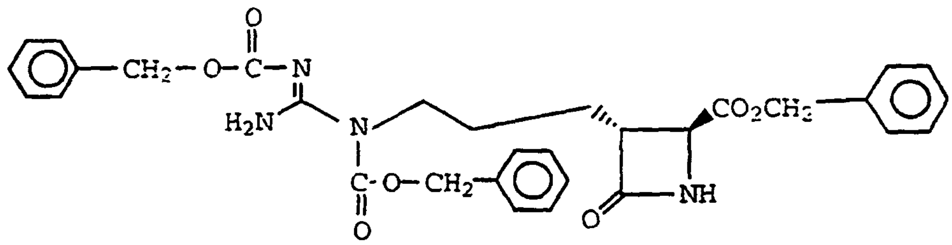

The crude oil product from step (e) was dissolved in acetonitrile (50 ml) and water (5 ml). Ammonium fluoride (3.4 g, 92 mmol) and glacial acetic acid (5.25 ml, 92 mmol) were added and the mixture was stirred for 30 minutes. The reaction was diluted with ethyl acetate (150 ml), transferred to a separatory funnel and washed with saturated sodium bicarbonate (30 ml). The layers were separated and the aqueous layer was back extracted with ethyl acetate. The organic solutions were combined, washed with saturated sodium chloride, dried over sodium sulfate, filtered and concentrated to an oil. The oil was dissolved in ethyl acetate (30 ml) and warmed to 60°C. Hexane (25 ml) was added and the mixture was allowed to cool slowly with stirring and seeding. As crystallization occurred, hexane (75 ml) was added portionwise. The resultant solid was filtered, washed with hexane, and dried to give 8.91 g of the desired product.

g)

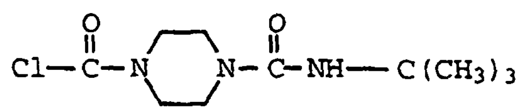

A 2 liter dried flask equipped with a mechanical stirrer and argon inlet was charged with 1-benzylpiperazine (40 ml, 230 mmol) and toluene (250 ml). With vigorous stirring *t*-butyl isocyanate (27 ml, 236 mmol) was added in rapid dropwise fashion over 15 minutes. The product precipitated to form a thick slurry. The slurry was stirred over an hour to reach 25°C. Heptane (570 ml) was added to the slurry over 30 minutes. The flask was stoppered and placed in a cold room (5°C) for 4 hours. The product was collected by filtration, rinsed with heptane (1 x 200 ml) and air dried to give 61.0 g of the piperazine of the formula

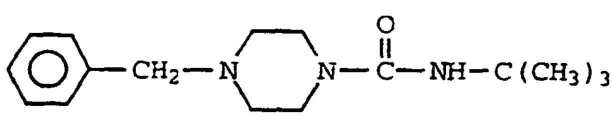

as a white solid.

A 500 ml flask equipped with a magnetic stir bar and a sparging tube was charged with methanol (200 ml). The flask was cooled to 1°C and with stirring acetyl chloride (5.7 ml. 79.9 mmol) was added over 10 minutes. The solution was allowed to reach room temperature and the above piperazine (20.0 g, 72.6 mmol) was added. Palladium hydroxide on carbon (8.0 g, moisture content less than or equal to 50%) was added and the mixture was then sparged with argon for 10 minutes. The reaction mixture was then sparged with hydrogen. After 3.5 hours, HPLC indicated the starting material was consumed completely. The mixture was filtered through Celite® and the Celite® rinsed with methanol (60 ml). The filtrate was concentrated until solid started to crystallize (175 ml methanol collected). Isopropyl alcohol (200 ml) was added slowly with manual stirring. The mixture was concentrated to a solid/liquid mixture (153 g weight). The mixture was allowed to stand for 2 hours. The product was collected by filtration, washed with isopropyl alcohol (1 x 30 ml) and air dried to yield 14.0 g of the hydrochloride salt of the formula

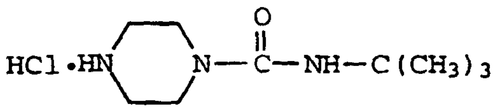

as a light yellow solid.

A 100 ml, three-necked flask with a magnetic stir bar and a sparging tube was charged with this hydrochloride salt (5.0 g, 22.6 mmol) and anhydrous methylene chloride (50 ml). 1.8-Diazabicyclo[5.4.0]undec-7-ene (6.7 ml, 45.1 mmol) and pyridine (1.8 ml, 22.6 mmol) were added to the mixture. The mixture became homogeneous. The mixture was sparged with dry carbon dioxide gas at room temperature for 1 hour.

A dry 500 ml flask with a magnetic stir bar was charged with thionyl chloride (4.9 ml, 67.7 mmol) and anhydrous methylene chloride (25 ml). The solution was cooled to -10°C and dimethylformamide (0.17 ml, 2.26 mmol) was added. The above carbon dioxide sparged mixture was added via cannula under carbon dioxide pressure over 35 minutes. The flask was rinsed with methylene chloride (5 ml) and the rinse was added to the reaction. The reaction was stirred at -10°C for 30 minutes. The reaction mixture was poured into 0.5 M HCl (75 ml) and shaken vigorously. The organic layer was collected, dried over magnesium sulfate, filtered and concentrated *in vacuo* to give 4.95 g of the desired product as a light yellow solid.

h)

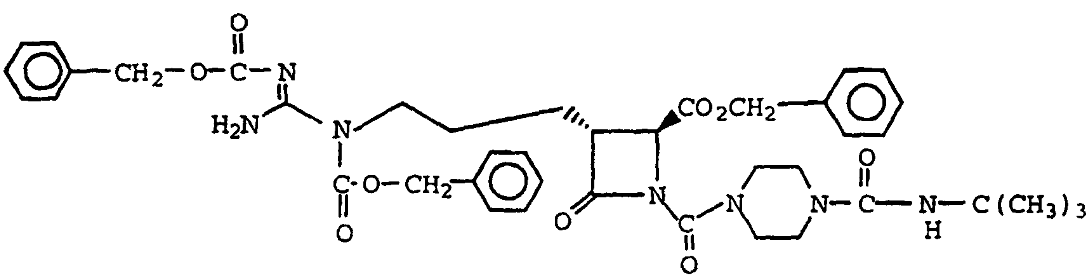

The product from step (f) (11.84 g, 20.7 mmol) was dissolved in anhydrous methylene chloride (100 ml) under argon with stirring. The carbamoyl chloride product from step (g) (7.47 g, 26.9 mmol, 1.3 eq.), triethylamine (4.6 ml, 33.1 mmol, 1.6 eq.), and 4-dimethylaminopyridine (0.76 g, 6.2 mmol. 0.3 eq) were added, and the reaction was allowed to stir at ambient temperature overnight. The reaction was poured into 0.5 N HCl (110 ml), the layers were separated, and the organic layer was washed with a second portion of 0.5 N HCl. The acidic aqueous layers were back extracted with methylene chloride (50 ml) and combined with the main organic portion. The combined organic layers were washed with saturated sodium bicarbonate (100 ml) and saturated sodium chloride, and dried over sodium sulfate. The solution was filtered and concentrated to give 17.0 g of the desired product as a crude white solid.

i)

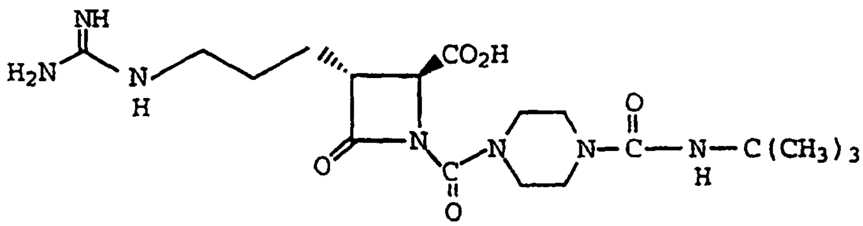

The crude product from part (h) (17.0 g) was dissolved in absolute ethanol (350 ml) with stirring. The solution was sparged with argon and 10% palladium on carbon catalyst (1.7 g, 50% by weight water) was added in one portion followed by additional argon sparging. The solution was sparged with hydrogen for 2 minutes, and then placed under atmospheric hydrogen pressure (balloon). Two additional charges of palladium on carbon catalyst (1.7 g each) were added to the reaction, along with a repeat of the sparging procedure. The reaction was judged complete in approximately 4 hours (HPLC analysis). The reaction was sparged with argon for 5 minutes and filtered through a packed pad of Celite®. The Celite® was washed with ethanol (2 x 125 ml). The combined ethanol filtrates were concentrated to approximately 50 g and allowed to stir for 4 days. Crystals formed in the flask. The crystals were filtered, washed with absolute ethanol (25 ml) and dried to give 7.74 g of the desired product as white crystalline material. This material was further purified by warming in 95% ethanol to approximately 40°C for 30 minutes, followed by cooling, filtration and drying.

**Example 194**

The product of Examples 21, 32 and 193 was also prepared as a zwitterion or inner salt as follows:

a)

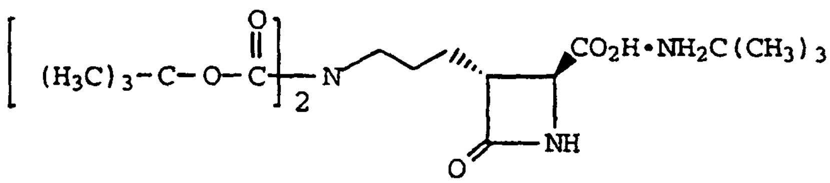

A dry, 3-necked, 12-L flask (flask A) was charged with 769.4 g of (4S)-N-(t-butyldimethylsilyl)azetidinone carboxylic acid followed by 6L of dry tetrahydrofuran. A separate 3-necked, 5-L flask (flask B) was charged with 1537.8 g of the iodo product from Example 192 step (b) followed by 2L of dry tetrahydrofuran. Under a nitrogen atmosphere, 4L of dry tetrahydrofuran was charged into a 22-L flask (flask C) followed by 3.69 L of lithium diisopropylamide. The solution of lithium diisopropylamide was cooled to -30 to -35°C. While maintaining the temperature at less than -20°C, the contents of flask A were added to flask C. The mixture was stirred at -20 to -25°C for 30 to 60 minutes and cooled to -35°C to -40°C. The contents of flask B were then added portionwise over 25 to 45 minutes while maintaining the internal temperature of flask C at less than about -20°C. The resulting mixture was stirred for 2 to 3 hours between -20°C and -23°C. The reaction was quenched by the addition of 6L of cold water while maintaining the internal batch temperature at -20°C to +5°C. After stirring for an additional 15 to 30 minutes to ensure removal of the silyl protecting group, the pH was adjusted to 8.0 by the addition of cold 6N HCl(1.69 L). The reaction mixture was transferred to a phase splitter and the top organic layer was discarded. The aqueous layer was washed twice with 4L portions of hexane. The aqueous phase was cooled to about 0°C and treated with 6N HCl (about 400 ml) until the pH was 3.0. The batch temperature was maintained at less than 5° during this operation. The cloudy aqueous phase was extracted three times with 4L portions of ethyl acetate. The combined organic extracts were washed with brine (3 x 3L) and concentrated to an oil. The oil was redissolved in 8L of fresh ethyl acetate, transferred to a 22-L flask, and cooled under nitrogen. While maintaining the batch temperature at less than 8°C, *tert*-butylamine was added and the resulting mixture was stirred overnight at room temperature. The mixture was concentrated to a yellow slurry, treated with 6L of methyl *tert*-butyl ether and stirred for 3 hours at room temperature. The mixture was filtered and the filter cake was washed with methyl *tert*-butyl ether (1.5 L) and dried to a constant weight of 805.9 g of the desired product.

b)

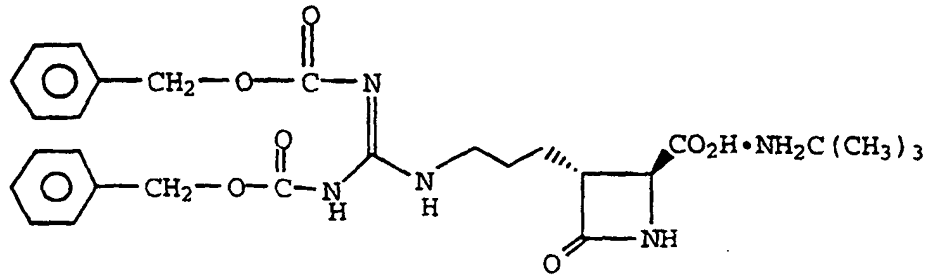

Trifluroacetic acid (31.1 ml, 403.9 mmol, 18 equivalents) was added dropwise to a suspension of the product from part (a) (10.0 g, 22.44 mmol) in methylene chloride (40 ml) at between -5°C to +5°C under nitrogen. The resulting light yellow clear solution was stirred at 0°C until less than 1% of the mono *tert*-butoxycarbonyl intermediate was detected by HPLC (about 4 to 7 hours). The methylene chloride and trifluoroacetic acid were removed *in vacuo* at room temperature. Toluene (30 ml) was added to the residue and then removed *in vavuo*. The residue was treated with isopropyl alcohol (10 ml) followed by toluene (20 ml) and the resulting solution was concentrated *in vacuo* to an oil. This step was repeated one time.

Isopropyl alcohol (50 ml) was added to the above oil (about 23 g) and the resulting solution was cooled to 0°C. The pH was adjusted to 8.5 to 9.0 by the dropwise addition of triethylamine between -5°C to 5°C (18 ml of triethylamine was used in this procedure). N,N'-Bis(benzyloxycarbonyl)-1-guanylpyrazole (8.07 g, 21.32 mmol, 0.95 equivalents) was added in one portion and the cooling bath was removed. The mixture was stirred under nitrogen at room temperature for approximately 30 hours until the ratio of product/pyrazole was greater than 25: 1 as determined by HPLC. The solvent was removed *in vacuo* at room temperature to afford approximately 42 g of yellow oil. This oil was diluted with ethyl acetate (70 ml) and water (70 ml), and cooled to 0°C. The pH of the solution was adjusted to 3.0 with 2M potassium bisulfate and treated with sodium chloride until saturated. The organic layer was separated and the aqueous layer was extracted with ethyl acetate (2 x 60 ml). The combined ethyl acetate layers were washed with saturated sodium chloride solution (2 x 60 ml), dried over sodium sulfate and filtered. The solvent was evaporated to give a yellow oil (14.4 g) which was redissolved in ethyl acetate (40 ml). The resulting clear yellow solution was warmed to 36 - 40°C and treated dropwise with *tert*-butylamine (3.3 ml). After crystallization of the salts, the slurry was cooled to room temperature, stirred for 12 hours, then cooled to 4°C, and stirred for an additional 12 hours. The product was filtered, washed with cold ethyl acetate/hexane (2 x 5 ml) and cold hexane (2 x 5 ml), and dried *in vacuo* to give 8 g of the desired product.

c)

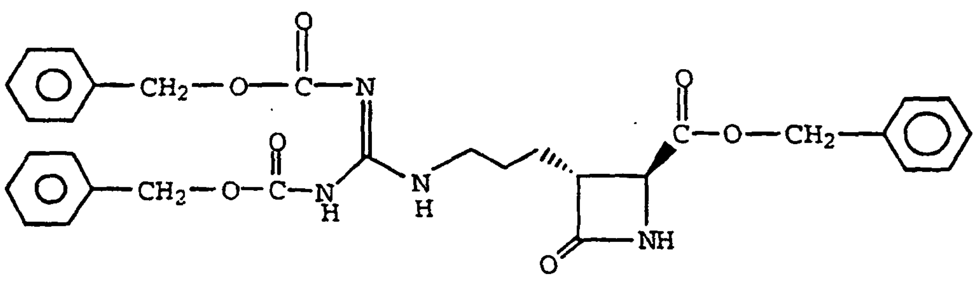

A dry, argon atmosphere 50 ml flask was charged with the product from part (b) (5.0 g). N,N'-Dimethylpropyleneurea (15 ml) was added, and the mixture was stirred for 5 minutes. The system was not homogeneous at this time. A 22°C water bath was applied to the flask, and benzyl bromide (2.1 ml, 1.96 equivalents) was added rapidly (no exotherm was observed). *tert*-Butylamine (0.90 ml, 0.95 equivalents) was added dropwise (the temperature rose to 27.5°C during the addition, held there for approximately 1 minute after the addition was complete, and then began to fall). When the temperature dropped to 25°C, the water bath was removed and the reaction was stirred overnight (16 hours). Completion of the reaction was confirmed by HPLC analysis. The reaction was diluted with ethyl acetate (30 ml) and *tert*-butyl methyl ether (30 ml). This solution was washed three times with 5% citric acid (1 x 30 ml, 2 x 15 ml) and then washed with saturated sodium chloride (1 x 15 ml). The resulting solution was dried

over magnesium sulfate, filtered and concentrated *in vacuo* to give 5.85 g of the desired product as an orange oil.

d)

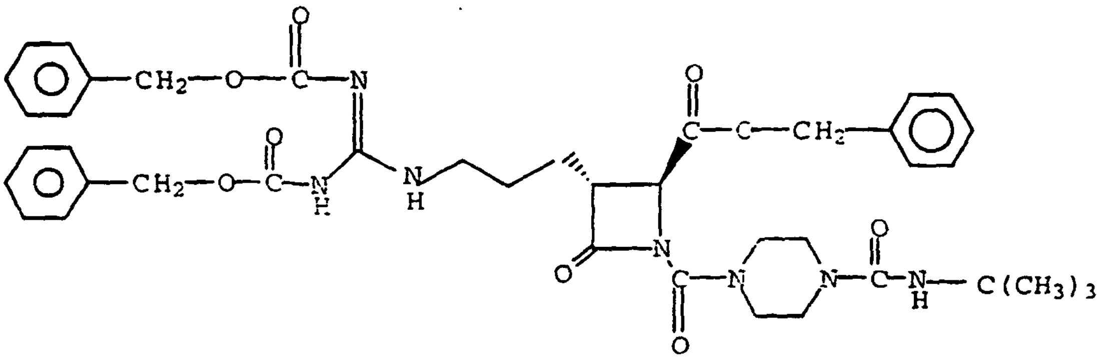

A dry, argon atmopsphere 50 ml flask was charged with the product from part (c) (2.5 g), ethyl acetate (35 ml), triethylamine (0.98 ml, 1.6 equivalents), and 4-dimethylaminopyridine (0.16 g, 0.3 equivalents). The reaction was stirred overnight (approximately 20 hours). HPLC analysis confirmed that the reaction was 99.4% complete. The reaction was filtered through a fine glass frit and the solids were washed twice with ethyl acetate. The filtrate was washed with 5% citric acid (2 x 15 ml) and once with saturated sodium chloride (15 ml). The organic phase was dried over magnesium sulfate, filtered and concentrated *in vacuo* to afford 3.46 g of the desired product as a tan foam.

e)

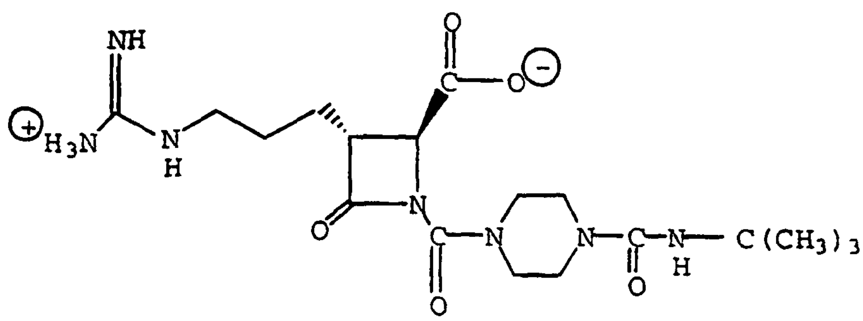

A 12 L three necked round bottom flask was charged with 10% palladium on carbon catalyst (50.34 g, 47.31 mmol), water (362 ml), ethanol (6883 ml), and the product from part (d) (345 g). The mixture was agitated and sparged with nitrogen for approximately 20 to 30 minutes. then continuously sparged with hydrogen gas at 15° to 25°C until HPLC analysis confirmed completion of the reaction. The reaction mixture was sparged with nitrogen for approximately 20 - 30 minutes, filtered, and the filter was washed 2L ethanol/water (95/5). The ethanol/water was partially concentrated *in vacuo* at room temperature to a solution of 8 to 10 ml per gram of product. Concentration gave a cloudy to white solution.

The above solution was allowed to crystallize overnight at room temperature with agitation (120 - 200 revolutions per minute). The product was filtered and the filter cake was washed three times with 300 ml of cold ethanol/water (95/5, 0° to 5°C). The filter cake was dried *in vacuo* for 10 to 20 minutes. The resulting solid was dried to constant weight in a vacuum oven at room temperature to give 155 g of the desired final product as a white solid.

Example 197

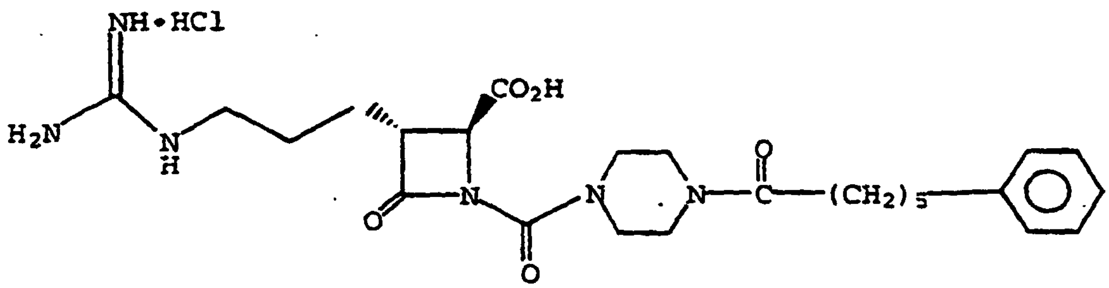

(homochiral)

a)

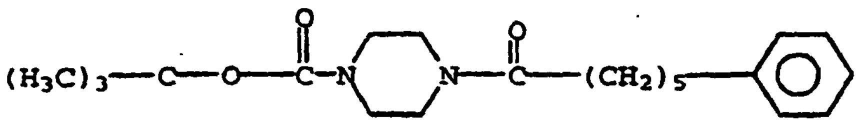

To a solution of 6-phenylhexanoic acid (4.0 g, 20.81 mmol) and hydroxybenztriazole (3.50 g, 22.89 mmol) in anhydrous methylene chloride (100 ml) was added ethyl-3-(dimethylamino)propyl carbodiimide, hydrochloride salt (4.39 g, 22.89 mmol) at 0°C. The mixture was stirred for 30 minutes. 1-tert-Butoxycarbonylpiperazine (3.88 g, 20.81 mmol) and diisopropylethylamine (4.35 ml, 24.97 mmol) were added and the mixture became a homogeneous solution. The solution was slowly warmed to room temperature over 3 hours and stirred overnight. The solvent was replaced with ethyl acetate (300 ml). The resulting solution was washed with 0.25 M potassium bisulfate (pH of 3 to 4), saturated sodium bicarbonate (pH of 9 to 10), and brine, dried over magnesium sulfate, and concentrated to give the desired product in crude form as a colorless oil.

b)

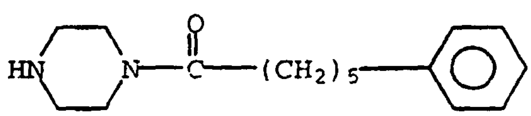

The crude product from part (a) was dissolved in methylene chloride (160 ml). The solution was cooled to 0°C and trifluoroacetic acid (40 ml) was added dropwise. The ice-bath was removed. The mixture was stirred at room temperature for one hour. The solvents were removed under vacuum. The residue was diluted with ethyl acetate (200 ml). The solution was neutralized with saturated sodium bicarbonate (pH of 10). The aqueous layer was extracted with ethyl acetate (2 x 100 ml). The combined ethyl acetate solution was washed with brine, dried over magnesium sulfate and concentrated to give the desired product as a colorless oil.

c)

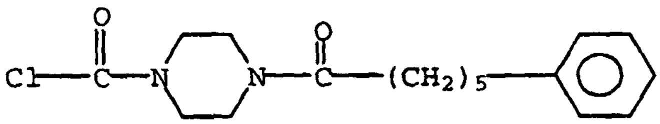

A solution of phosgene (20% in toluene, 50 mmol, 26.3 ml) was dissolved in methylene chloride (30 ml) and cooled to 0°C. A solution of the crude product from part (b) and triethylamine in methylene chloride (30 ml) was slowly added to the above solution over 20 minutes. The resulting solution was stirred at 0°C for 1.5 hours. The precipitate was removed by filtration. The filtrate was concentrated and the residue was purified with silica gel chromatography (hexane:ethyl acetate, 2: 1, $R_f$ = 0.15) to afford 5.00 g of the desired product as a white solid; $(M+H)^+$ = 323.3; IR (KBr) 1731 $cm^{-1}$.

d)

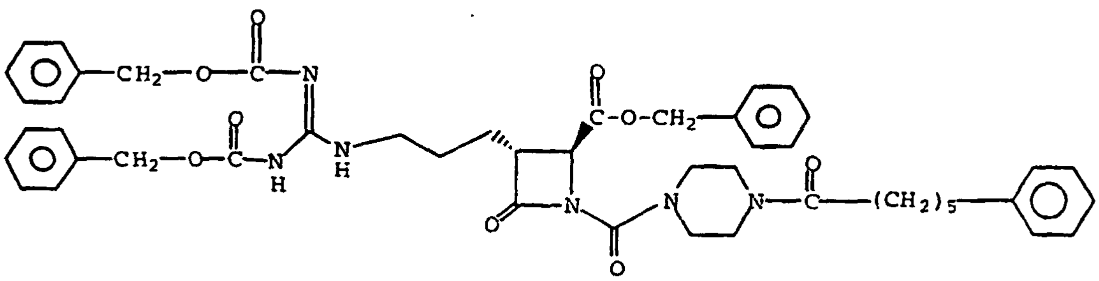

To a solution of the benzyl ester product from Example 1(c) (150 mg, 0.26 mmol) in methylene chloride (3 ml) was added triethylamine (0.043 ml, 0.31 mmol), the product from part (c) (102 mg, 0.31 mmol), and 4-dimethylaminopyridine (1.6 mg, 0.015 mmol). The solution was stirred for 3 hours and the solvent was removed. The residue was purified with silica gel chromatography (hexane:ethyl acetate, 1:1, $R_f$ = 0.22) to afford 210 mg of the desired product as a colorless oil. $(M + H)^+$ = 859.5; $(M - H)^-$ = 857.5.

e)

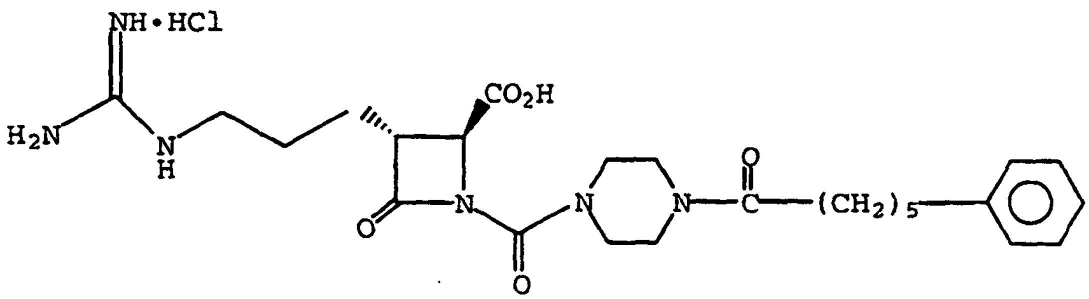

(homochiral)

A mixture of the product from part (d) (100 mg, 0.115 mmol), palladium on carbon catalyst (10%, 30 mg), and 1N HCl (115 µl, 0.115 mmol) was stirred under a hydrogen atmosphere (balloon) at room temperature for 45 minutes. Analytical HPLC indicated the reaction was completed. The reaction mixture was diluted with water (6 ml), filtered, and lyophilized to give 53 mg of the desired product as a white powder. $(M + H)^+$ = 501.3; ; $(M - H)^-$ = 499.2; IR (KBr) 1785 $cm^{-1}$.

**Example 198**

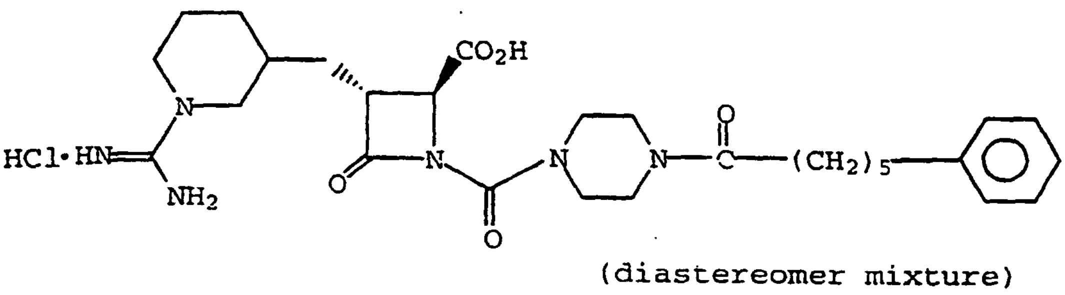

a)

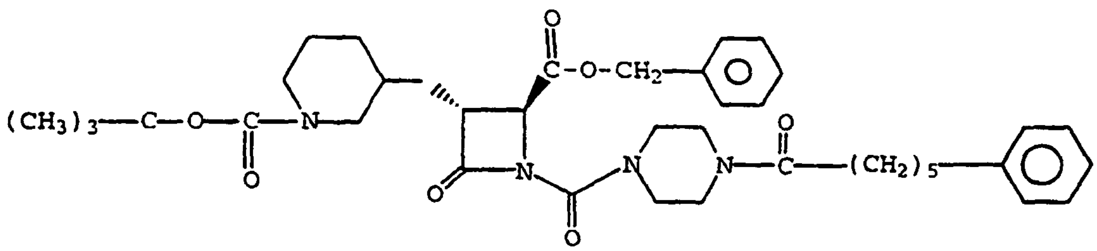

A solution of the product from Example 172(f) (80.5 mg, 0.20 mmol), triethylamine (0.056 ml, 0.40 mmol), the product of Example 197 (c) (97 mg, 0.30 mmol) and dimethylaminopyridine (6 mg, 0.05 mmol) in methylene chloride (1 ml) was stirred at room temperature under argon for 21 hours. The reaction was concentrated *in vacuo* and the residue was taken up in ethyl acetate, 10% potassium bisulfate, and water. The ethyl aceate layer was washed again with dilute potassium bisulfate, water (2x) and brine, dried over sodium sulfate and concentrated to an oil (179 mg). Chromatography of the oil over silica gel using 15% and then 25% ethyl acetate in methylene chloride provided 122 mg of the desired product as an oil.

b)

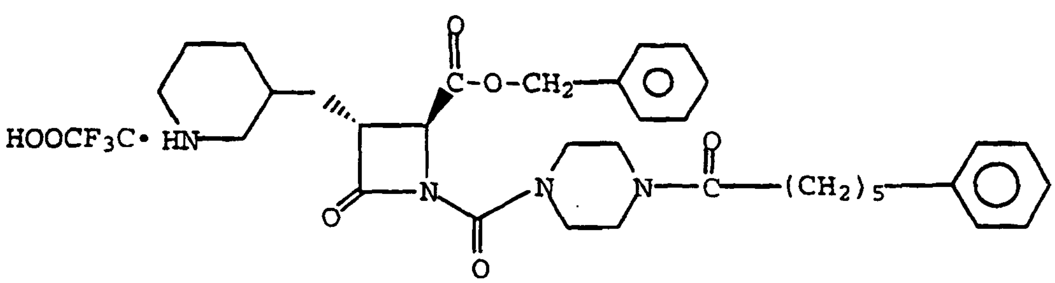

Treatment of the product from part (a) (120 mg, 0.174 mmol) with trifluoroacetic acid in methylene chloride according to the procedure described in Example 172 step (h) afforded 174 mg of the crude desired product.

c)

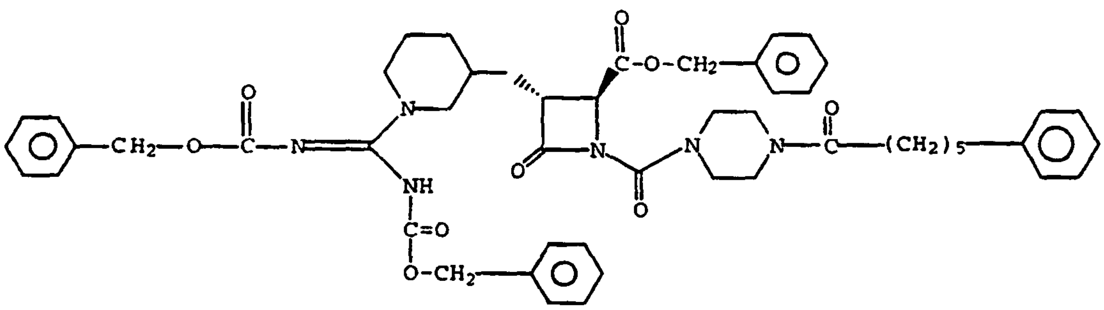

Treatment of the crude product from part (b) with N,N'-dicarbobenzyloxy-S-methylisothiourea according to the procedure in Example 172 step (i) gave 204 mg of crude product. Purification by chromatography over silica gel using methylene chloride: ethyl acetate (3:1) gave 79 mg of the desired product as an oily residue.

d)

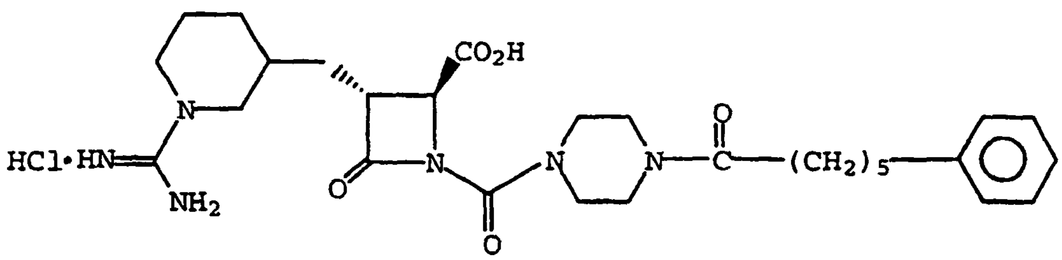

(diastereomer mixture)

The product from step (c) (76 mg, 0.085 mmol) was hydrogenated in dioxane (3 ml) and 1.0N HCl (0.085 ml, 0.085 mmol) in the presence of 10% palladium on carbon catalyst (24 mg) at 1 atmosphere of hydrogen for 1 hour. After filtration using aqueous dioxane, the filtrate was concentrated to remove dioxane, filtered and lyophilized to give 42 mg of the desired proudct as a white solid; (IR (KBr) 1784 $cm^{-1}$, consisting of a mixture (62.38) of diastereomers as determined by HPLC.

| Ex | $X_2$ |
|---|---|
| 200 | 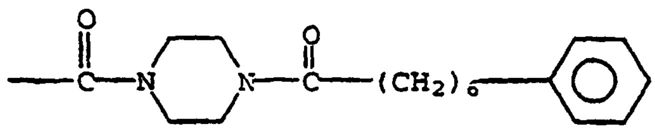 |
| 201 | 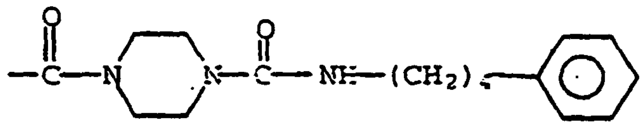 |
| 202 | 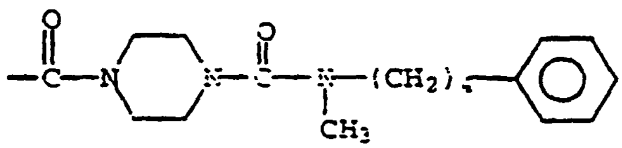 |
| 203 | 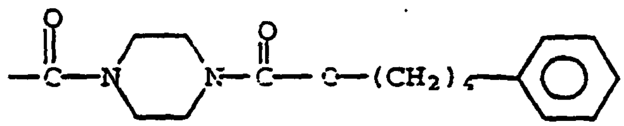 |
| 204 | 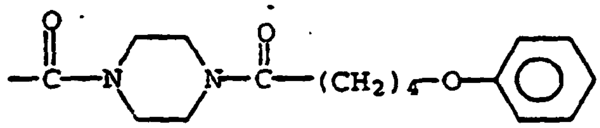 |
| 205 | 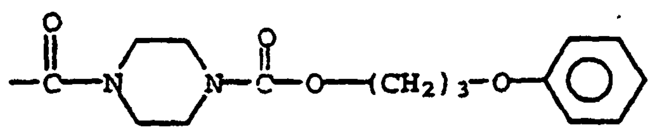 |
| 206 | 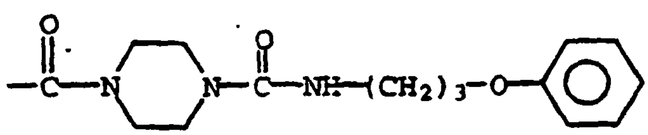 |

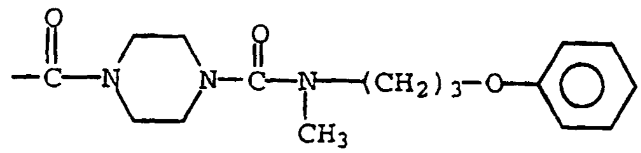

207

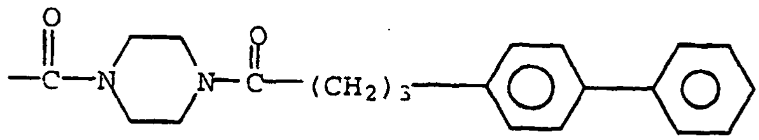

208

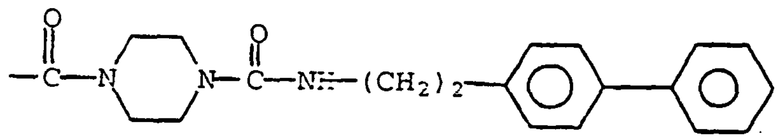

209

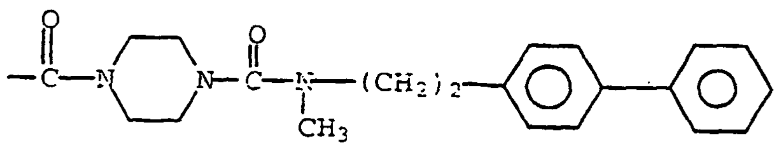

210

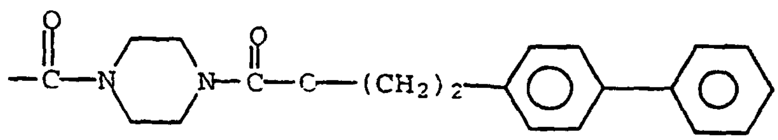

211

## Claims

**1.** A compound of the formulas

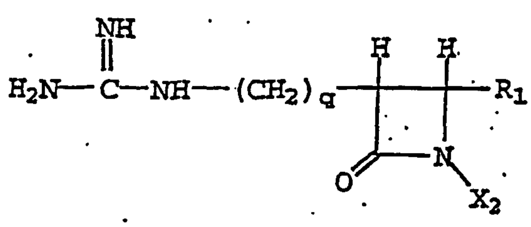

(IV)

;

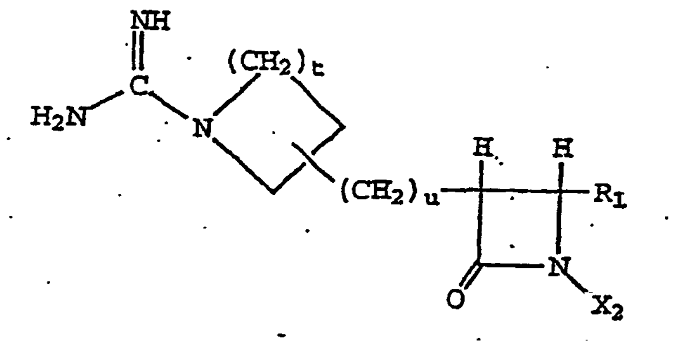

(I')

including an inner salt or a pharmaceutically acceptable salt thereof wherein:

q is 3; t is two or three, u is one;

$R_1$ is carboxy,

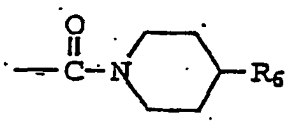,

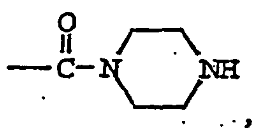,

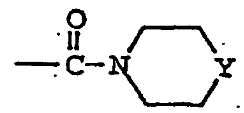,

or

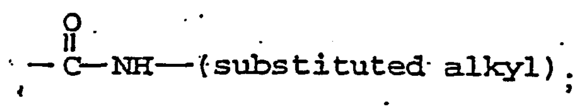;

$X_2$ is

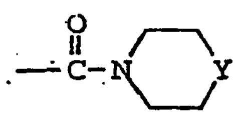,

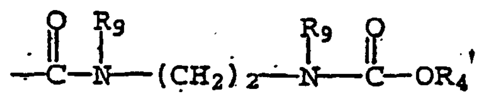,

or

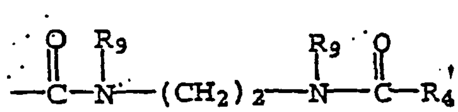;

$R_6$ is aminocarbonyl,

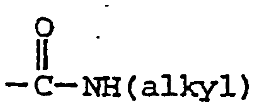, or 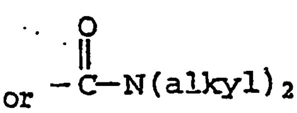;

Y is N-$R_4'$,

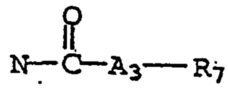, 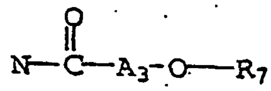

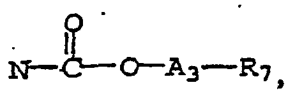, 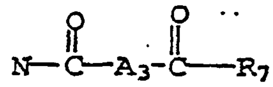,

or

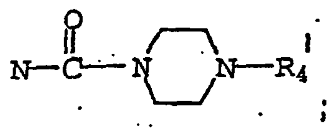;

$R_4'$ in the definition of Y and $X_2$ is alkyl, cycloalkyl, substituted alkyl, substituted cycloalkyl, or heteroaryl;

- $R_7$ is alkyl, cycloalkyl, substituted alkyl, substituted cycloalkyl, phenyl, $-(CH_2)_{1\text{ to }4}$-phenyl, $-(CH_2)_{1\text{ to }4}$-phenyl-$A_3$-phenyl,

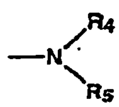 or 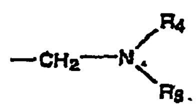;

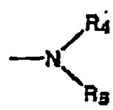

is amino, -NH(alkyl) or $-N(alkyl)_2$-;

$R_9$ is $C_1$-$C_4$ alkyl;
$A_3$ is a bond, an alkylene bridge of 1 to 6 carbons,

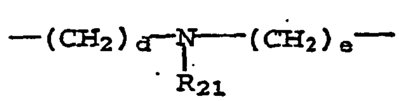

or $-(CH_2)_d$-O-$(CH_2)_e$- ;
d and e are independently selected from zero and an integer from 1 to 6; and
$R_{21}$ is hydrogen or $C_1$-$C_4$ alkyl, wherein

The term "substituted alkyl" refers to such straight or branched chain radicals of 1 to 10 carbons wherein one or more, hydrogens have been replaced by a hydroxy, amino, cyano, halo, trifluoromethyl, nitro, -NH($C_1$-$C_4$ alkyl), -N($C_1$-$C_4$ alkyl)$_2$, alkoxy, alkylthio, carboxy, alkoxycarbonyl, aminocarbonyl, or alkoxycarbonylamino;

The term "substituted cycloalkyl" refers to rings of 3 to 7 carbons having one or more substituents selected from $C_1$-$C_4$ alkyl, $C_1$-$C_4$ alkoxy, $C_1$-$C_4$ alkylthio, halo, hydroxy, trifluoromethyl, nitro, cyano, amino; -NH($C_1$-$C_4$ alkyl), -N($C_1$-$C_4$ alkyl)$_2$. or carboxy as well as such rings fused to a phenyl ring; and

the term "heteroaryl" refers to unsaturated and partially saturated rings of 4 to 7 atoms containing one or two O and S atoms and/or one to four N atoms, one to three N atoms when the ring is 4 atoms, provided that the total

number of hetero atoms in the ring is 4 or less, 3 or less when the ring is 4 atoms, and the heteroaryl ring is attached by way of an available carbon or nitrogen atom, preferred bicyclic rings are 2- and 3-indolyl and 4-and 5-quinolinyl, and the mono or bicyclic heteroaryl ring can also be additionally substituted at one or more available carbon atoms by a $C_1$-$C_4$ alkyl, halo, carboxy, hydroxy, $A_2$-$C_1$-$C_4$ alkoxy, $A_2$-guanido, benzyl or cyclohexylmethyl, and, if the mono or bicyclic ring has an available N-atom such N atom can also be substituted by an N-protecting group selected from benzyloxycarbonyl, *tert*-butoxycarbonyl, benzyl or benzhydryl.

**2.** A compound of claim 1

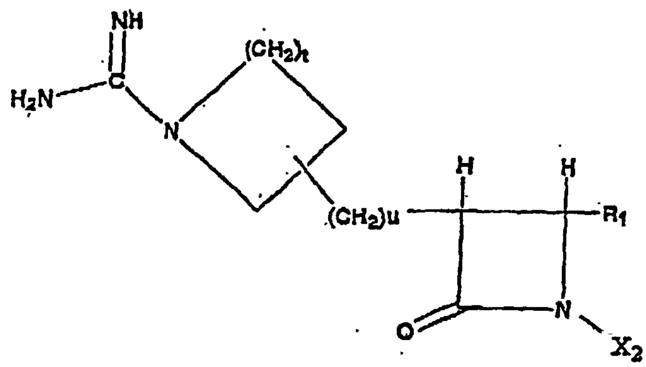

including an inner salt or pharmaceutically acceptable salt thereof wherein:

$R_1$, $X_2$, t and u, are as defined in Claim 1;

**3.** A compound of Claim 1 including an inner salt or a pharmaceutically acceptable salt thereof selected from the group consisting of

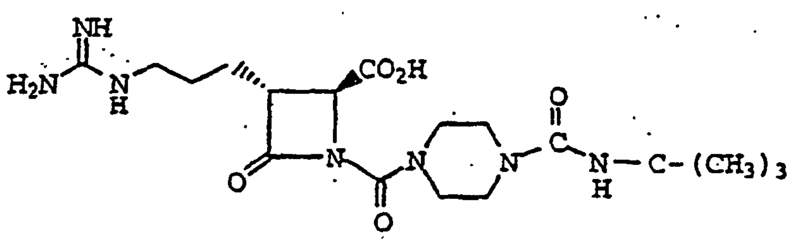

;

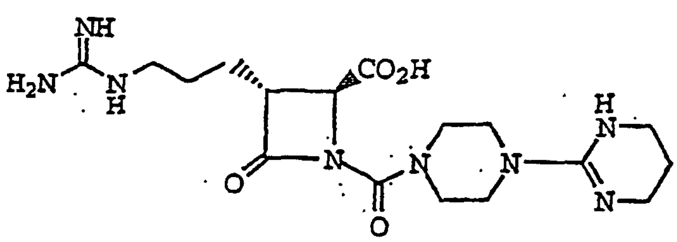

;

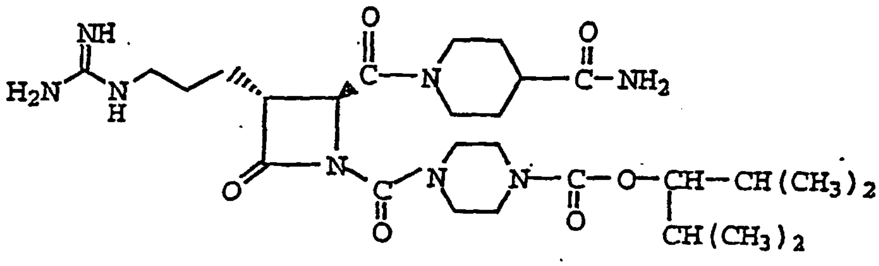

;

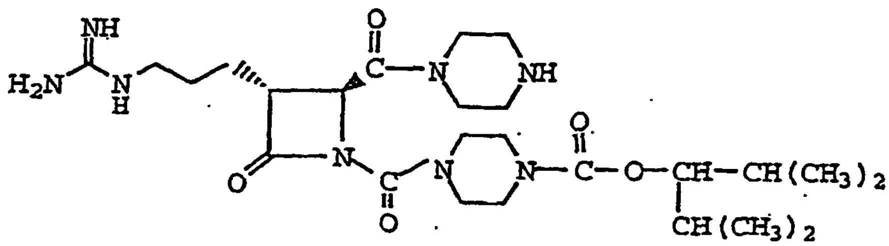

;

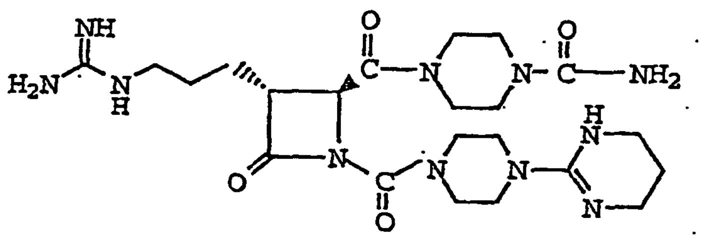

;

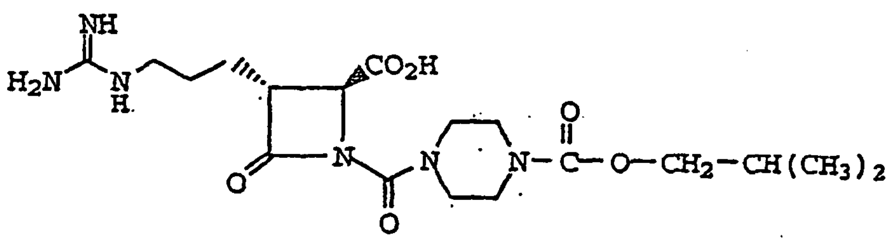

;

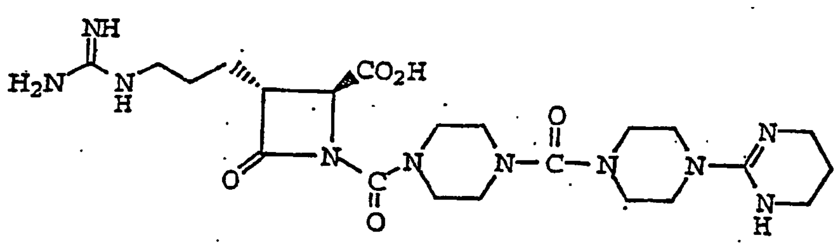

;

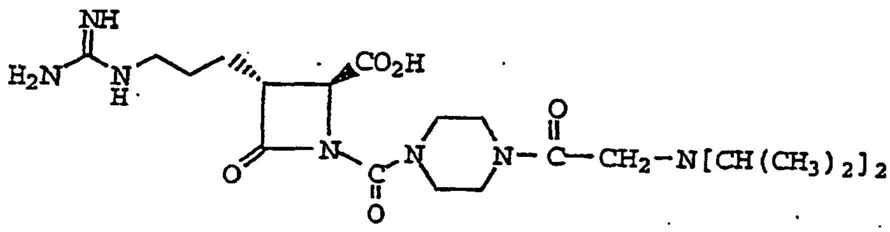

;

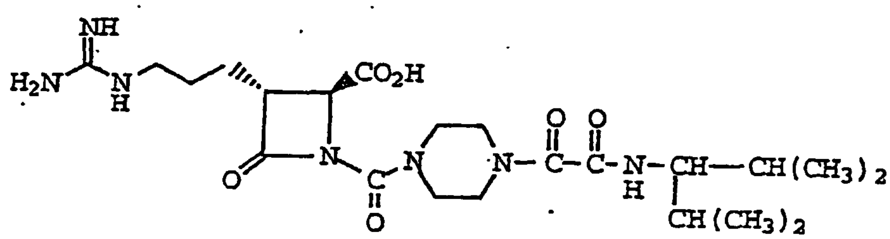

;

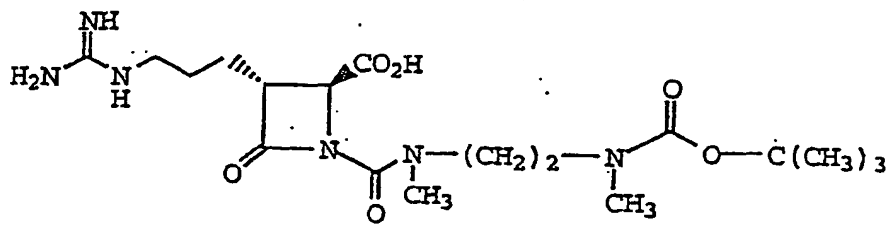

;

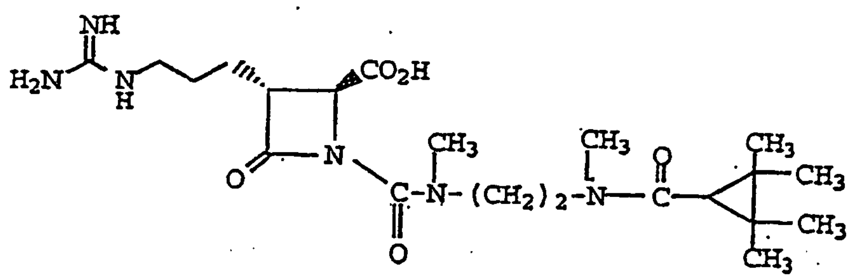

;

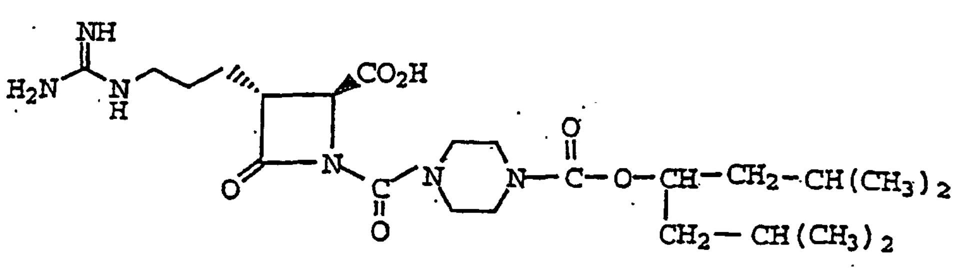

;

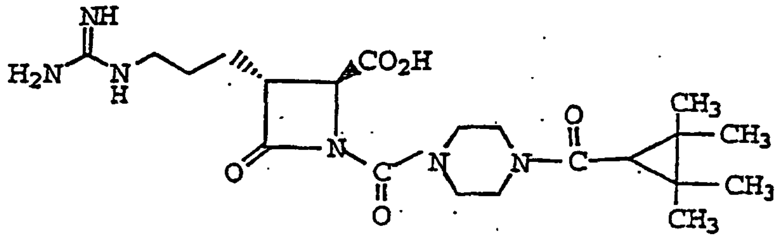

;

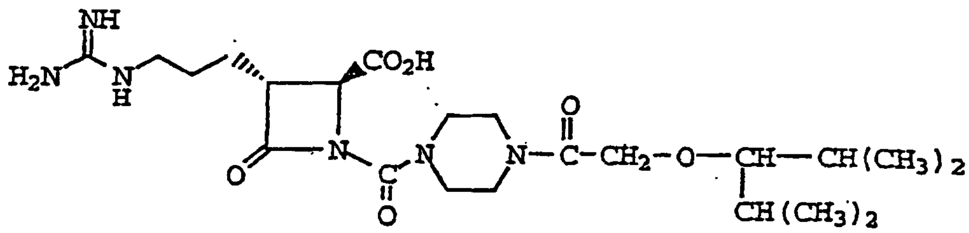

;

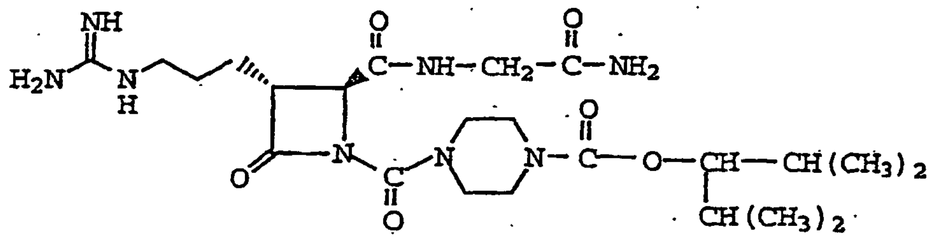

;

and

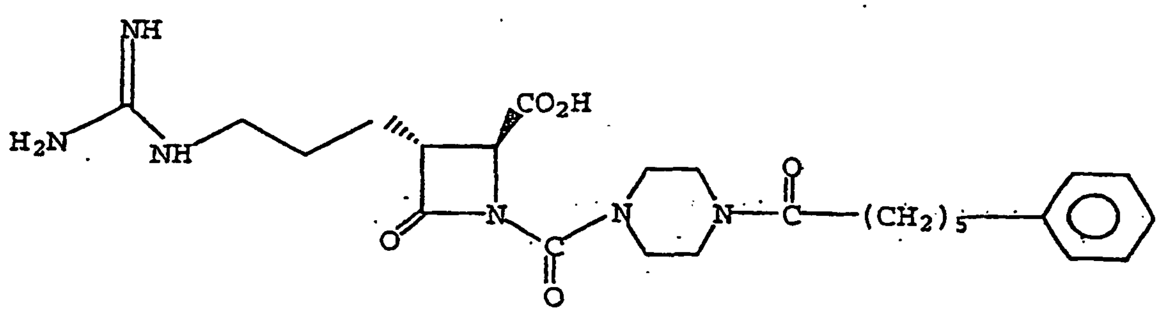

**4.** The compound of Claim 3

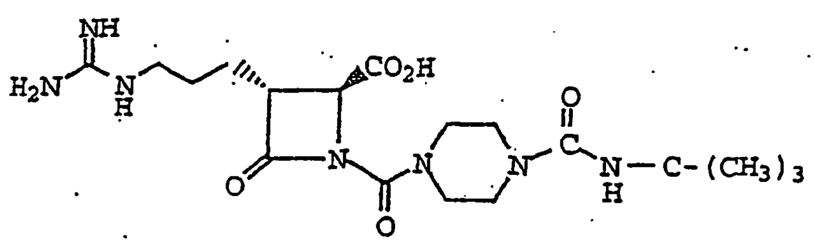

including an inner salt or a pharmaceutically acceptable salt thereof.

5. The compound of Claim 3

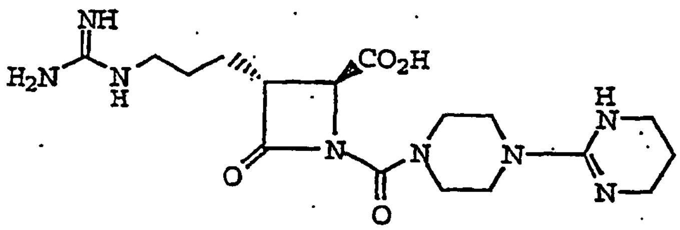

including an inner salt or a pharmaceutically acceptable salt thereof.

6. The compound of Claim 3

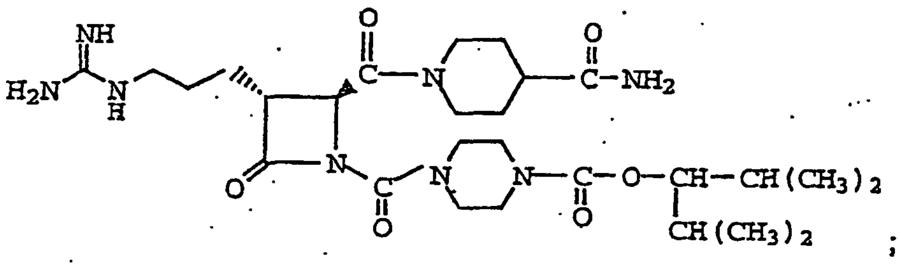

or a pharmaceutically acceptable salt thereof.

7. The compound of Claim 3

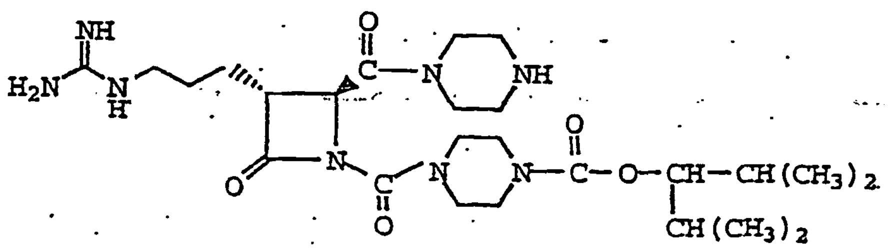

or a pharmaceutically acceptable salt thereof.

8. The compound of Claim 3

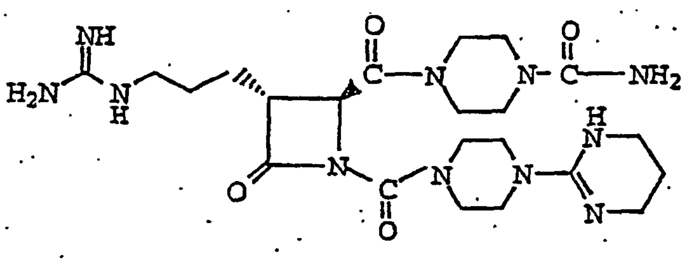

or a pharmaceutically acceptable salt thereof.

9. The compound of Claim 3

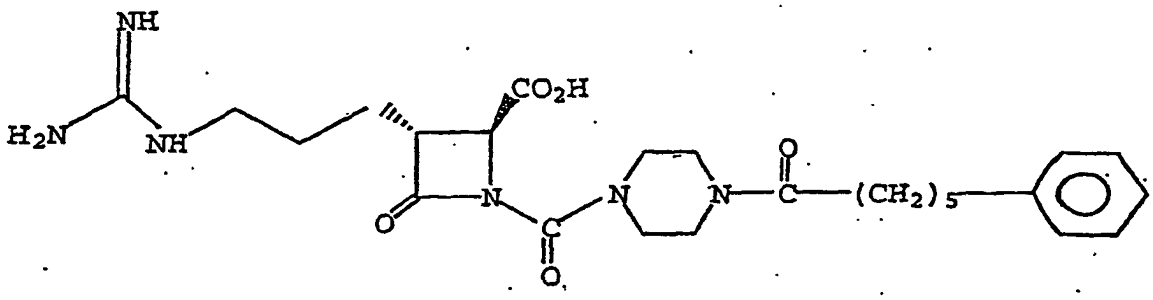

or a pharmaceutically acceptable salt thereof.

**10.** The compound of Claim 1 including an inner salt or a pharmaceutically acceptable salt thereof of the formula

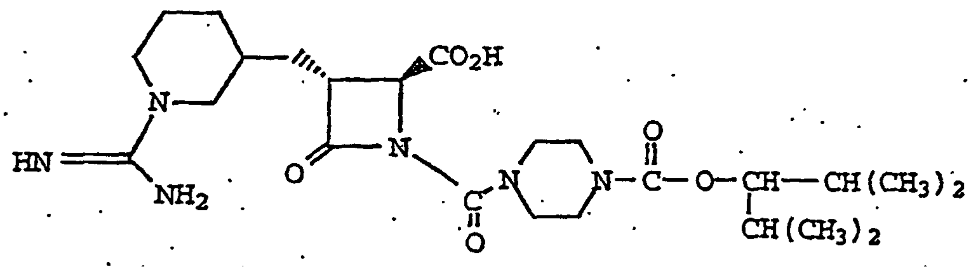

**11.** The compund of Claim 1 incuding an inner salt or a pharmaceutically acceptable salt thereof of the formula

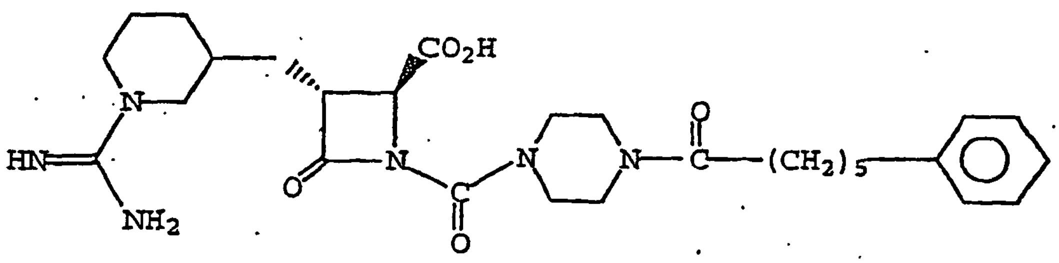

**12.** Use of a compound as defined in any one of Claims 1-11 for the preparation of a pharmaceutical composition for treating and/or preventing medical conditions in a mammalian species related to tryptase, thrombin, trypsin, Factor Xa, Factor VIIa, or urokinase-type plasminogen activator.

**13.** Use of a compound as defined in anyone of Claims 1-11 for the preparation of a pharmaceutical composition for treating and/or preventing asthma or allergic rhinitis, in a mammalian species.

**14.** Use of a compound as defined In Claim 3 for the preparation of a pharmaceutical composition for treating chronic asthma in a mammalian species wherein the composition maybe administered by inhalation to the bronchioles.

**Patentansprüche**

**1.** Verbindung der Formeln

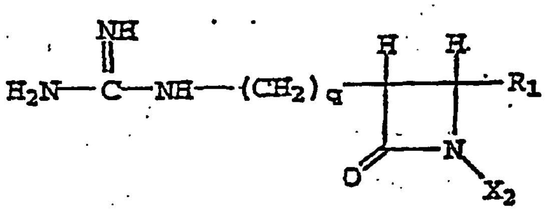 (IV)

;

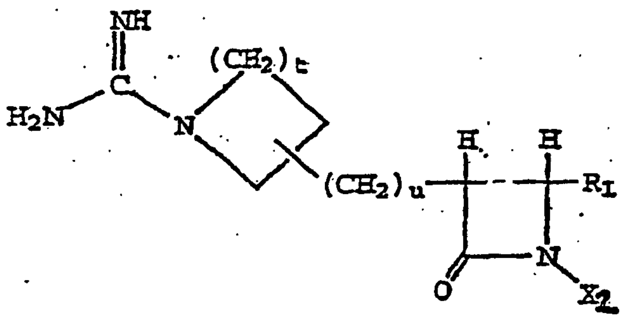 (I')

einschließlich eines inneren Salzes oder eines pharmazeutisch verträglichen Salzes davon, wobei:

q 3 ist, t zwei oder drei ist, u eins ist;
$R_1$ Carboxy,

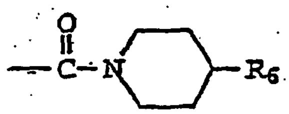 , 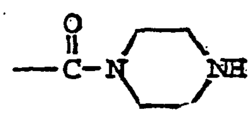 , 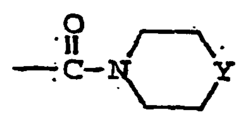 ,

oder

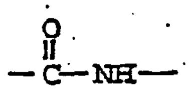 (substituiertes Alkyl) ist;

$X_2$

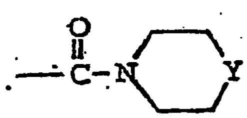 , 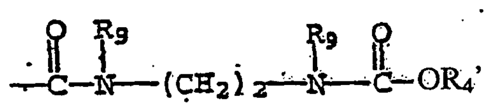

oder

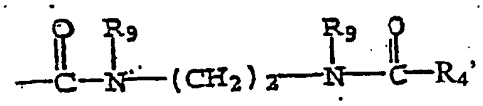

ist;
$R_6$ Aminocarbonyl,

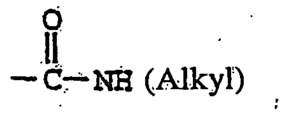

oder

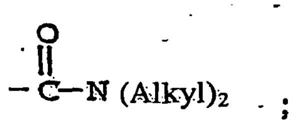

ist;
Y N-$R_4$',

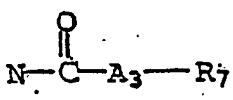 , 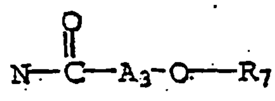

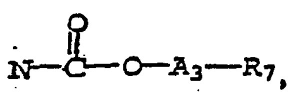 , 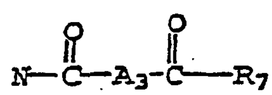

oder

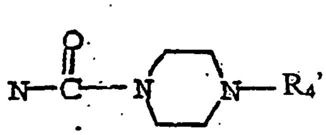

ist;
$R_4$' in der Definition von Y und $X_2$ Alkyl, Cycloalkyl, substituiertes Alkyl, substituiertes Cycloalkyl oder Heteroaryl ist;

- $R_7$ Alkyl, Cycloalkyl, substituiertes Alkyl, substiuiertes Cycloalkyl, Phenyl, $-(CH_2)_{1 \text{ bis } 4}$-Phenyl, $-(CH_2)_{1 \text{ bis } 4}$-Phenyl-$A_3$-phenyl

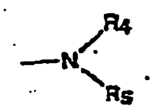

oder

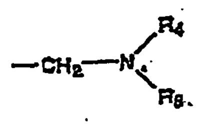

ist;

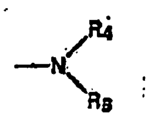

Amino, -NH-(Alkyl) oder -(N-Alkyl)$_2$- ist;
$R_9$ $C_1$-$C_4$-Alkyl ist;
$A_3$ eine Bindung, eine Alkylenbrücke mit 1 bis 6 Kohlenstoffatomen,

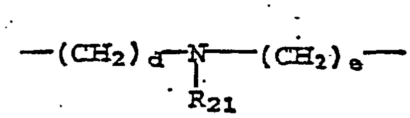

oder

$$-(CH_2)_d-O-(CH_2)_e-$$

ist;
d und e unabhängig ausgewählt sind aus Null und einer ganzen Zahl von 1 bis 6; und
$R_{21}$ Wasserstoff oder $C_1$-$C_4$-Alkyl ist, wobei

der Begriff "substituiertes Alkyl" solche geradkettigen oder verzweigten Kettenradikale mit 1 bis 10 Kohlenstoffatomen betrifft, bei denen ein oder mehrere Wasserstoffatome durch Hydroxy, Amino, Cyano, Halogen, Trifluormethyl, Nitro, -NH($C_1$-$C_4$-Alkyl), -N($C_1$-$C_4$-Alkyl)$_2$, Alkoxy, Alkylthio, Carboxy, Alkoxycarbonyl, Aminocarbonyl oder Alkoxycarbonylamino ersetzt worden sind;
der Begriff "substituiertes Cycloalkyl" Ringe mit 3 bis 7 Kohlenstoffatome betrifft, welche einen oder mehrere Substituenten aufweisen, ausgewählt aus $C_1$-$C_4$-Alkyl, $C_1$-$C_4$-Alkoxy, $C_1$-$C_4$-Akylthio, Halogen, Hydroxy, Trifluormethyl, Nitro, Cyano, Amino, -NH($C_1$-$C_4$-Alkyl), -N($C_1$-$C_4$-Alkyl)$_2$ oder Carboxy, sowie solche Ringe betrifft, welche an einen Phenylring kondensiert sind; und
der Begriff "Heteroaryl" ungesättigte und teilweise gesättigte Ringe mit 4 bis 7 Atomen betrifft, welche ein oder zwei O- und S-Atome und/oder ein bis vier N-Atome enthalten, ein bis drei N-Atome, wenn der Ring 4 Atome enthält, mit der Maßgabe, dass die Gesamtzahl an Heteroatomen im Ring 4 oder weniger beträgt, 3 oder weniger, wenn der Ring 4 Atome enthält, und der Heteroarylring über ein verfügbares Kohlenstoff- oder Stickstoffatom gebunden ist, bevorzugte bicyclische Ringe 2- und 3-Indolyl und 4- und 5-Chinolinyl sind, und der mono- oder bicyclische Heteroarylring ebenfalls zusätzlich an einem oder mehreren verfügbaren Kohlenstoffatomen durch ein $C_1$-$C_4$-Alkyl, Halogen, Carboxy, Hydroxy, $A_2$-$C_1$-$C_4$-Alkoxy, $A_2$-Guanido, Benzyl oder Cyclohexylmethyl substituiert sein kann, und wenn der mono- oder bicyclische Ring ein verfügbares N-Atom aufweist, dieses N-Atom ebenfalls durch eine N-Schutzgruppe, ausgewählt aus Benzyloxycarbonyl, tert.-Butoxycarbonyl, Benzyl oder Benzhydryl

substituiert sein kann.

2. Verbindung nach Anspruch 1

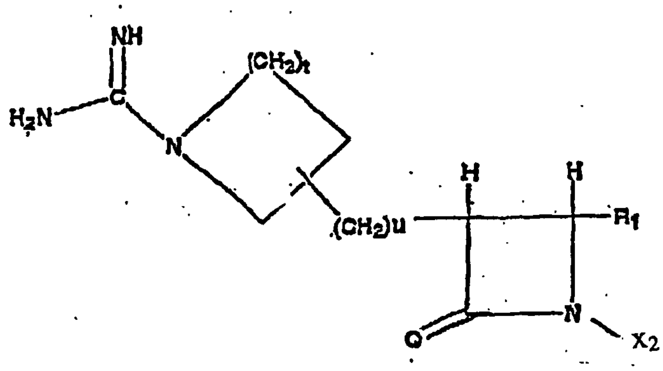

einschließlich eines inneren Salzes oder eines pharmazeutisch verträglichen Salzes davon, wobei:

$R_1$, $X_2$, t und u wie in Anspruch 1 definiert sind.

3. Verbindung nach Anspruch 1, einschließlich eines inneren Salzes oder eines pharmazeutisch verträglichen Salzes davon, ausgewählt aus

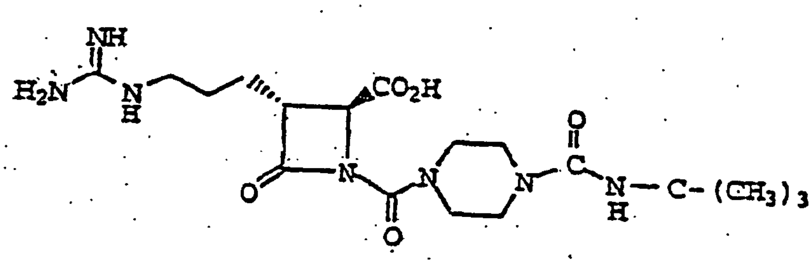

;

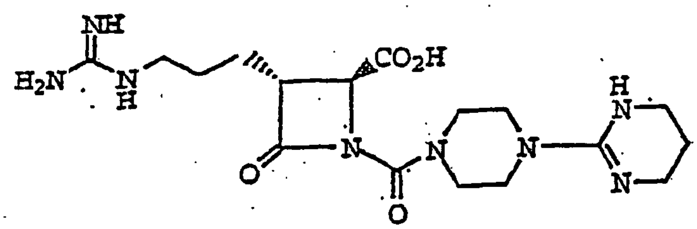

;

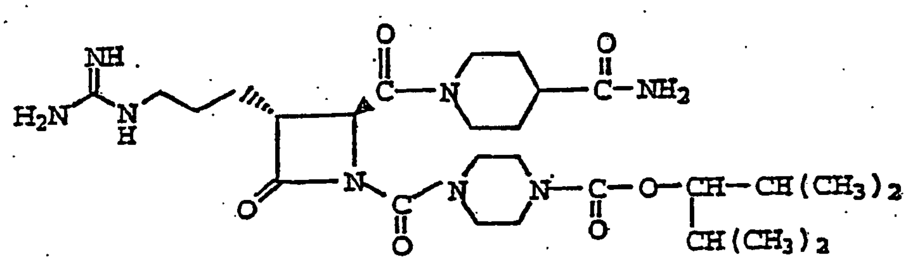

;

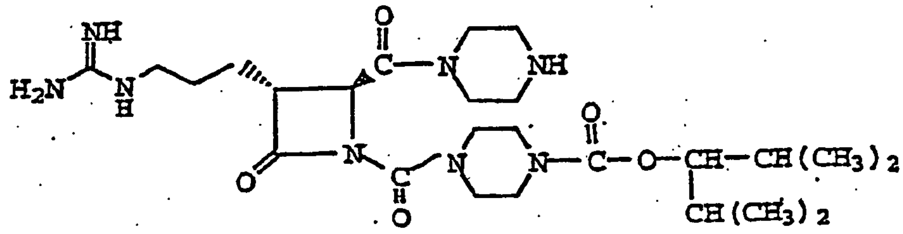

;

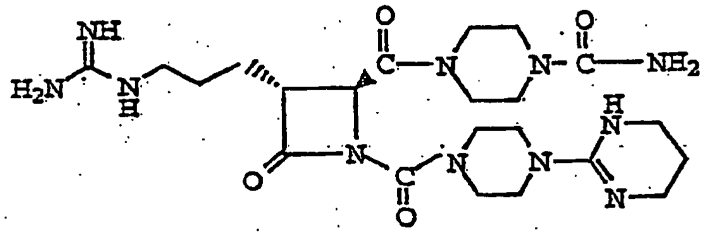

;.

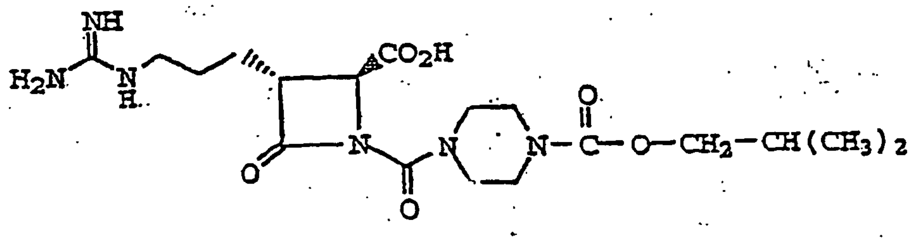

;

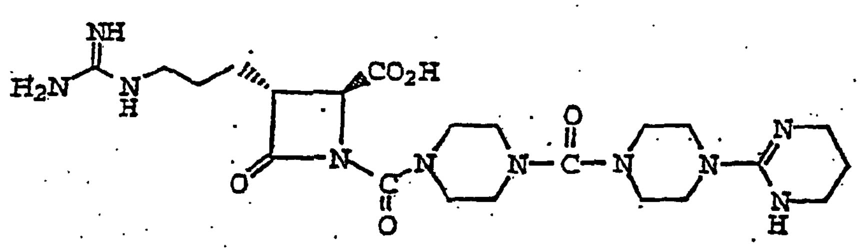

;

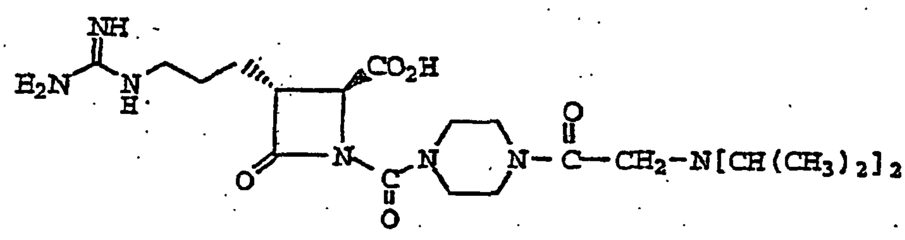

;

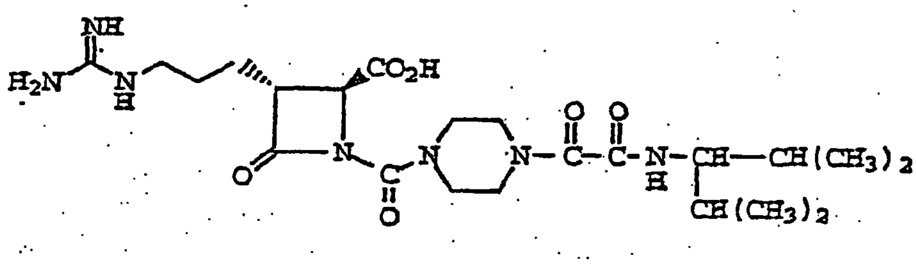

;

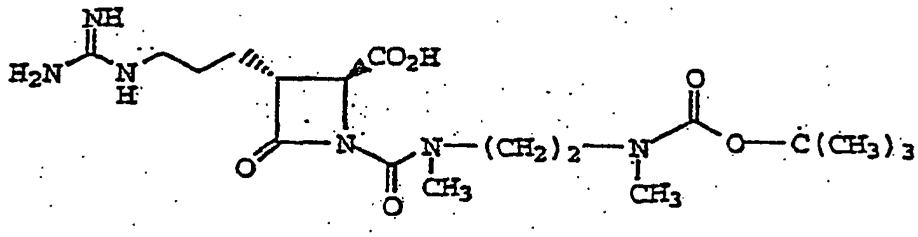

;

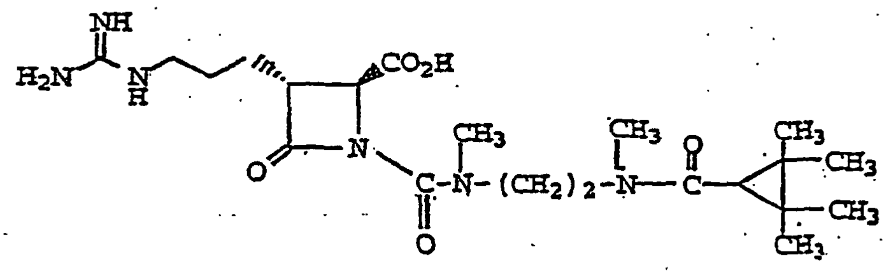

;

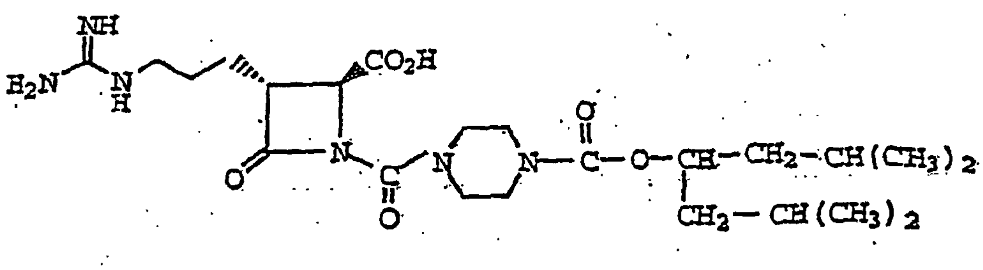

;

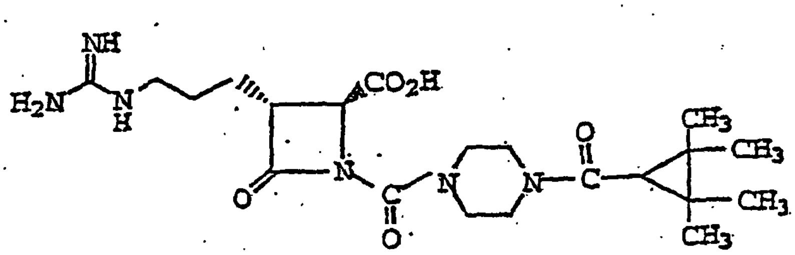

;

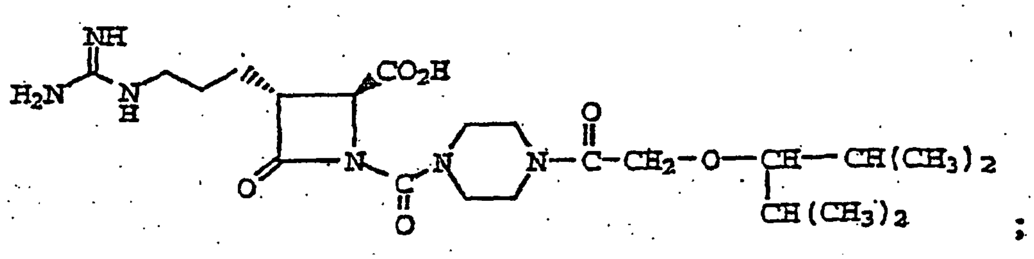

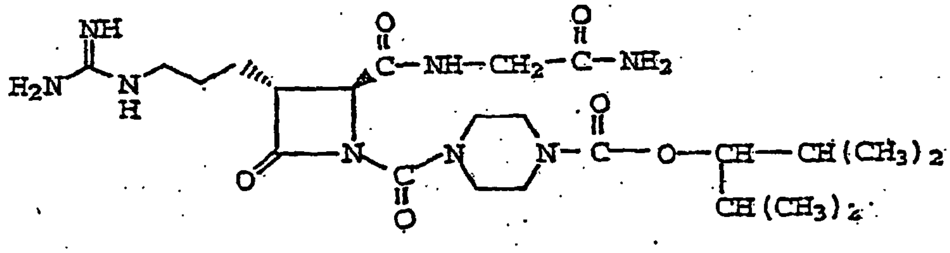

und

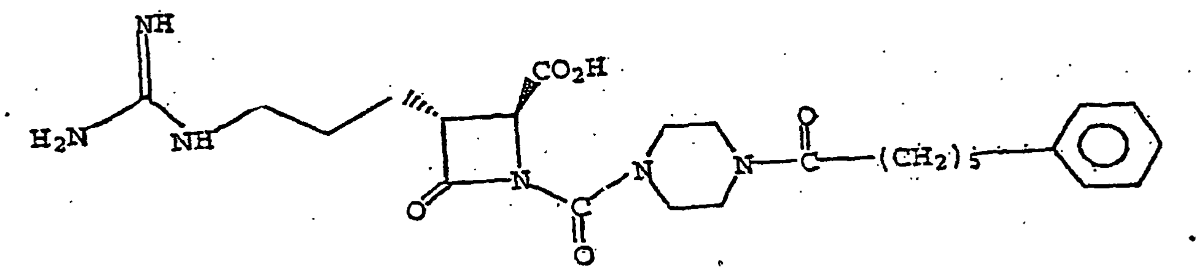

**4.** Verbindung nach Anspruch 3,

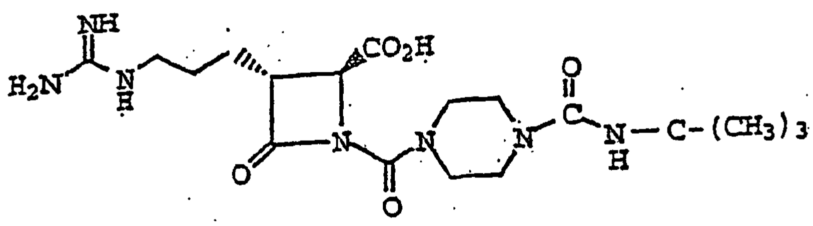

einschließlich eines inneren Salzes oder eines pharmazeutisch verträglichen Salzes davon.

**5.** Verbindung nach Anspruch 3,

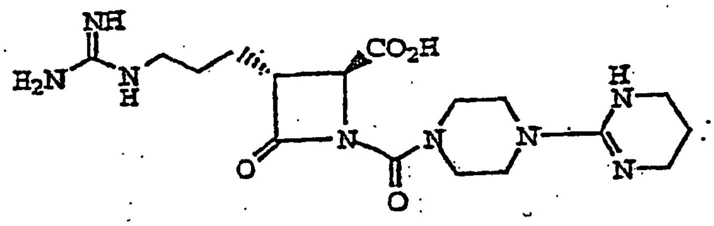

einschließlich eines inneren Salzes oder eines pharmazeutisch verträglichen Salzes davon.

**6.** Verbindung nach Anspruch 3,

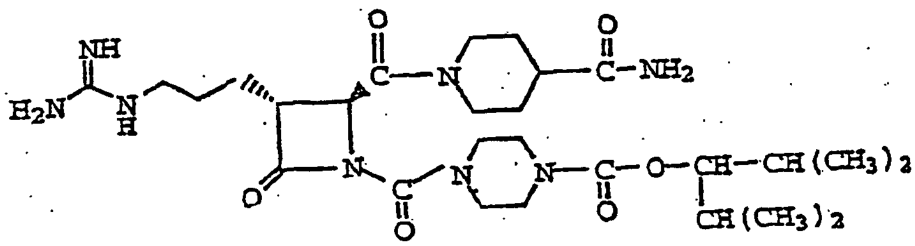

oder eines pharmazeutisch verträglichen Salzes davon.

**7.** Verbindung nach Anspruch 3,

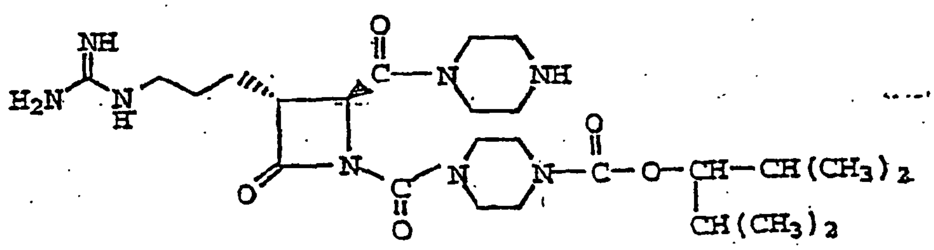

oder eines pharmazeutisch verträglichen Salzes davon.

**8.** Verbindung nach Anspruch 3,

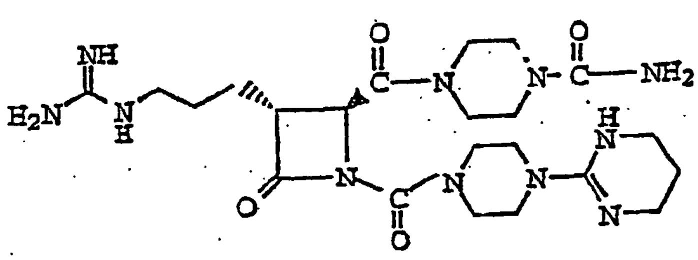

oder ein pharmazeutisch verträgliches Salz davon.

**9.** Verbindung nach Anspruch 3,

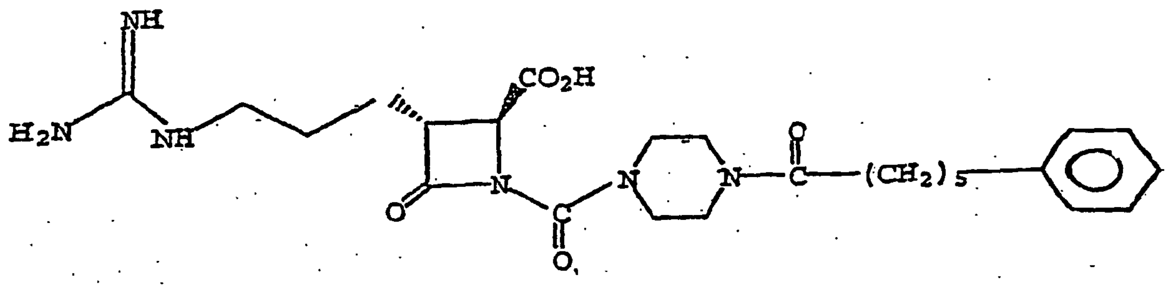

oder ein pharmazeutisch verträgliches Salz davon.

**10.** Verbindung nach Anspruch 1, einschließlich eines inneren Salzes oder eines pharmazeutisch verträglichen Salzes davon, mit der Formel

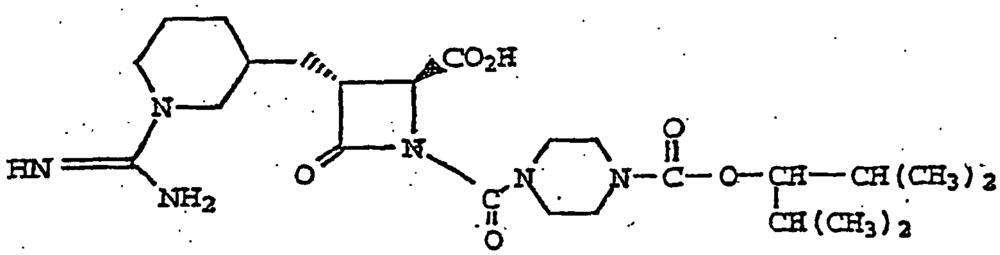

**11.** Verbindung nach Anspruch 1, einschließlich eines inneren Salzes oder eines pharmazeutisch verträglichen Salzes davon, mit der Formel

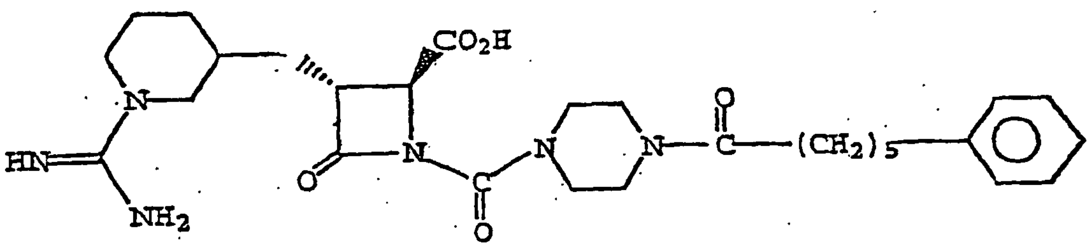

**12.** Verwendung einer Verbindung wie in einem der Ansprüche 1 bis 11 definiert, für die Herstellung eines Arzneimittels zur Behandlung und/oder Vorbeugung medizinischer Zustände in einer Säuger-Spezies, welche im Zusammenhang stehen mit Tryptase, Thrombin, Trypsin, Faktor Xa, Faktor VIIa oder dem Plasminogenaktivator vom Urokinase-Typ.

**13.** Verwendung einer Verbindung wie in einem der Ansprüche 1 bis 11 definiert, für die Herstellung eines Arzneimittels zur Behandlung und/oder Vorbeugung von Asthma oder allergischer Rhinitis in einer Säuger-Spezies.

**14.** Verwendung einer Verbindung wie in Anspruch 3 definiert, für die Herstellung eines Arzneimittels zur Behandlung von chronischem Asthma in einer Säuger-Spezies, wobei die Zusammensetzung durch Inhalation in die Bronchiolen verabreicht werden kann.

## Revendications

**1.** Composé de formules

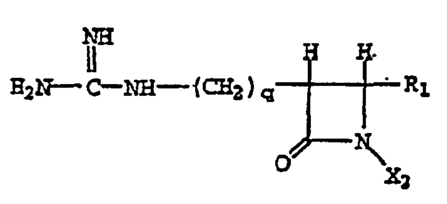

(IV)

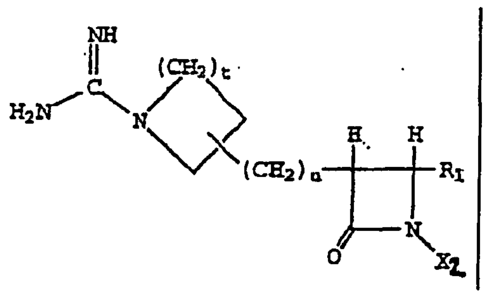

( I')

incluant un sel interne ou un sel pharmaceutiquement acceptable de celui-ci dans lequel :

q = 3 ; t = 2 ou 3 ; u = 1 ;
$R_1$ = carboxy,

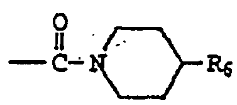 , 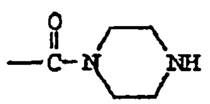 , 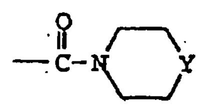

ou

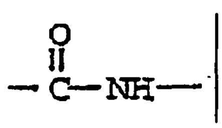 (alkyl substitué)

$X_2$ =

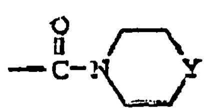 , 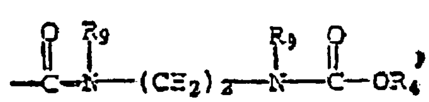 ,

ou

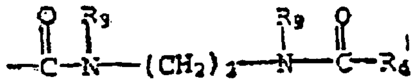 ;

$R_6$ = aminocarbonyle,

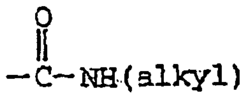 , ou 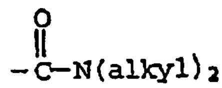 ;

Y = N-$R_4$'

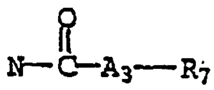 , 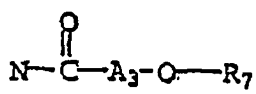

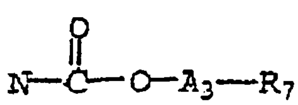 , 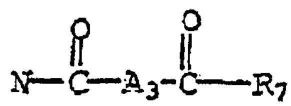

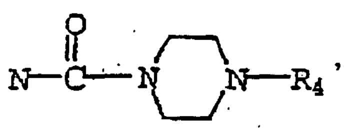 ;

$R_4$' pour la définition de Y et $X_2$ est un alkyl, cycloalkyl, alkyl substitué, cycloalkyl substitué, ou hétéroaryle ;

- $R_7$ est un alkyl, cycloalkyl, alkyl substitué, cycloalkyl substitué, phényl, $-(CH_2)_{1 \text{ à } 4}$-phényl, $-(CH_2)_{1 \text{ à } 4}$-phényl-$A_3$-phényl,

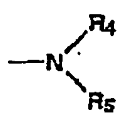 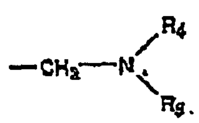 ;

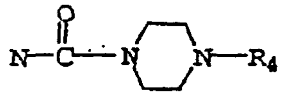 = amino,

-NH(alkyl) ou -N(alkyk)$_2$-

$R_9$ = $C_1$-$C_4$ alkyl ;
$A_3$ est une liaison, un pont alkylène comprenant de 1 à 6 carbones,

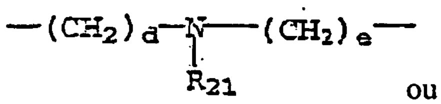 ou $-(CH_2)_d-O-(CH_2)_e-$ ;

d et e sont indépendamment sélectionnés entre 0 et un nombre entier entre 1 et 6 ; et
$R_{21}$ est un hydrogène ou $C_1$-$C_4$ alkyl, dans lequel :

le terme "alkyl substitué" désigne des radicaux linéaires ou ramifiés contenant de 1 à 10 carbones, dans lesquels 1 ou plusieurs hydrogènes ont été remplacés par un groupe hydroxy, amino, cyano, halo, trifluorométhyl, nitro, -NH($C_1$-$C_4$ alkyl), -N($C_1$-$C_4$ alkyl)$_2$, alkoxy, alkylthio, carboxy, alkoxycarbonyl, aminocarbonyl, ou alkoxycarbonylamino ;
le terme "cycloalkyl substitué" désigne des cycles comprenant de 3 à 7 carbones ayant un substituant ou plus, sélectionnés parmi $C_1$-$C_4$ alkyl, $C_1$-$C_4$ alkoxy, $C_1$-$C_4$ alkylthio, halo, hydroxy, trifluorométhyl, nitro, cyano, amino, -NH($C_1$-$C_4$ alkyl), -N($C_1$-$C_4$ alkyl)$_2$, ou carboxy, ainsi que les mêmes cycles fusionnés à un cycle phényl, et
le terme "hétéroaryle" désigne des cycles insaturés ou partiellement saturés comprenant de 4 à 7 atomes de carbone contenant 1 ou 2 atomes d'oxygène et de soufre et/ou 1 à 4 atomes d'azote, 1 à 3 atomes d'azote lorsque le cycle contient 4 atomes, à condition que le nombre total d'hétéroatomes dans le cycle soit de 4 ou moins, 3 ou moins lorsque le cycle a 4 atomes, et le cycle hétéroaryle est lié par le biais d'un atome de carbone ou d'hydrogène disponible, et un cycle hétérocycloalkyl. Les cycles bi-cycliques préférés sont les 2- et 3-indolyl et 4- et 5-quinolynyl et les cycles mono ou bi-cycliques hétéroaryle peuvent aussi être en plus substitués au niveau d'un ou plusieurs atomes de carbone disponibles par un $C_1$-$C_4$ alkyl, halo, carboxy, hydroxy et $A_2$-$C_1$-$C_4$ alkoxy, $A_2$-guanido, benzyl ou cyclohexylméthyl, et si le cycle mono ou bi-cyclique a un atome d'azote disponible, cet atome d'azote peut aussi être substitué par un groupe N protecteur sélectionné parmi le benzyloxycarbonyl, le *tert*-butoxycarbonyl, le benzyl ou le benzhydryl.

**2.** Composé selon la revendication 1

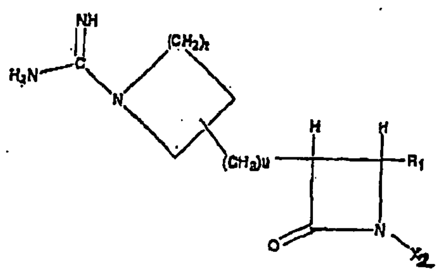

incluant un sel interne ou un sel pharmaceutiquement acceptable de celui-ci, dans lequel :

$R_1$, $X_2$, t et u sont tels que définis dans la revendication 1.

**3.** Composé selon la revendication 1, incluant un sel interne ou un sel pharmaceutiquement acceptable de celui-ci, choisi dans le groupe constitué de

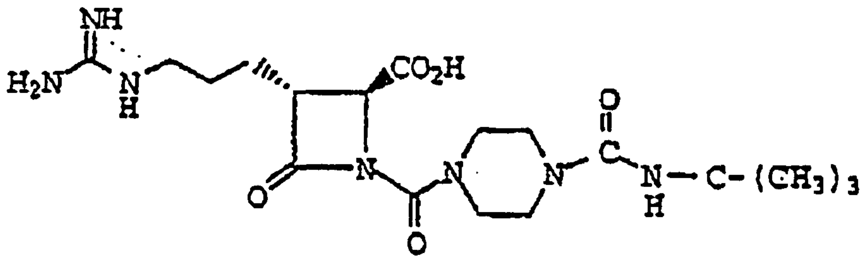

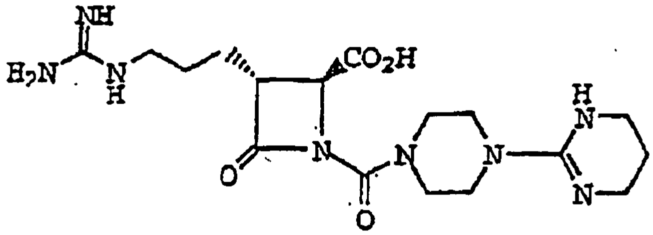

;

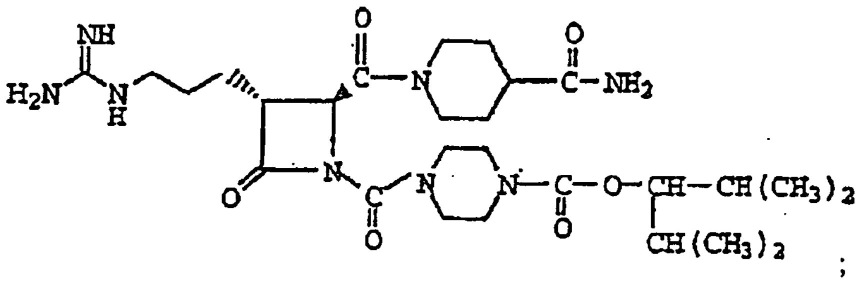

;

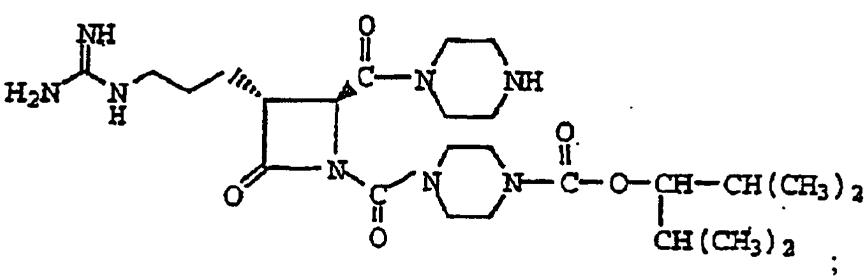

;

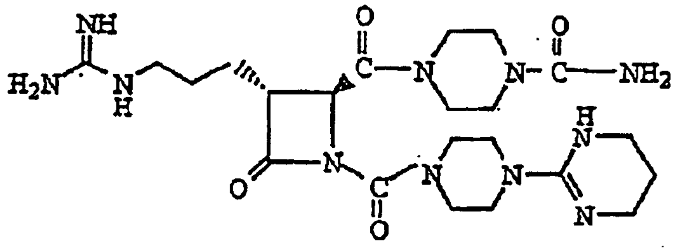

;

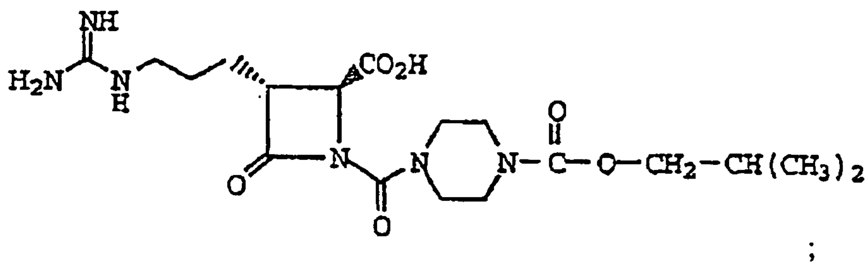

;

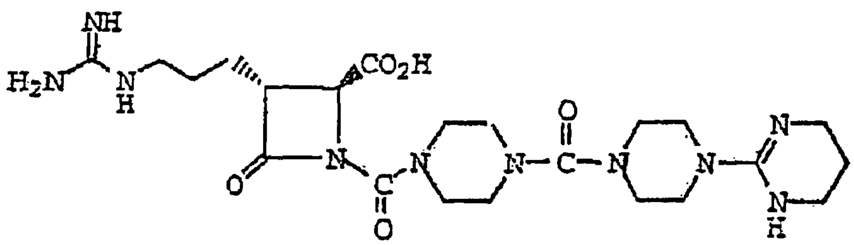

;

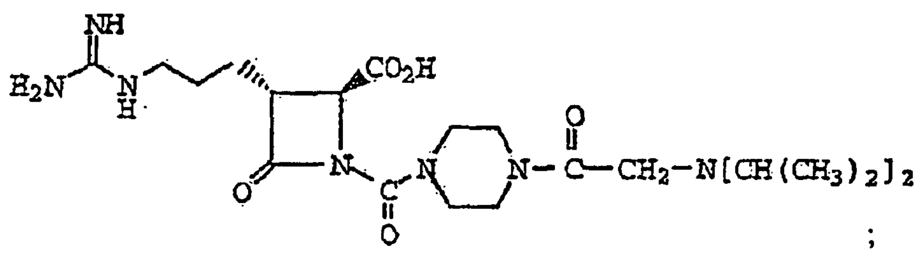

;

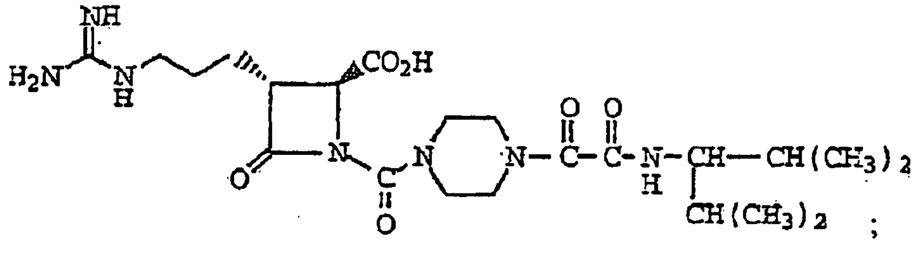

;

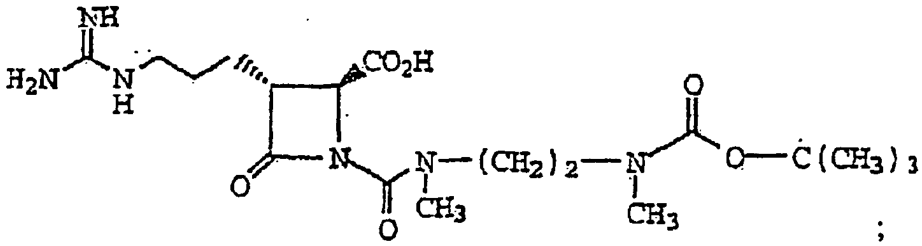

;

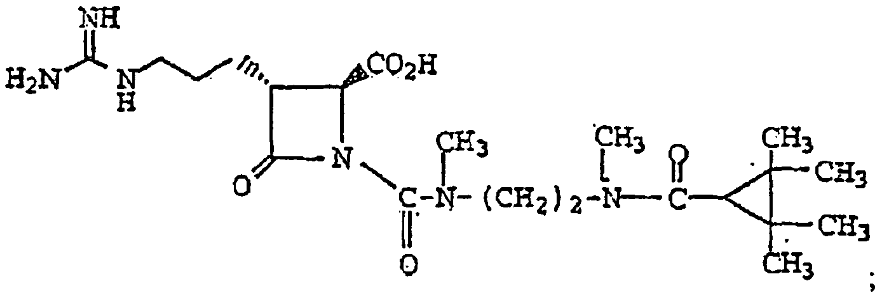

;

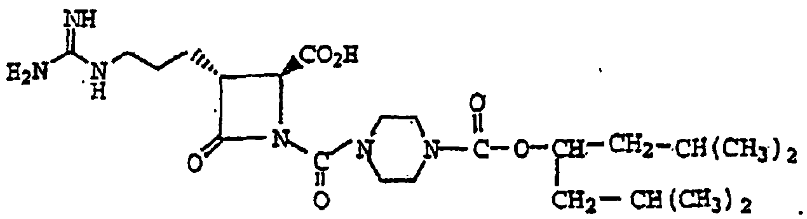

;

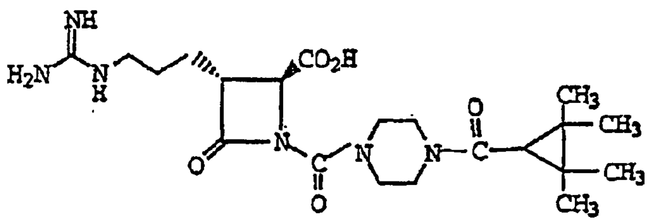

;

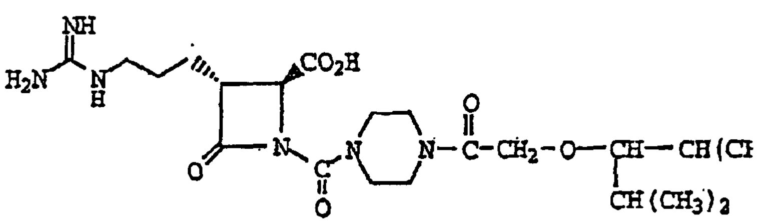

;

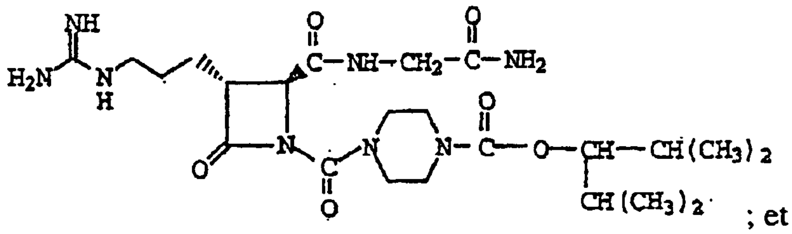

; et

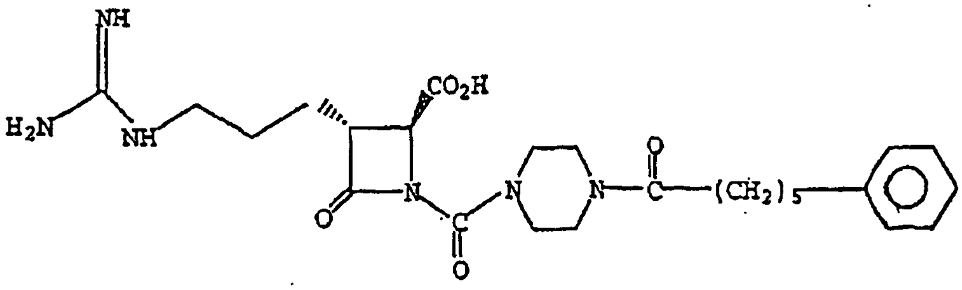

4. Composé selon la revendication 3

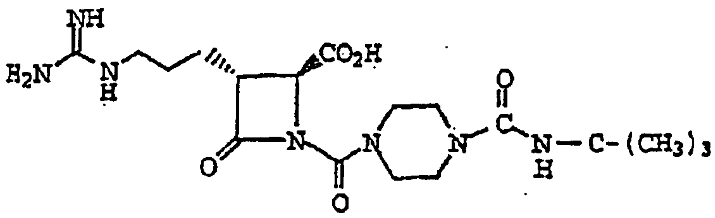

incluant un sel interne ou un sel pharmaceutiquement acceptable de celui-ci.

5. Composé selon la revendication 3

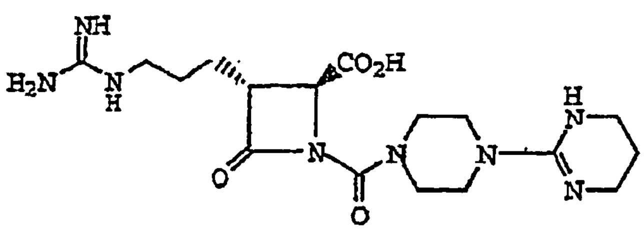

incluant un sel interne ou un sel pharmaceutiquement acceptable de celui-ci.

6. Composé selon la revendication 3

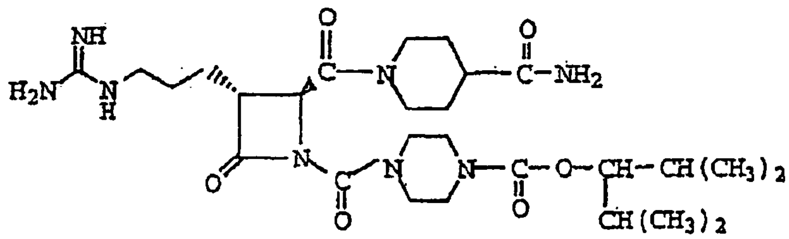

;

ou un sel pharmaceutiquement acceptable de celui-ci.

7. Composé selon la revendication 3

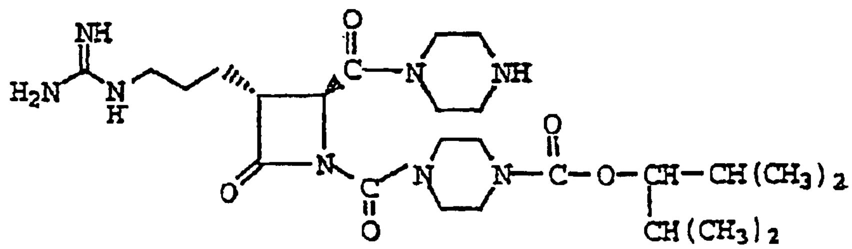

ou un sel pharmaceutiquement acceptable de celui-ci.

8. Composé selon la revendication 3

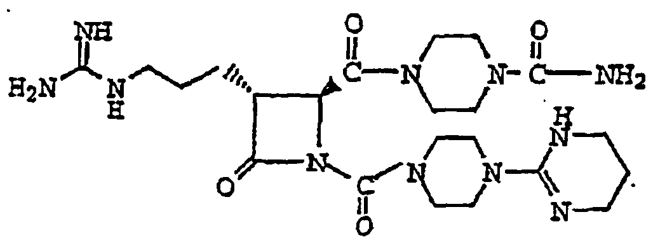

ou un sel pharmaceutiquement acceptable de celui-ci.

9. Composé selon la revendication 3

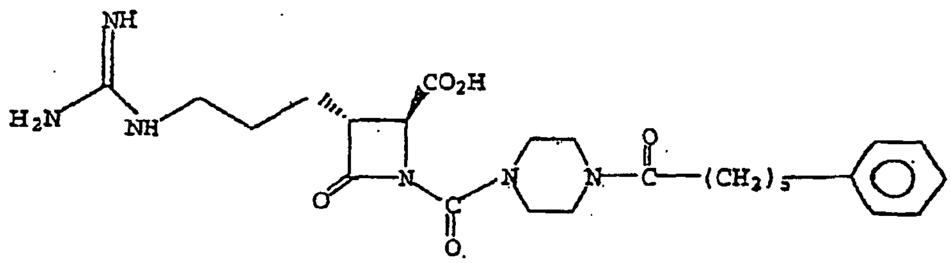

ou un sel pharmaceutiquement acceptable de celui-ci.

10. Composé selon la revendication 1, incluant un sel interne ou un sel pharmaceutiquement acceptable de celui-ci de formule

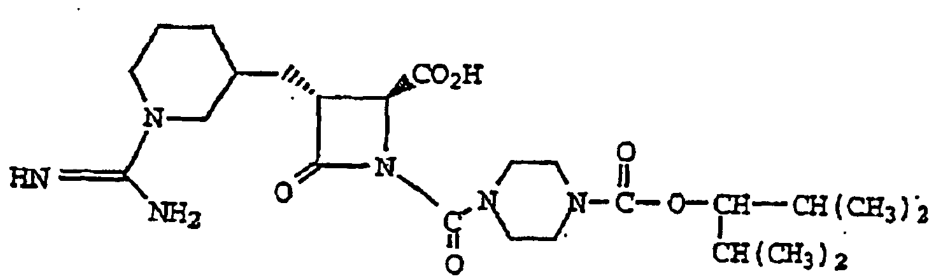

**11.** Composé selon la revendication 1, incluant un sel interne ou un sel pharmaceutiquement acceptable de celui-ci de formule

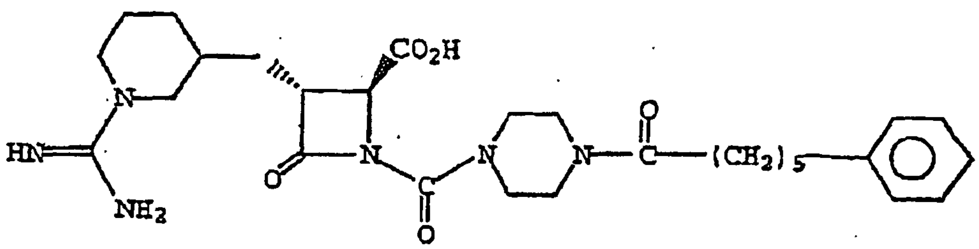

**12.** Utilisation d'un composé tel que défini dans l'une des revendications 1 à 11 pour la fabrication d'une composition pharmaceutique pour le traitement et/ou la prévention chez le mammifère, des conditions médicales impliquant tryptase, thrombine, trypsine, Facteur Xa, Facteur VIIa, ou activateur plasminogène du type urokinase.

**13.** Utilisation de l'un des composés tel que défini dans l'une des revendications 1 à 11 pour la fabrication d'une composition pharmaceutique pour le traitement et/ou la prévention de l'asthme ou de la rhinite allergique du mammifère.

**14.** Utilisation d'un composé tel que défini dans la revendication 3 pour la fabrication d'une composition pharmaceutique pour le traitement de l'asthme chronique du mammifère, dans laquelle la composition peut être administrée par inhalation aux bronchioles.